# EUROPEAN PATENT APPLICATION

(11) **EP 3 584 249 A1**
(43) Date of publication of application: **25.12.2019**
(21) Application number: 18305771.0
(22) Date of filing: 19.06.2018
(51) Int. Cl.: C07F 9/50, A61K 31/66, A61P 31/04

(54) **GOLD (I)-PHOSPHINE 1,2,3-TRIAZOLE DERIVATIVES WITH ANTIOBIOTIC PROPERTIES**

(71) Applicant: Université de Strasbourg, 67000 Strasbourg (FR); CENTRE NATIONAL DE LA RECHERCHE SCIENTIFIQUE, 75016 Paris (FR); Centre Hospitalier Régional Universitaire de Besançon, 25000 Besançon (FR); Université de Franche-Comté, 25000 Besançon (FR)
(72) Inventor: MISLIN, Gaëtan, 67400 ILLKIRCH-GRAFFENSTADEN (FR); SCHALK, Isabelle, 67230 SAND (FR); PLÉSIAT, Patrick, 25000 BESANÇON (FR); PAULEN, Aurélie, 67000 STRASBOURG (FR)
(74) Representative: Novagraaf Technologies

(57) **Abstract**

The present invention relates to gold (I)-phosphine 1,2,3-triazole compounds, and their use in a human or animal medicine. The present invention also relates to using such compounds for the prevention and/or treatment of an infection, i.e. inhibitors of growth of Gram-positive and/or Gram-negative bacteria. On another aspect, the invention relates to the synthesis of the gold (I)-phosphine compounds of the invention and to their synthesis intermediates.

The present invention finds applications in the medical, veterinary and/or chemical fields.

## Description

### Technical field

The present invention relates to gold (I)-phosphine 1,2,3-triazole compounds, and their use in a human or animal medicine. The present invention also relates to using such compounds for the prevention and/or treatment of an infection, i.e. inhibitors of growth of Gram-positive and/or Gram-negative bacteria. On another aspect, the invention relates to the synthesis of the gold (I)-phosphine compounds of the invention and to their synthesis intermediates.

In the description below, references between **[]** refer to the list of references at the end of the examples.

### Technological background

Interest in antimicrobial gold complexes first originated by Robert at the end of 19th century. Dr. Robert Koch demonstrated that potassium dicyanidoaurate(I), K[Au(CN)₂], showed activity against Mycobacterium tuberculosis, a causative agent of tuberculosis.

Subsequently, there has been a large number of gold(I) and gold(III) complexes which have been evaluated for their possible antimicrobial agents properties against a broad spectrum of bacteria, fungi and parasites.

Gold (I)-complexes and their use as inhibitors of growth of Gram-positive and/or Gram-negative bacteria are known in the art.

Bacteria and other microorganisms becoming more and more resistant to antibiotics and antimicrobial agents is of major threat on both the short and the long term. Over the last 50 to 60 years, an increasing number of antimicrobial-resistant bacterial pathogens has emerged and continues to emerge.

In recent years, a large number of gold(I) and gold(III) complexes have been evaluated as possible antimicrobial agents against a broad spectrum of bacteria, fungi and parasites **[1].**

For instance, some anti-bacterial compounds based on amino-gold phosphine complexes are described in the art **[2].** Among those are disclosed 1,2,3-triazole derivatives where the gold (I)-phosphine is on one of the nitrogen. The result is that the structural assignments for those compounds may be unconfirmed due to regioisomerism: the process cannot control which nitrogen will bear gold (I)-phosphine. The result is a mixture of compounds which can make difficult to predict which isomer is really responsible for the effect. Moreover, random compounds may have more difficulties to obtain marketing authorization files in several countries.

There is therefore a need to provide compounds that resolve any regioisomerism drawback while also showing good activity for the prevention and/or treatment of an infection of the state of the art.

### Detailed description

Applicants have surprisingly obtained a new class of compounds, using click-chemistry of therapeutic interest. The compounds of the invention display antimicrobial activity. They may be used in the prevention and/or treatment of an infection, preferably bacterial or fungal.

Applicants have surprisingly observed that the antimicrobial activity of the compounds according to the invention does not only depend on the presence of an antibiotic moiety within the structure of the gold (I)-phosphine 1,2,3-triazole compounds.

In a first aspect, the present invention relates to a new class of gold (I)-phosphine 1,2,3-triazole compound of formula I or I': 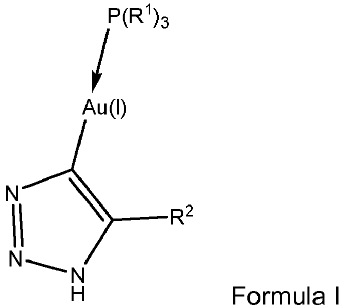 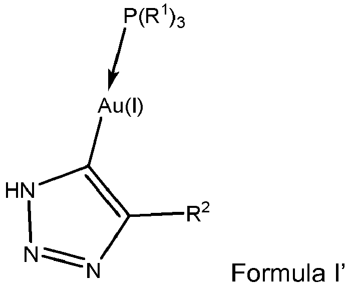 wherein:
- each R¹ group, identical or different, independently represents a linear C₁ to C₁₄ alkyl group, branched or unbranched, cyclic or non-cyclic, saturated or unsaturated, optionally comprising heteroatom(s) chosen from the group consisting of O, N and S and optionally comprising aromatic or non-aromatic cycle(s) or heterocycle(s), two or more R¹ groups being optionally covalently linked to form a substituted or non-substituted heterocycle,
- R² represents a substituent of general formula II:

   -X-([Y]ₐ-[Z]_{b})_{c} Formula II

   in which,
   ∘ a is an integer equal to 0, 1 or 2,
   ∘ b is an integer equal to 0, 1 or 2,
   ∘ c is an integer equal to 0, 1 or 2,
   ∘ X is a monovalent, divalent or trivalent C₁ to C₂₀ group, branched or unbranched, saturated or unsaturated, substituted or non-substituted, optionally comprising heteroatom(s) chosen from the group consisting of O, N and S and optionally comprising one or more aromatic or non-aromatic, substituted or non-substituted cycle(s) or heterocycle(s), preferably a monovalent or divalent C₁ to C₁₄ group and more preferably a monovalent or divalent C₁ to C₉ group,
   ∘ Y independently represents an amino acid residue,
   ∘ Z independently represents -NR³R'³, -SO₂NR³R'³ -OR³, a beta-lactam derivative, a (fluoro)quinolone derivative, a cycline derivative, an oxazolidinone derivative, a macrolide derivative, a ketolide derivative, a puromycin derivative, a aminoside derivative, a lincosamide derivative, a sulfamide derivative, a phenicol derivative, a polymyxin derivative, a rifamycin derivative, a glycopeptide derivative or biotin,
   ∘ R³ and R'³, identical or different, independently represent H or a C₁ to C₁₈ linear or branched alkyl group, optionally comprising heteroatom(s) chosen from the group consisting of O and N and optionally comprising one or more aromatic or non-aromatic, substituted or non-substituted cycle(s) or heterocycle(s), preferably a C₁ to C₉ linear or branched alkyl group and more preferably C₁ to C₅ linear or branched alkyl group, R³ and R'³ are optionally covalently linked to form a substituted or non-substituted heterocycle.

Advantageously , the compound of the invention may be in the form or a pharmaceutically acceptable salt or solvate thereof. Pharmaceutically acceptable salts may be an alkali metal cation salt such as Na⁺ or K⁺, an alkaline earth metal cation salt such as Mg²⁺ or Ca²⁺, an organic anion salt such as ammonium, alkyl ammonium or acetate.

Advantageaouly, the compound of the invention may be an isotope of the compound of formula I and I', preferably a deuterium, tritium or ¹⁴C isotope.

Advantageously , each R¹ group is identical or different. Each R¹ group may independently represent a linear C₁ to C₁₄ alkyl group, branched or unbranched, cyclic or non-cyclic, saturated or unsaturated, optionally comprising heteroatom(s) chosen from the group consisting of O, N and S, preferably O and N, and optionally comprising aromatic or non-aromatic cycle(s) or heterocycle(s), two or more R¹ groups being optionally covalently linked to form a substituted or non-substituted heterocycle. Preferably, each R¹ group may independently represent a C₁ to C₈ group.

For example, each R¹ group may be chosen in the group comprising methyl, ethyl, n-propyl, i-propyl, n-butyl, i-butyl, t-butyl, pentyl, cyclopentyl, hexyl, cyclohexyl, phenyl, benzyl, preferably methyl, ethyl, cyclohexyl, phenyl and i-propyl.

Advantageously, the trialkyl phosphine goup P(R¹)₃ may be chosen from the following structures:

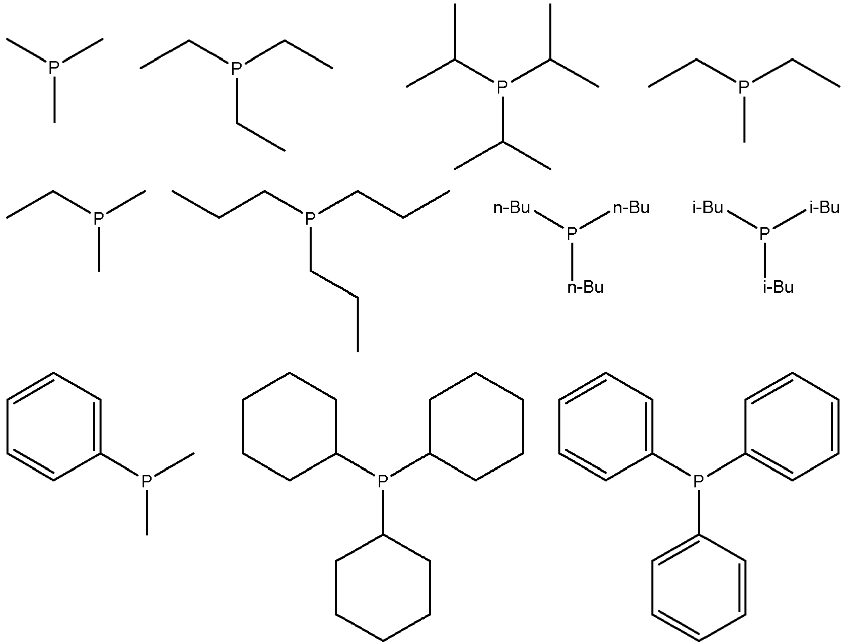

Advantageously , R² represents a substituent of general formula II:

-X-([Y]ₐ-[Z]_{b})_{c} Formula II

in which X, Y, Z, a , b and c are defined as above.

Advantageously, X may be a monovalent, divalent or trivalent C₁ to C₂₀ group, branched or unbranched, saturated or unsaturated, substituted or non-substituted, optionally comprising heteroatom(s) chosen from the group consisting of O, N and S and optionally comprising one or more aromatic or non-aromatic, substituted or non-substituted cycle(s) or heterocycle(s). Preferably, X may be a monovalent, divalent or trivalent C₁ to C₁₄ group, branched or unbranched, saturated or unsaturated, substituted or non-substituted, optionally comprising heteroatom(s) chosen from the group consisting of O, N and S and optionally comprising one or more aromatic or non-aromatic, substituted or non-substituted cycle(s) or heterocycle(s) and more preferably a monovalent, divalent or trivalent C₁ to C₉ group, branched or unbranched, saturated or unsaturated, substituted or non-substituted, optionally comprising heteroatom(s) chosen from the group consisting of O, N and S and optionally comprising one or more aromatic or non-aromatic, substituted or non-substituted cycle(s) or heterocycle(s). X may comprise protected or unprotected functional groups such as -OH, -NH₂, -COOH, -SO₂NH₂. The functional groups may be protected by ester, carbamate, amide, carbonate, urea or more generally by the protective groups described in the Greene reference **[3].** Protective groups may be ester.

Advantageously, when a = b = c = 0, X is monovalent. When c = 1 and a and/or b ≠ 0, X is divalent, meaning X is covalently linked to the triazole and the Y or Z group. When c = 2, X is trivalent.

Advantageously, when a = 1, b = 1 and c = 1, X is divalent and R² may be of formula IIa:

-X-Y-Z Formula IIa,

wherein X, Y and Z are defined as above.

Advantageously, when a = 0, b = 1 and c = 1, X is divalent and R² may be of formula IIb:

-X-Z Formula IIb,

wherein X and Z are defined as above.

Advantageously, when a = 2, b = 1 and c = 1, X is divalent and R² may be of formula IIc:

-X-Y-Y-Z Formula IIc,

wherein X, Y and Z are defined as above, each Y may be identical or different.

Advantageously, when a = 1, b = 1 and c = 2, X is trivalent and R² may be of formula IId:

-X(Y-Z)-Y-Z Formula IId,

wherein X, Y and Z are defined as above, each Y and/or Z may be identical or different.

Adavantageously, the X group may be further chosen from the following divalent structures: 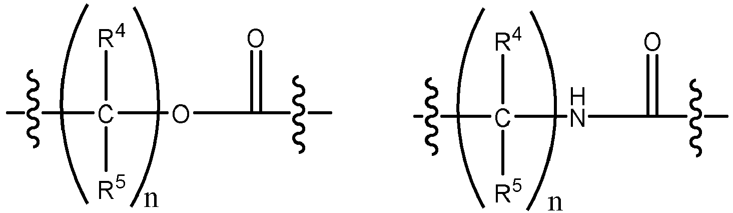 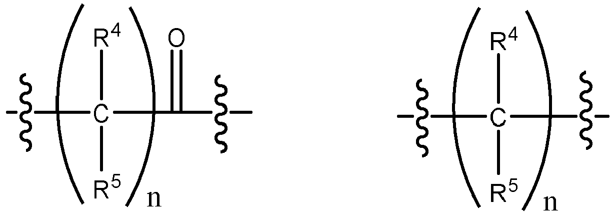 wherein
∘ each R⁴ and R⁵, identical or different, independently represents -H, a halogen, a C₁ to C₅ linear or branched alkyl group, a C₁ to C₅ linear or branched alkoxy group, -OH or - NR³R'³, R³ and R'³ identical or different, independently being H or a C₁ to C₁₈ linear or branched alkyl group, optionally comprising heteroatom(s) chosen from the group consisting of O and N, R³ and R'³ are optionally covalently linked to form a substituted or non-substituted heterocycle, R⁴ and R⁵ being optionally covalently linked to form a cycle or heterocycle,
∘ n is an integer from 1 to 5, 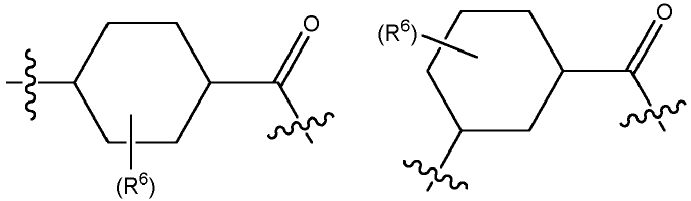 wherein
∘ R⁶ is one ore more substituent(s) on the ring and independently represents one or more -H, halogen(s), C₁ to C₅ linear or branched alkyl group(s), a C1 to C5 linear or branched alkoxy group(s), -OH or -NR¹⁰R'¹⁰, R¹⁰ and R'¹⁰ identical or different, independently being H or a C₁ to C₁₃ linear or branched alkyl group, optionally comprising heteroatom(s) chosen from the group consisting of O and N, R¹⁰ and R'¹⁰ are optionally covalently linked to form a substituted or non-substituted heterocycle, R⁶ groups being optionally covalently linked to form a cycle or heterocycle, 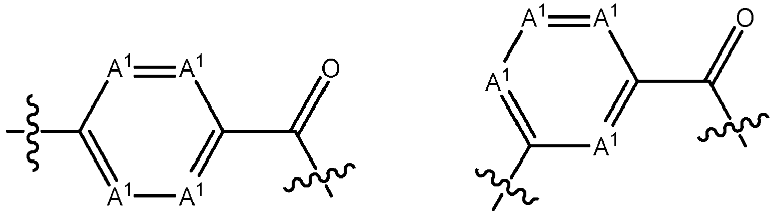 wherein
∘ each A¹ independently represents N, CH or CR¹¹,
∘ R¹¹ is one ore more substituent(s) on the ring and independently represents one or more -H, halogen(s), a C₁ to C₅ linear or branched alkyl group(s), a C₁ to C₅ linear or branched alkoxy group(s), -OH or -NR¹²R'¹², R¹² and R'¹² identical or different, independently being H or a C₁ to C₁₇ linear or branched alkyl group, optionally comprising heteroatom(s) chosen from the group consisting of O and N, R¹² and R'¹² are optionally covalently linked to form a substituted or non-substituted heterocycle, R¹¹ groups being optionally covalently linked to form a cycle or heterocycle, 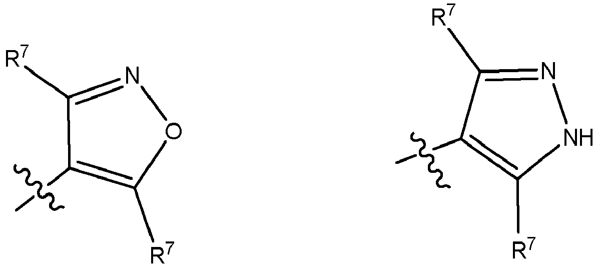 wherein
∘ each R⁷ independently represents -H, a halogen, a C₁ to C₅ linear or branched alkyl group, a C₁ to C₅ linear or branched alkoxy group, -OH or -NR¹²R'¹², R¹² and R'¹² identical or different, independently being H or a C₁ to C₁₇ linear or branched alkyl group, optionally comprising heteroatom(s) chosen from the group consisting of O and N, R¹² and R'¹² are optionally covalently linked to form a substituted or non-substituted heterocycle, R⁷ groups being optionally covalently linked to form a cycle or heterocycle, 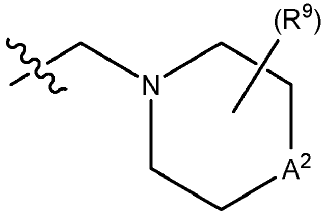 wherein
∘ A² represents CH₂, CHR⁸, NR⁸, O, S or SO₂,
∘ R⁹ is one ore more substituent(s) on the ring and independently represents one or more -H, halogen(s), C₁ to C₅ linear or branched alkyl group(s), C₁ to C₅ linear or branched alkoxy group(s), -OH or -NR¹³R'¹³, R¹³ and R'¹³ identical or different, independently being H or a C₁ to C₁₅ linear or branched alkyl group, optionally comprising heteroatom(s) chosen from the group consisting of O and N, R¹³ and R'¹³ are optionally covalently linked to form a substituted or non-substituted heterocycle, R⁹ groups being optionally covalently linked to form a cycle or heterocycle, ∘ R⁸ represents -H or a C₁ to C₅ linear or branched alkyl group.

Advantageously, the X group may be further chosen from the following structures: 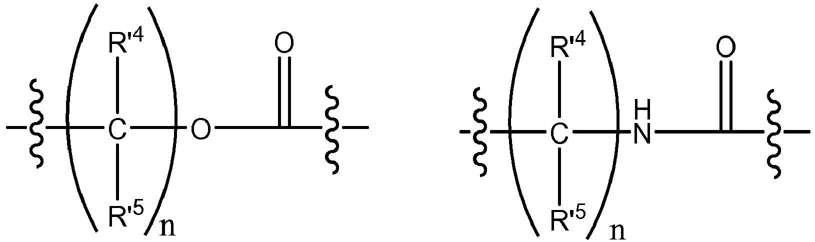 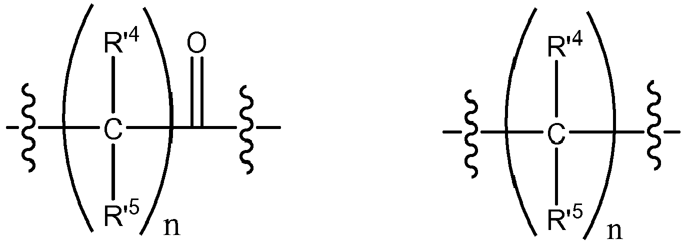 wherein
∘ each R'⁴ and R'⁵, identical or different, independently represents -H, a C₁ to C₅ linear or branched alkyl group, -OH or -NH₂,
∘ n is an integer from 1 to 5, 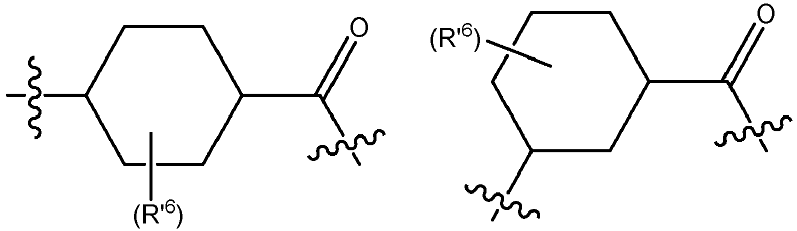 wherein
∘ R'⁶ is one ore more substituent(s) on the ring and independently represents one or more -H, C₁ to C₅ linear or branched alkyl group(s), -OH or -NH₂, 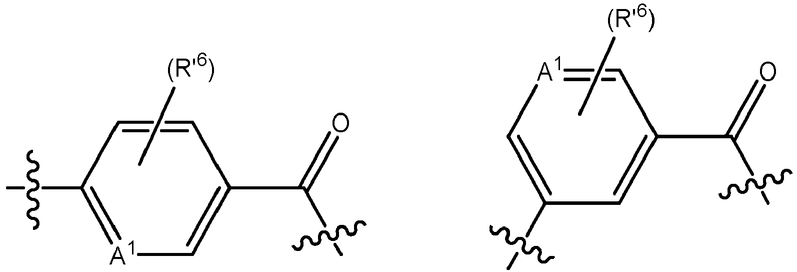 wherein
∘ A¹ represents N or CH,
∘ R'⁶ is one ore more substituent(s) on the ring and independently represents one or more -H, C₁ to C₅ linear or branched alkyl group(s), -OH or -NH₂, 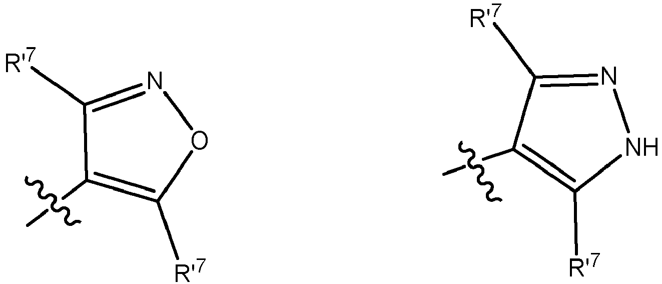 wherein
∘ each R⁷ independently represents -H, a C₁ to C₅ linear or branched alkyl group, -OH or -NH₂, 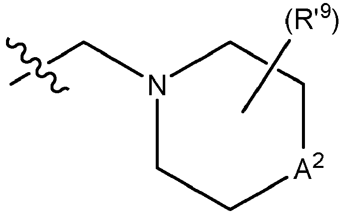 wherein
∘ A² represents NR'⁸ or O,
∘ R'⁹ is one ore more substituent(s) on the ring and independently represents one or more -H, C₁ to C₅ linear or branched alkyl group(s), -OH or -NH₂,
∘ R'⁸ represents -H or a C₁ to C₅ linear or branched alkyl group.

In the represented structures, the 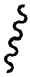 represents, depending on the group X, Y or Z which is represented:
- the point(s) of attachment of the X group to the 1,2,3-triazole and/or to the Y or Z group,
- the points of attachement of the Y group either to the X or Z group, or
- the point of attachment of the Z group either to the X or Y group.

Advantageously , the X group may be chosen from the following monovalent and divalent structures:

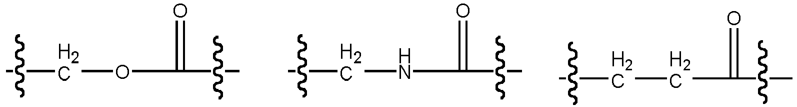

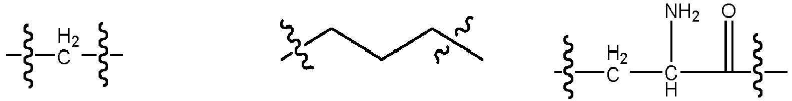

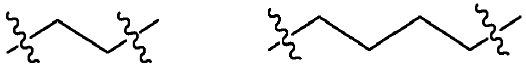

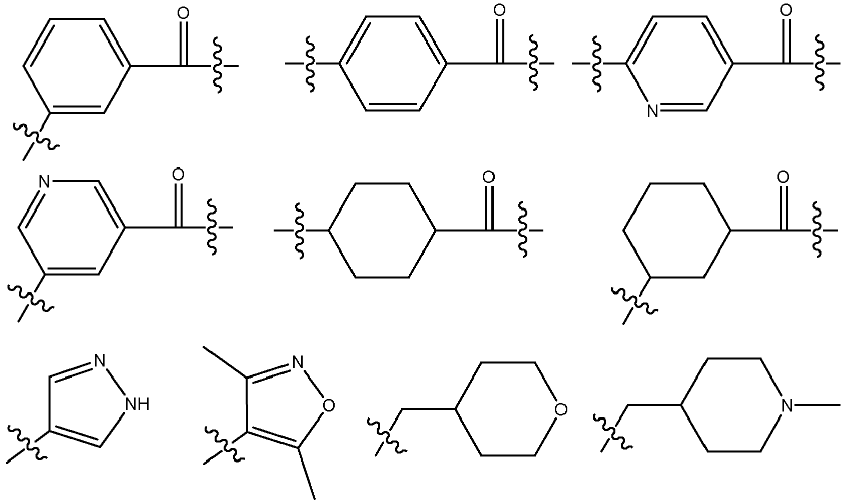

Advantageously, Y may be an amino acid residue. The amino acid may be chosen from the group comprising natural and synthetic amino acids. The amino acid may be an alpha, beta or gamma amino acid. Y may be an amino acid residue comprising from 1 to 14 carbon atoms. Alpha amino acids may be isomer D, L, D/L or non racemic mixtures thereof. In particular, isomers D which are not sensible to proteases may be preferred. According to the present disclosure, the amino acid may be chosen from the group comprising arginine, ω-NO₂-arginine, histidine, lysine, aspartic acid, glutamic acid, serine, threonine, asparagine, glutamine, selenocysteine, glycine, proline, alanine, isoleucine, leucine, phenylalanine, tyrosine, tryptophan, valine, methionine, cysteine, phenylglycine, hydroxyphenylglycine, substituted or not substituted. Preferably the amino acid may be further chosen from the group comprising glycine, phenylglycine, hydroxyphenylglycine, ω-NO₂-arginine, aspartic acid, tryptophan, tyrosine and phenylalanine.

Advantageously, the Y group may be linked to the X group via an amine, amide, carbamate or urea functional group.

Advantageously, Z may be a group chosen from -NR³R'³, - SO₂NR₃R₃', -OR³, a beta-lactam derivative, a (fluoro)quinolone derivative, a cycline derivative, an oxazolidinone derivative, a macrolide derivative, a ketolide derivative, a puromycin derivative, a aminoside derivative, a lincosamide derivative, a sulfamide derivative, a phenicol derivative, a polymyxin derivative, a rifamycin derivative, a glycopeptide derivative or biotin. R³ and R'³ identical or different, may independently represent H or a C₁ to C₁₈ linear or branched alkyl group, optionally comprising heteroatom(s) chosen from the group consisting of O and N, R³ and R'³ are optionally covalently linked to form a substituted or non-substituted heterocycle. Preferably, R³ and R'³ identical or different, may independently represent H or a C₁ to C₅ linear or branched alkyl group. The Z group may preferably represent -NR³R'³, -SO₂NH₂, -OR³, a beta-lactam derivative or a fluoroquinolone derivative. The beta-lactam derivative may be chosen from penicillins or cephalosporins derivatives.

Adavntageaously, the Z group may be chosen from the following structures:

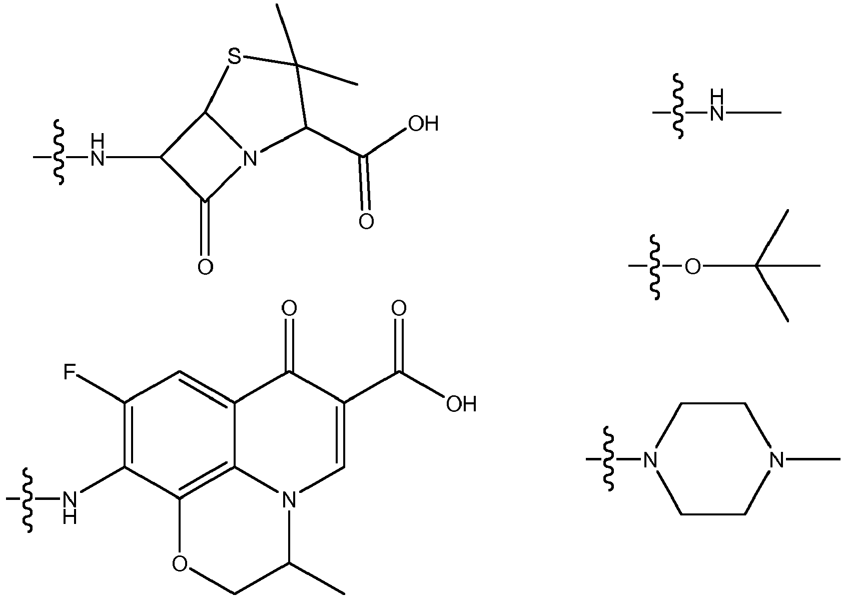

Advantageously, the Z group may be linked to the X or Y group via an amine, amide, carbamate or urea functional group.

### Definitions

To facilitate an understanding of the present invention, a number of terms and phrases are defined below:
In chemical synthesis, "click" chemistry is a class of biocompatible small molecule reactions commonly used in bioconjugation, allowing the joining of substrates of choice with specific biomolecules. Click chemistry is not a single specific reaction, but describes a way of generating products that follow examples in nature, which also generates substances by joining small modular units. In general, click reactions usually join a biomolecule and a reporter molecule. Click chemistry is not limited to biological conditions: the concept of a "click" reaction has been used in pharmacological and various biomimetic applications. However, they have been made notably useful in the detection, localization and qualification of biomolecules. As used herein, "click chemistry" include but is not limited to the azide-alkyne 1,3-dipolar cycloaddition.

The term "derivative" refers to a chemical compound or molecule made from a parent compound by one or more chemical reactions.

In general, the term "substituted" or "non-substituted" whether preceded by the term "optionally" or not, and substituents contained in formula of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds.

As used herein, it is meant by « amino acid residue », what remains of an amino acid, when the elements of water are removed due to linkage. By natural amino acid is meant amino acids for example alanine, valine, glycine, leucine, isoleucine, lysine, arginine, glutamic acid, glutamine, aspartic acid, asparagine, histidine, tyrosine, phenylalanine, tryptophan, serine, proline, methionine, cysteine or threonine. By "synthetic" or "unnatural" amino acids" is meant an analog or derivative of a natural amino acid. Synthetic amino acids have a modified side chain, e.g. shorter, longer or with different functional groups, preferably a modified arginine, substituted with nitrogen dioxide group.

As used herein, the term "alkyl", refers to straight (linear) and branched alkyl groups. An analogous convention applies to other generic terms such as "alkenyl", "alkynyl" and the like. Illustrative alkyl groups include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-l-yl, and the like. Representative alkynyl groups include, but are not limited to, ethynyl, 2-propynyl (propargy1), 1-propynyl and the like.

As used herein, the terms "optionally comprising heteroatom(s)", refers to groups bearing or having included in the main chain heteroatoms either chosen from O, N and S or O and N.

In general, the terms "saturated" or "unsaturated", as used herein, refers to groups whose molecular structure contains one or more carbon-carbon double bonds or triple bonds.

In general, the terms "aromatic moiety", "aromatic cycle or heterocyle" or "aryl or heteroaryl", as used herein, refers to stable, substituted or unsubstituted, unsaturated mono- or polycyclic hydrocarbon moieties having preferably 3-14 carbon atoms, comprising at least one ring satisfying the Hückel rule for aromaticity. Examples of aromatic moieties include, but are not limited to, phenyl, indanyl, indenyl, naphthyl, phenanthryl and anthracyl.

In general "cyclic" or "non-cyclic", as used herein, refers to a 3 to 8 membered-ring cyclic moiety, in the main chain or on a side chain, substituted or non substituted and optionally comprising heteroatom(s). Examples of such heteroaryl moiety include, but are not limited to, pyridinyl, thiazolyl, thienyl, furanyl, pyrrolyl, pyrazolyl, imidazolyl, triazolyl, tetrazolyl, benzofuranyl, benzazepinyl, thianaphthalenyl, indolyl, indolinyl, quinolinyl, isoquinolinyl, benzimidazolyl, tetrahydroquinolinyl, tetrahydroisoquinolinyl, triazinyl, thianthrenyl, isobenzofuranyl, chromenyl, xanthenyl, phenoxanthinyl, isothiazolyl, isoxazolyl, pyrazinyl, pyridazinyl, indolizinyl, isoindolyl, indazolyl, purinyl, quinolizinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, indolinyl, isoindolinyl, oxazolidinyl, benzotriazolyl, benzisoxazolyl, oxindolyl, benzoxazolinyl, benzothienyl, benzothiazolyl, isatinyl, dihydropyridyl, pyrimidinyl, s-triazinyl, oxazolyl and thiofuranyl.

As used herein, the term "independently" refers to the fact that the substituents, atoms or moieties to which these terms refer, are selected from the list of variables independently from each other (i.e., they may be identical or the same).

As used herein, the term "isomer" refers to compounds that may exist in one or more particular geometric, optical, enantiomeric, diastereomeric, epimeric, atropic, stereoisomeric, tautomeric, conformational or anomeric form. Examples of isomers with being limited to include cis- and trans-forms; *E*- and *Z*-forms; c-, t-, and r- forms; endo- and exo-forms; *R-, S-,* and meso-forms; D- and L-forms; d- and I-forms; (+) and (-) forms; keto-, enol-, and enolate-forms; syn- and anti-forms; synclinal- and anticlinal-forms; a- and β-forms; axial and equatorial forms; boat-, chair-, twist-, envelope-, and halfchair-forms; and combinations thereof, hereinafter collectively referred to as "isomers" (or "isomeric forms"). In the present disclosure, "isomer" exclude structural isomers or constitutional isomers where the isomers differs in constitution and described by different line formula (IUPAC **[4]**).

As used herein, "salt" refers to compounds bearing functional groups that may be anionic or cationic such as -COOH/-COO⁻ and -NH₂/- NH₃⁺, for instance, that may respectively form a salt with a suitable cation or anion. Suitable cations include but are not limited to: alkali metal ions such as Na⁺, K⁺, alkaline earth cation such as Ca²⁺, Mg²⁺, and other cations such as Al³⁺, ammoniun ions and substituted ammonium ions. Suitable anions include but are not limited to: anions derived from organic and inorganic acids such as acetic, hydrochloric, hydroboric, hydroiodic, sulfuric, sulphurous, nitric, nitrous, phosphoric and phosphorous.

As used herein, "solvate" refers to an aggregate that consists of a solute ion or molecule with one or more solvent molecules, or a substance (such as a hydrate) containing such ions.

As used herein, the term "and/or" means any one of the items, any combination of the items, or all of the items with which this term is associated.

As used herein the term "infection" means the invasion of an organism's, preferably a host, body tissues by disease-causing microorganisms, their multiplication, and the reaction of organism's, preferably the host, to these microorganisms and possibly the toxins they produce.

As used herein the term "bacteria" refers to a type of biological cell. They constitute a large domain of prokaryotic microorganisms. Typically a few micrometres in length, bacteria exhibit specific shapes, ranging from spheres to rods and spirals. According to the present disclosure, the bacteria may also be an ESKAPE pathogen.

As used herein, the term "ESKAPE pathogen" refers to bacteria selected from the group constituted of *Enterococcus faecium, Staphylococcus aureus, Klebsiella pneumoniae, Acinetobacter baumannii, Pseudomonas aeruginosa,* and *Enterobacter species.*

As used herein, the term"ESKAPE pathogen" refers also to multidrug-resistant nosocomial bacterial species.

According to the present disclosure, the bacteria may be Gram-positive or Gram-negative, preferably Gram-positive bacteria.

For example, the Gram-negative bacteria/species may be selected from the group comprising *Pseudomonas aeruginosa, Acinetobacter baumannii, Stenotrophomonas maltophilia, Escherichia coli, Klebsiella pneumoniae, Enterobacter species* and *Legionella pneumophila.*

For example, the Gram positive bacteria/species *may be* selected from the group comprising *a Staphylococcus* spp. and *Enterococcus* spp.

Bacteria of the *"Staphylococcus"* genus are non-spore-forming, catalase-positive, oxidase-negative, Gram-positive cocci grouped together in grape-like clusters. Observed by Pasteur in 1879 in furuncle pus, staphylococci owe their name to Ogsten (1881) who isolated them in acute chronic abscesses. Bacteria of the *"Staphylococcus"* genus, such as, for example, *S. aureus, S. epidermidis, S. haemolyticus,* and *S. lugdunensis* are common agents of infections.

According to the present disclosure, the Staphylococcus may be selected from S. aureus, S. epidermidis, S. capitis, S. caprae, S. haemolyticus, S. lugdunensis, S. schleiferi, S. simulans and S. warneri, preferably S. aureus and S. epidermidis, more preferably S. aureus.

Bacteria of the "Micrococcus" genus are generally thought to be a saprotrophic or commensal organism, though it can be an opportunistic pathogen, particularly in hosts with compromised immune systems, such as HIV patients. Micrococci are normally present in skin microflora, and the genus is seldom linked to disease. However, in rare cases, death of immunocompromised patients has occurred from pulmonary infections caused by Micrococcus. Micrococci may be involved in other infections, including recurrent bacteremia, septic shock, septic arthritis, endocarditis, meningitis, and cavitating pneumonia in particular in immunosuppressed patients.

Bacteria of the *"Enterococcus"* genus are a frequent cause of infections such as urinary tract infections, bacteremia, bacterial endocarditis, and meningitidis.

For example, some strains of *Enterococcus,* for example faecalis and *E. faecium spp.* may be glycopeptide-resistant.

As used herein, "fungus" or "fungi" refers to eukaryotic organisms that includes microorganisms such as yeasts and molds, involved in a variety of infections. For example it may be "fungus" or "fungi selected from the group consisting such as aspergillosis, blastomycosis, candidiasis, coccidioidomycosis, cryptococcosis, histoplasmosis, ucormycosis, paracoccidioidomycosis, sporotrichosis, and pneumocystis.

As used herein, the terms "antimicrobial activity" refer to the capacity of a molecule and /or compound to inhibit the growth of or to kill microorganisms, for example pathogenic microorganisms. For example, terms "antimicrobial activity" refers comprises antiseptical activity, an antibiotic activity, an antibacterial activity, an antiviral activity, an antifungal activity, an antiprotozoal activity and/or an antiparasite activity.

As used herein, the terms "antibacterial activity" refer to the treatment and prevention of bacterial infections.

As used herein, "antifungal activity" refers to the treatment and prevention of fungal infections.

On another aspect, the invention also relates to the process of synthesis of a compound according to the invention comprising a step of reacting a compound of formula Ia : 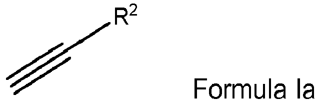 with a compound of formula Ib: 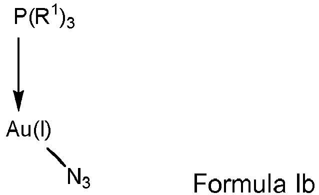 wherein R¹ and R² are defined as above.

Advantageously, the process of the invention may be carried out in an organic solvent, preferably chosen from THF or aqueous NaHCO₃, acetonitrile.

Advantageously, the process of the invention may be carried out at a temperature ranging from -10°C to 100 °C, preferably from 0 to 40°C more preferably to RT ("RT" means Room Temperature), i.e. from 20° to 30°C.

Advantageously, the process of the invention may require compound of formula Ib to be in stoichiometric excess with regards to compound Ia. From 1 to 5 equivalents of compound Ib may be used according to the process of the invention, preferably from 1 to 3 equivalents.

Advantageously, the process may further comprise a step of deprotection of any protected functional group. Functional groups that may be protected during the step of formation of the gold (I)-phosphine 1,2,3-triazole include but are not limited to -OH, -NH₂ and/or -COOH groups. They may be protected as ether, ester, carbamate, carbonate, urea and other protective groups such as described in the Green reference **[3].**

Advantageously, the process may further comprise one or more step(s) of purification. Purification may be achieved by conventional methods such as extraction, silica gel chromatography and or seim preparative reversed phase HPLC. For example, the process may comprise two purification steps such as silica gel chromatography and semi preparative reversed phase HPLC.

Additonally, the invention also relates to intermediate compounds of formula Ia: 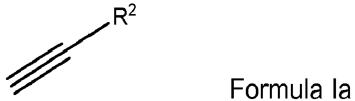 wherein R² is defined as above.

Additonally, the invention also relates to intermediate compounds of formula Ib: 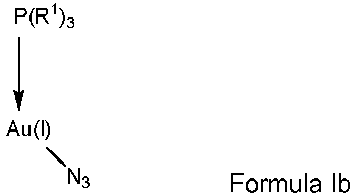 wherein R¹ is defined as above.

On another aspect, the invention relates to the compound of formula I or I' for use in a human or animal medicine.

The invention further relates to the compound of formula I or I' for use the prevention or treatment of an infection. The compounds of the invention may have antimicrobial properties.

Advantageously , the infection can have a bacterial, a fungal, a viral and/or a parasite origine. A bacterial infection may any infection involving Gram-positive bacteria, Gram-negative bacteria or non-Gram stained microorganisms, for example *Mycobacterium, for example Mycobacterium tuberculosis, Mycoplasma, for example Mycoplasma pneumonia.*

The at least one Gram-positive or Gram-negative or or non-Gram stained bacteria may be multiresistant bacteria. *Mycobacterium, for example Mycobacterium tuberculosis, Mycoplasma, for example Mycoplasma pneumonia.*

The invention generally further relates to the use of compounds according to the invention or pharmaceutical formulations or compositions thereof. The use include but are not limited to using as a drug, using for the manufacture of a medicine, using in the treatment of an infection, using for the preparation of a medicament for treating infections in a subject, using for elimination of bacterial biofilms, for preparing a medical device or implant comprising applying a coating of compound according to the invention, or placing in contact, with at least one surface of the device or implant.

As used herein the term "Biofilm" refers to complex three-dimensional structures attached to biotic or abiotic surfaces and in which the bacteria cells are embedded in a polysaccharide extracellular matrix called slime or glycocalyx. These specific structures may be formed by bacteria of the same species or of different species. In comparison with their living congeners in free (or 'planktonic') form, these bacteria are in a state of quiescence indicated by a low level of metabolic activity. Due to their reduced metabolic activity, bacteria living in a biofilm are, for example, more resistant to antibacterial treatments.

The present invention also provides a pharmaceutical composition comprising a compound according to the invention.

Advantageously , the pharmaceutical composition may comprise one or more pharmaceutically acceptable excipients such as antiadherents, binders, coating agents, disintegrants, flavouring agents, binding agents, lubricants, preservatives, sweeteners, vehicles, builking agents, fillers, thickeners, glidants, sorbents, surfactant and/or diluents, that may for instance confer a therapeutic enhancement on the active ingredient in the final dosage form, such as facilitating drug absorption, reducing viscosity or enhancing solubility.

Other adavantages may appear to the one skilled in the art from the examples.

### Brief description of the figures

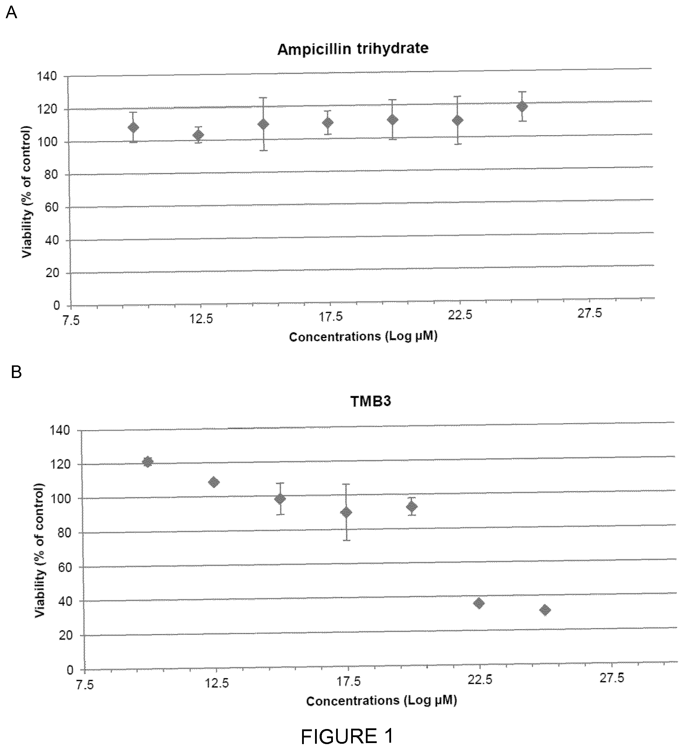

- Figure 1 is a diagram representing the TMB3 human hepatocyte cytotoxicity between 7 to 25µM (Figure 1 A) and the ampicillin human hepatocyte cytotoxicity between 7 to 25µM (control) (Figure 1B. In the diagram, the abscissa represents the concentration in Log per µM and the ordinate the viability of the cells in percentage compared to the human hepatocyte culture without ampicillin trihydrate or TMB3.

### EXAMPLES

### Examples part A : Synthesis part I

All compound and intermediate references below are in the context of part I of the examples.

All trialkylphosphine-gold(I)-azide intermediates and compounds according to the invention in the present disclosure should be understood as compounds where the gold atom is a gold(I) (oxidation state = I), even if Au is not always represented Au(I) in the following structures.

### Preparation of trialkylphosphine-gold(I)-azide intermediates

### a. Preparation of triethylphosphine-gold(I)-azide (B)

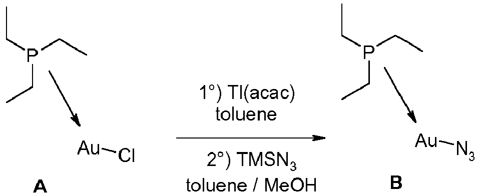

Chloro(triethylphosphine) gold (I) **A** (2.00 g, 5.70 mmol, 1.0 eq.) and thallium(I)acethylacetonate (1.77 g, 5.82 mmol, 1.02 eq.) were suspended in dry toluene (130 mL). Reaction mixture was stirred in the dark at RT for 4h. Reaction mixture was filtered through celite ® pad and washed with a minimum of toluene. Trimethylsilyl azide (0.92 g, 8.00 mmol, 1.06 mL, 1.4 eq.) was added dropwise to the filtrate, followed by dry methanol (16 mL). Reaction mixture was stirred in the dark at RT for 18h. Precipitate was removed by filtration through celite ® pad and the filtrate was evaporated under *vacuum.* Residue was precipitated with toluene/*n-*pentane (1/50). Supernatant was removed and the white precipitate was wasched 3 times with n-pentane and dried under *vacuum,* affording compound **B** (1.90 g, 92% yield) as a grey solid.

### b. Preparation of triphenylphosphine-gold(I)-azide (D)

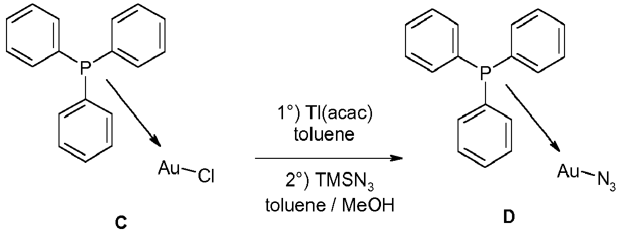

The procedure described above, with chloro(triphenylphosphine) gold (I) **C** (1.10 g, 2.22 mmol, 1.0 eq.), thallium(I)acethylacetonate (0.69 g, 2.27 mmol, 1.02 eq.) and trimethylsilyl azide (0.35 g, 3.08 mmol, 0.41 mL, 1.4 eq.), afford compound **D** (0.55 g, 50% yield) as a white/pinky solid.

### c. Preparation of dimethylphenylphosphine-gold(I)-azide (F)

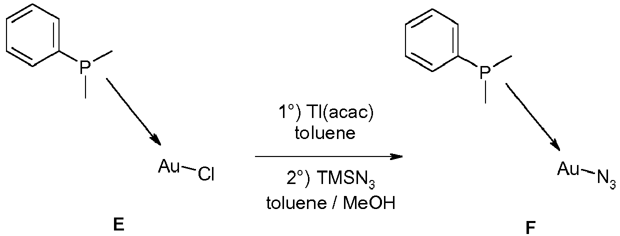

The procedure described above, with chloro(dimethylphenylphosphine) gold (I) **E** (0.25 g, 0.67 mmol, 1.0 eq.), thallium(I)acethylacetonate (0.21 g, 0.69 mmol, 1.02 eq.) and trimethylsilyl azide (0.11 g, 0.94 mmol, 0.13 mL, 1.4 eq.), afford compound **F** (0.23 g, 90% yield) as a beige solid.

### Preparation of compounds and intermediates according to the invention

### I. Preparation of TMB3

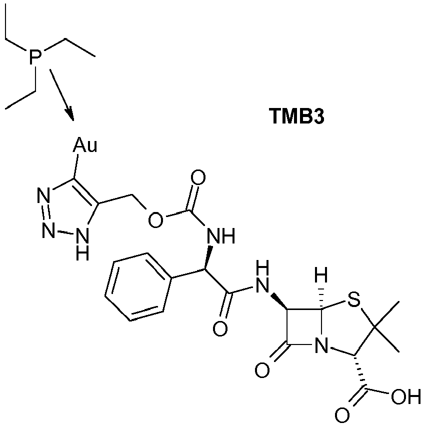

### a. Preparation of TMB2

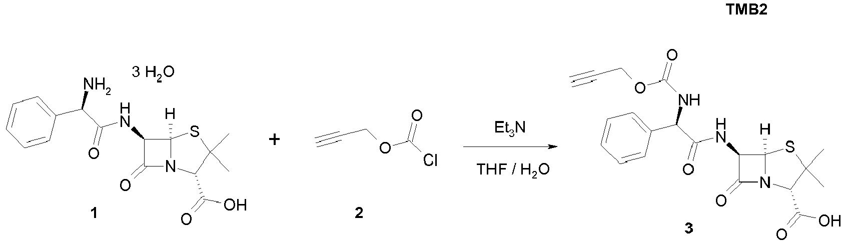

Ampicillin trihydrate **1** (1.00 g, 2.48 mmol, 1.0 eq.) was suspended in a mixture of tetrahydrofurane (10.5 mL) and water (4.5 mL). Triethylamine (0.4 mL) was added to reaction mixture in order to adjust pH (8.0) → solubilization. Reaction mixture was cooled down at 0°C, and propargylchloroformate **2** (0.29 g, 2.48 mmol, 0.24 mL, 1.0 eq.) was added dropwise. Another portion of triethylamine (0.5 mL) was added to reaction mixture in order to adjust pH (8.0) and reaction mixture was allowed to warm up to RT for 30 minutes. Tetrahydrofurane was removed under *vacuum,* water (20 mL) was added to the residue and the resulting aqueous solution was extracted with ethyl acetate (3 x 30 mL). Organics layers were discarded and aqueous layer was acidified with (1N) hydrochloric acid solution. The white precipitate was collected by filtration, affording compound **3** (0.75 g, 60% yield) as a white/yellowish solid.

### b. Preparation of TMB3

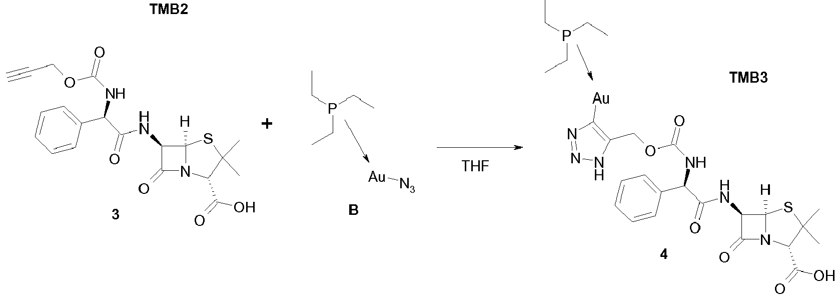

Intermediate TMB2 **3** (250 mg, 0.58 mmol, 1.0 eq.) and triethylphosphine-gold-azide **B** (414 mg, 1.16 mmol, 2.0 eq.) were suspended in degassed dry tetrahydrofurane (60 mL) and stirred in the dark at RT for 18h. Solvents were evaporated under *vacuum* and 450 mg of the residue were purified by flash chromatography [Biotage ®; column AIT ® 12g; eluant: EtOAc/ (EtOAc/H₂O/AcOH 3/1/1); gradient : 100/0 → 20/80 (20CV)] followed by a direct lyophilization, affording compound **4** (130 mg, 28% yield) as a white solid **(TMB3).** In some cases, a semi-preparative HPLC was necessary in order to obtain compound **4** with a satisfactory purity.

### II. Preparation of TMB1

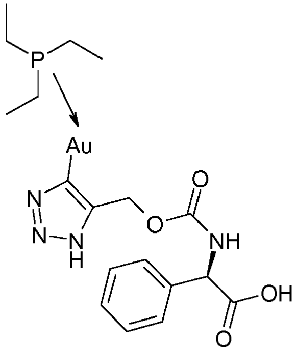

### a. Preparation of intermediate 6

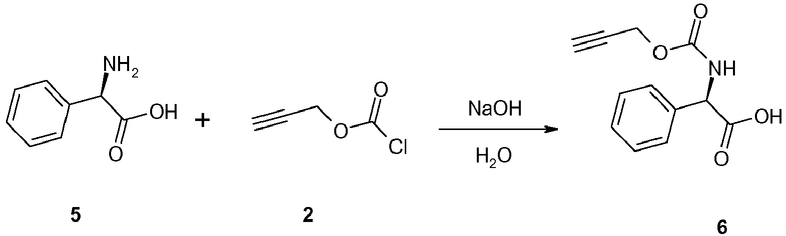

D-Phenylglycine **5** (1.00 g, 6.61 mmol, 1.0 eq.) was suspended in water (10 mL). Solubilization occurs after sodium hydroxide (1.06 g, 26.50 mmol, 4.0 eq.) addition. Reaction mixture was cooled down at 0°C, and propargylchloroformate **2** (1.57 g, 13.22 mmol, 1.29 mL, 2.0 eq.) was added dropwise. Reaction mixture was allowed to warm up to RT for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane/ EtOAc; gradient : 100/0 → 0/100 (12CV)] affording compound **6** (1.09 g, 71% yield) as a white solid.

### b. Preparation of compound 7

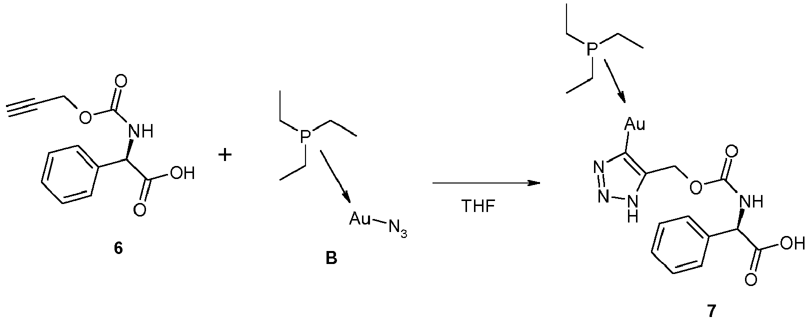

Compound **6** (250 mg, 1.07 mmol, 1.0 eq.) and triethylphosphine-gold-azide **B** (383 mg, 1.07 mmol, 1.0 eq.) were suspended in degassed dry tetrahydrofurane (20 mL) and stirred in the dark at RT for 18h. Solvents were evaporated under *vacuum* and the 250 mg of the residue was purified by flash chromatography [Biotage ®; column AIT ® 12g; eluant: EtOAc/ (EtOAc/H₂O/AcOH 3/1/1); gradient : 100/0 → 100/0 (5 CV), 100/0 → 0/100 (20 CV) then 0/100→ 0/100 (2 CV)] followed by a direct lyophilization, affording compound **7** (170 mg, 27% yield) as a white solid **(TMB1).**

### III. Preparation of TMB5

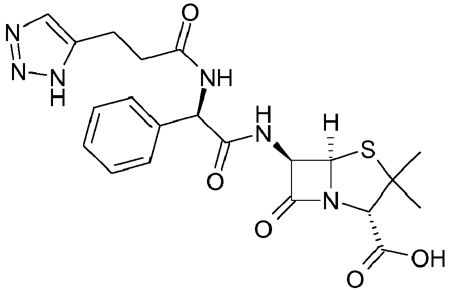

### a. Preparation of intermediate 12

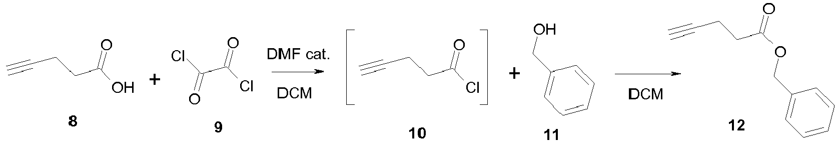

Pentynoic acid **8** (1.00 g, 10.19 mmol, 1.0 eq.) was solubilized in dichloromethane (20 mL). 3 drops of dimethylformamide, followed by oxallyl chloride **9** (1.42 g, 11.21 mmol, 0.95 mL, 1.1 eq.) were added dropwise to the reaction mixture. After 1h of stirring at RT, benzyl alcohol **11** (1.87 g, 17.32 mmol, 1.79 mL, 1.7 eq.) was added and the stirring at RT was continued for 18h. Reaction was quenched by addition of a saturated solution of NaHCO₃ (20 mL), and the resulting mixture was extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane/ EtOAc; gradient : 100/0 → 100/0 (3 CV), 100/0 → 0/100 (12 CV) then 0/100→ 0/100 (3 CV)] affording compound **12** in mixture with starting benzyl alcohol **11** (2.41 g, yield not determined) as a colorless oil which became partially solid after overtime. The mixture was used "as is" in the following step.

### b. Preparation of intermediate 14

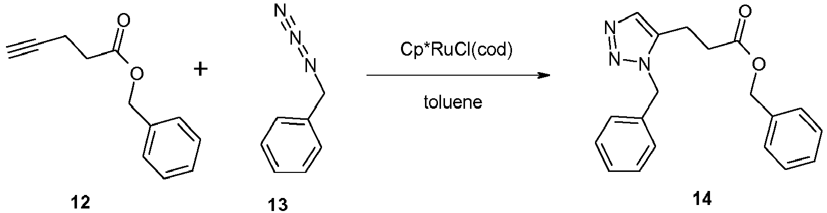

Compound **12** (in mixture with benzyl alcohol **11**) (1.20 g of the mixture, 5.10 mmol estimated, 1.0 eq.) was solubilized in toluene (20 mL). The solution was degassed by argon bubbling for 30 min. Benzyl azide **13** (1.36 g, 10.20 mmol, 1.06 mL, 2.0 eq.) was added to the reaction mixture and argon bubbling was continued for additionnal 15 min. Chloro(pentamethylcyclopentadienyl)(cyclooctadiene)ruthenium(II) (97 mg, 0.25 mmol, 0.05 eq.) was added, vessel was sealed and reaction mixture was stirred at 80°C for 18h. Reaction was cooled down to RT and solvents were evaporated under *vacuum.* The residue was purified by flash chromatography [Biotage ®; column AIT ® 80g; eluant: Cyclohexane/ EtOAc; gradient : 100/0 → 100/0 (3 CV), 100/0 → 0/100 (15 CV) then 0/100→ 0/100 (3 CV)] affording compound **14** (1.06 g, 65% yield) as a brown solid.

### c. Preparation of intermediate 15

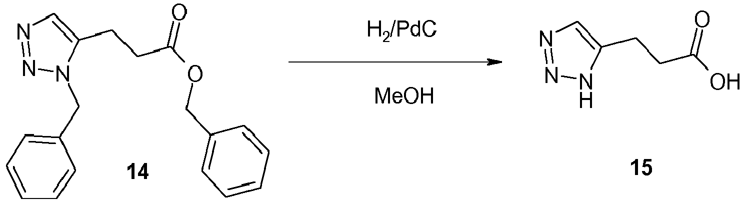

Compound **14** (1.06 g, 3.30 mmol, 1.0 eq.) was solubilized in methanol (100 mL). After Palladium (10% wet on carbon) addition, reaction mixture was hydrogenated (H₂, 5 bars) for 12h at 10-15°C. Reaction mixture was filtered through celite ® pad and washed with methanol. Solvents were evaporated under *vacuum,* affording compound **15** (460 mg, 99% yield) as a white/pinky solid.

### d. Preparation of intermediate 17

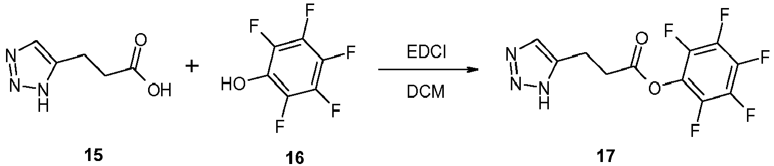

Compound **15** (380 mg, 2.62 mmol, 1.0 eq.) was solubilized in dichloromethane (10 mL). Pentafluorophenol **16** (471 mg, 2.56 mmol, 0.95 eq.) and EDCI (491 mg, 2.56 mmol, 0.95 eq.) were added to the reaction mixture and stirred at RT for 2h. Solvents were evaporated under *vacuum,* affording crude compound **17** as a sticky brownish solid which can be used "as is" in the following step. Formation of compound **17** is confirmed by LCMS.

### e. Preparation of compound 18

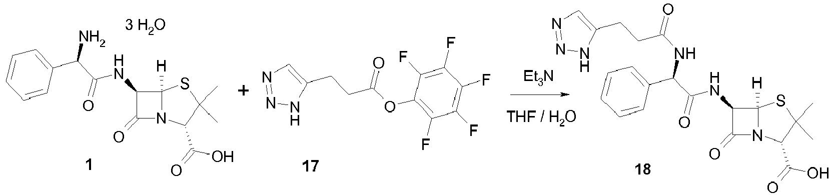

Crude pentafluorophenyl ester **17** (2.56 mmol estimated, 1.1 eq.) was suspended in tetrahydrofurane (20 mL). Ampicillin trihydrate **1** (939 mg, 2.33 mmol, 1.0 eq.) was solubilized in a mixture of tetrahydrofurane (20 mL), water (10 mL) and triethylamine (707 mg, 6.99 mmol, 0.97 mL, 3.0 eq.). Ampicillin solution was added to the pentafluorophenyl ester **17** suspension and was stirred 1h at RT. Solvents were evaporated under *vacuum,* the residual aqueous phase cooled down to 0°C and was acidified with (1N) hydrochloric acid solution. The resulting white precipitate was collected by filtration over fritté. Water was removed by trituration in toluene and solvents evaporation under *vacuum* **(x3).** The residue was purified by flash chromatography [Biotage ®; column AIT ® 40g; eluant: EtOAc/ (EtOAc/H₂O/AcOH 3/1/1); gradient : 100/0 → 100/0 (3 CV), 100/0 → 0/100 (30 CV) then 0/100→ 0/100 (3 CV)] followed by a direct lyophilization, affording compound **18** (90 mg, 8% yield) as a white solid. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done, followed by a direct lyophilization, affording compound **18** (21 mg, 2% yield) as a white solid **(TMB5).**

### IV. Preparation of TMB6

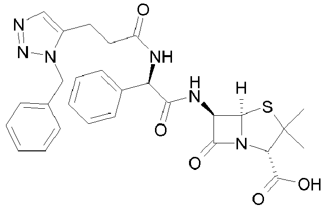

### a. Preparation of intermediate 19

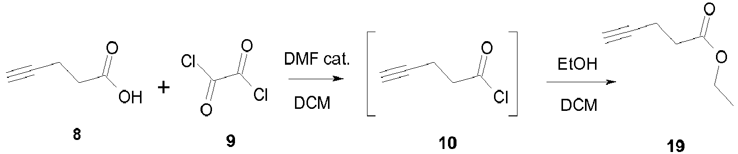

Pentynoic acid **8** (1.50 g, 15.29 mmol, 1.0 eq.) was solubilized in dichloromethane (30 mL). 3 drops of dimethylformamide, followed by oxallyl chloride **9** (2.13 g, 16.82 mmol, 1.1 mL, 1.42 mL, 1.1 eq.) were added dropwise to the reaction mixture. After 2h of stirring at RT, absolute ethanol (30 mL) was added and the stirring at RT was continued for 18h. Reaction was quenched by addition of a saturated solution of NaHCO₃ (20 mL). The resulting mixture was extracted with dichloromethane (3 x 100 mL), dried over magnesium sulfate and solvents were evaporated under *vacuum,* affording compound **19** (1.10 g, 58% yield) as a colorless oil.

### b. Preparation of intermediate 20

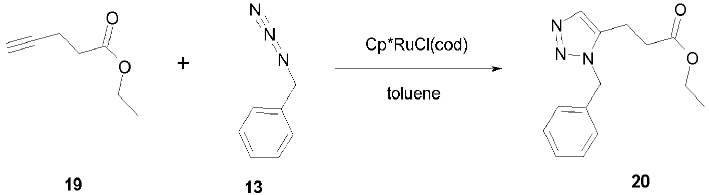

Pentynoic acid ethyl ester **19** (1.10 g, 8.72 mmol, 1.0 eq.) was solubilized in toluene (20 mL). The solution was degassed by argon bubbling for 30 min. Benzyl azide **13** (1.51 g, 11.34 mmol, 1.42 mL, 1.3 eq.) was added to the reaction mixture and argon bubbling was continued for additionnal 15 minutes. Chloro(pentamethylcyclopentadienyl) (cyclooctadiene) ruthenium(II) (166 mg, 0.44 mmol, 0.05 eq.) was added, vessel was sealed and reaction mixture was stirred at 80°C for 18h. Reaction was cooled down to RT and solvents were evaporated under *vacuum.* The residue was purified by flash chromatography [Biotage ®; column AIT ® 40g; eluant: Cyclohexane/ EtOAc; gradient : 100/0 → 100/0 (3 CV) then 100/0 → 0/100 (12 CV)] affording compound **20** (1.94 g, 86% yield) as a brown oil.

### c. Preparation of intermediate 21

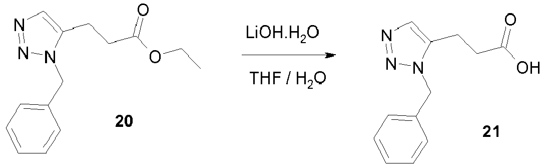

Compound **20** (1.94 g, 7.48 mmol, 1.0 eq.) was solubilized in a mixture of tetrahydrofurane (100 mL) and water (20 mL). Lithium hydroxide hydrate (470 mg, 11.22 mmol, 1.5 eq.) was added to the reaction mixture which was stirred at RT for 72h. Solvents were evaporated under *vacuum* and the resulting aqueous solution was was acidified with (1N) hydrochloric acid solution. After extraction with ethyl acetate (3 x 100 mL), drying over magnesium sulfate and solvents evaporation under *vacuum,* the residue was purified by flash chromatography [Biotage ®; column AIT ® 40g; eluant: Cyclohexane/ EtOAc; gradient : 100/0 → 0/100 (12 CV) then 0/100 → 0/100 (5 CV)] affording compound **21** (720 mg, 40% yield) as a white/yellowish powder.

### d. Preparation of intermediate 22

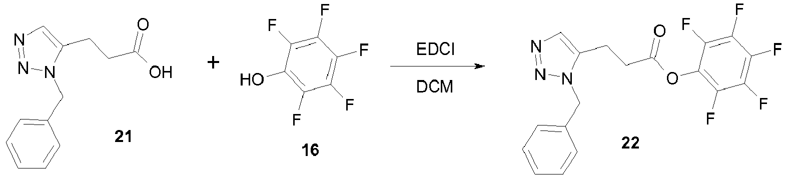

Compound **21** (440 mg, 1.90 mmol, 1.0 eq.) was solubilized in dichloromethane (10 mL). Pentafluorophenol **16** (350 mg, 1.90 mmol, 1.0 eq.) and EDCI (364 mg, 1.90 mmol, 1.0 eq.) were added to the reaction mixture and were stirred at RT for 2h. Solvents were evaporated under *vacuum,* affording crude compound **22** as a sticky brownish solid which can be used "as is" in the following step. Formation of compound **22** is confirmed by LCMS.

### e. Preparation of compound 23

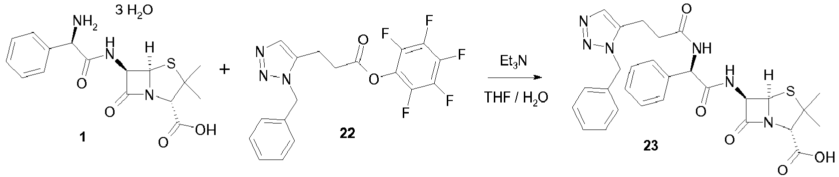

Crude pentafluorophenyl ester **22** (1.90 mmol estimated, 1.1 eq.) was suspended in tetrahydrofurane (20 mL). Ampicillin trihydrate 1 (697 mg, 1.73 mmol, 1.0 eq.) was solubilized in a mixture of tetrahydrofurane (20 mL), water (10 mL) and triethylamine (525 mg, 5.19 mmol, 0.72 mL, 3.0 eq.). Ampicillin solution was added to the pentafluorophenyl ester **22** suspension and was stirred 1h at RT. Solvents were evaporated under *vacuum,* the residual aqueous phase cooled down to 0°C and was acidified with (1N) hydrochloric acid solution. The resulting gum was isolated by supernatant removal. Water was removed by trituration in ethyl acetate/ tetrahydrofurane/ toluene mixture and solvents evaporation under *vacuum* **(x2).** The residue was purified by flash chromatography [Biotage ®; column AIT ® 12g; eluant: EtOAc/ (EtOAc/H₂O/AcOH 3/1/1); gradient : 100/0 → 100/0 (3 CV) then 100/0 → 50/50 (30 CV)] followed by a direct lyophilization, affording compound **23** (230 mg, 24% yield) as a white solid. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done on 100 mg of the residue, followed by a direct lyophilization, affording compound **23** (55 mg, 19% yield) as a white solid **(TMB6).**

### V. Preparation of TMB7

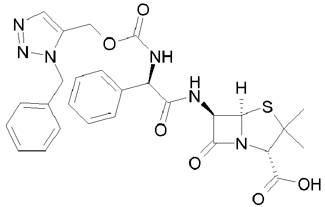

### a. Preparation of intermediate 25

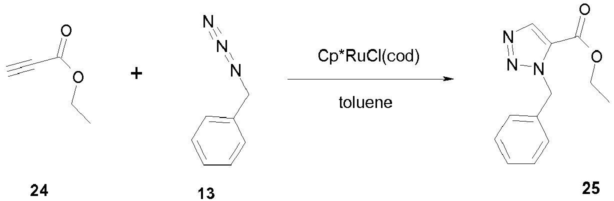

Ethyl propiolate **24** (1.10 g, 10.19 mmol, 1.0 eq.) was solubilized in toluene (20 mL). The solution was degassed by argon bubbling for 30 min. Benzyl azide **13** (1.76 g, 13.25 mmol, 1.66 mL, 1.3 eq.) was added to the reaction mixture and argon bubbling was continued for additionnal 15 min. Chloro(pentamethylcyclopentadienyl)(cyclooctadiene)ruthenium(II) (193 mg, 0.51 mmol, 0.05 eq.) was added, vessel was sealed and reaction mixture was stirred at 85°C for 24h. Reaction was cooled down to RT and solvents were evaporated under *vacuum.* The residue was purified by flash chromatography [Biotage ®; column AIT ® 80g; eluant: Cyclohexane/ EtOAc; gradient: 100/0 → 0/100 (12 CV)] affording compound **25** (480 mg, 20% yield) as a dark red oil.

### b. Preparation of intermediate 26

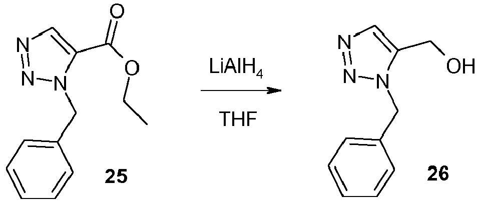

A solution of 1.0M lithium aluminium hydride in tetrahydrofurane (8.30 mmol, 8.30 mL, 4.0 eq.) was diluted in dry tetrahydrofurane (50 mL) and cooled down to -10°C, under argon atmosphere. Compound **25** (480 mg, 2.07 mmol, 1.0 eq.) was solubilized in dry tetrahydrofurane (50 mL). This solution was added dropwise to the cooled solution of lithium aluminium hydride. Reaction mixture was allowed to warm up to RT and was stirred for 2h. After cooling down at 0°C a saturated solution of Na₂SO₄ was carrefully added to the mixture. The resulting white precipitate/gum was removed by filtration over cotton pad, and the filtrate was extracted ethyl acetate (3 x 300 mL), dried over magnesium sulfate and solvents were evaporated under *vacuum,* affording compound **26** (400 mg, 99% yield) as a brown oil.

### c. Preparation of intermediate 28

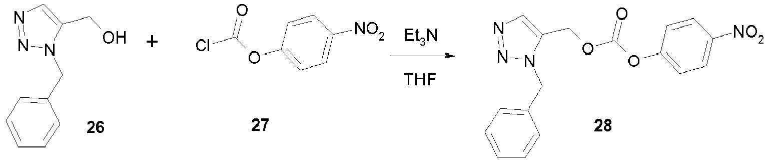

*p*-Nitrophenyl-chloroformate **27** (426 mg, 2.11 mmol, 1.0 eq.) was solubilized in tetrahydrofurane (30 mL). The solution was cooled down at 0°C under argon atmosphere. Triethylamine (427 mg, 4.22 mmol, 0.59 mL, 2.0 eq.) was added to the reaction mixture followed by compound **26** (400 mg, 2.11 mmol, 1.0 eq.) in solution in tetrahydrofurane (10 mL). Reaction mixture was stirred for 3h at RT and solvents were evaporated under *vacuum.* The residue was purified by flash chromatography [Biotage ®; column AIT ® 40g; eluant: Cyclohexane/ EtOAc; gradient : 100/0 → 100/0 (2 CV), 100/0 → 50/50 (15 CV), then 50/50 → 50/50 (3 CV)] affording compound **28** (180 mg, 24% yield) as a colorless oil.

### d. Preparation of compound 29

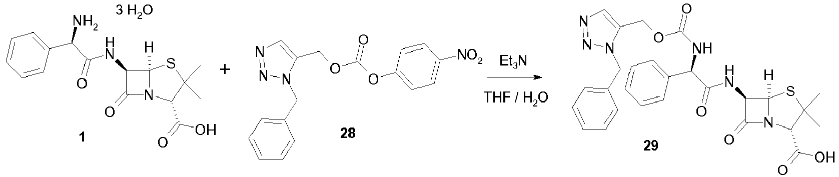

Compound **28** (180 mg, 0.51 mmol, 1.0 eq.) was suspended in tetrahydrofurane (20 mL). Ampicillin trihydrate **1** (205 mg, 0.51 mmol, 1.0 eq.) was solubilized in a mixture of tetrahydrofurane (20 mL), water (10 mL) and triethylamine (155 mg, 1.53 mmol, 0.21 mL, 3.0 eq.). Ampicillin solution was added to the compound **28** solution and was stirred 1h at RT. Solvents were evaporated under *vacuum,* the residual aqueous phase cooled down to 0°C and was acidified with (1N) hydrochloric acid solution. The resulting white precipitate was isolated by filtration over fritté. Water was removed by trituration in tetrahydrofurane/ toluene mixture and solvents evaporation under *vacuum* **(x2).** The gummy residue was purified by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) followed by a direct lyophilization, affording compound **29** (43 mg, 15% yield) as a white solid **(TMB7).**

### VI. Preparation of TMB8

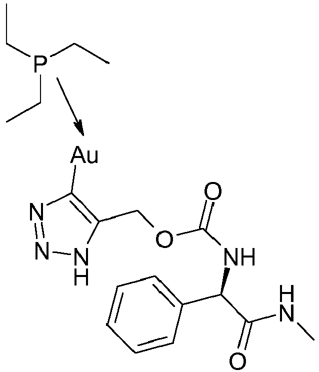

### a. Preparation of intermediate 31

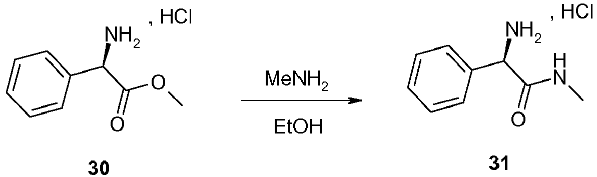

(*R*)-(-)-2-Phenylglycine methyl ester hydrochloride **30** (2.00 g, 9.92 mmol, 1.0 eq.) was stirred at RT for 18h in methylamine solution 33% in ethanol (30 mL). Solvents were evaporated under *vacuum.* To the resulting yellow/orange gum was added diethyl ether (100 mL) which was evaporated under *vacuum* **(x3).** After trituration in dichloromethane/ diethylether (1/10), the resulting precipitate was filtered over fritté to afford compound **31** (1.54 g, 77% yield) as a yellow/brownish powder.

### b. Preparation of intermediate 32

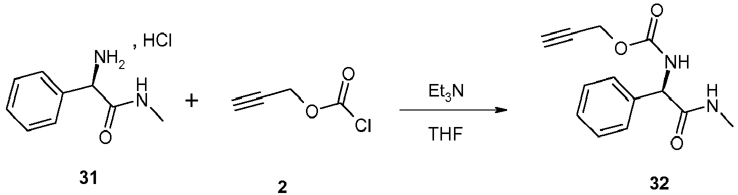

Intermediate **31** (500 mg, 2.49 mmol, 1.0 eq.) was suspended in dry tetrahydrofurane (20 mL). Dropwise addition of triethylamine (756 mg, 7.47 mmol, 1.04 mL, 3.0 eq.) followed by propargylchloroformate **2** (325 mg, 2.74 mmol, 0.27 mL, 1.1 eq.) and reaction mixture was stirred at RT for 1h. Solvents were evaporated under *vacuum,* affording a brown residue which was purified by flash chromatography [Biotage ®; column AIT ® 40g; eluant: DCM/ MeOH; gradient: 100/0 → 100/0 (2 CV), then 100/0 → 80/00 (30 CV)] affording compound **32** (100 mg, 16% yield) as a brown powder.

### c. Preparation of compound 33

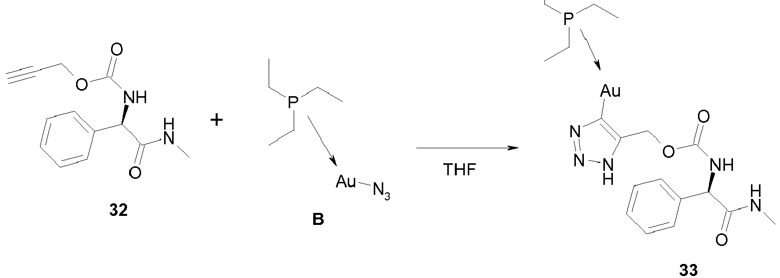

Compound **32** (100 mg, 0.41 mmol, 1.0 eq.) and triethylphosphine-gold-azide **B** (145 mg, 0.41 mmol, 1.0 eq.) were suspended in degassed dry tetrahydrofurane (25 mL) and stirred in the dark at RT for 72h. Solvents were evaporated under *vacuum,* the residue was triturated in a tetrahydrofurane/n-pentane (1/10) mixture and the white solid was collected by filtration affording compound **33** (210 mg, 85% yield). A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done on the residue, followed by a direct lyophilization, affording compound **33** (60 mg, 24% yield) as a white solid **(TMB8).**

### VII. Preparation of TMB9

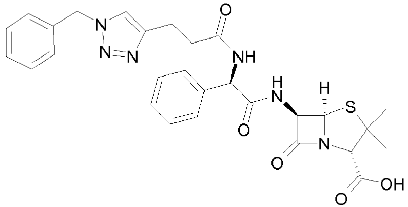

### a. Preparation of intermediate 34

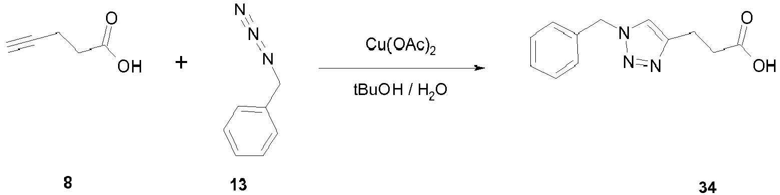

Benzyl azide **13** (1.00 g, 7.51 mmol, 0.94 mL, 1.0 eq.) and pentynoic acid **8** (810 mg, 8.26 mmol, 1.1 eq.) were solubilized in *tertio-*butyl alcohol (6.5 mL). A 0.4M aqueous solution of copper (II) acetate was prepared by solubilizing copper (II) acetate (72 mg, 0.40 mmol, 0.05 eq.) in water (1 mL). This solution was added to the reaction mixture and stirred at RT for 18h. Solvents were evaporated under *vacuum* and the residue was purified by flash chromatography [Biotage ®; column AIT ® 40g; eluant: DCM/ MeOH; gradient : 100/0 → 90/10 (12 CV)] affording compound **34** (1.10 g, 65% yield) as a white solid.

### b. Preparation of intermediate 35

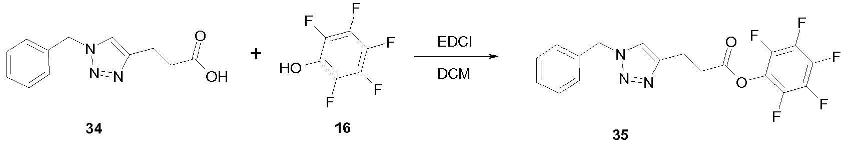

Compound **34** (1.10 g, 4.76 mmol, 1.0 eq.) was solubilized in dichloromethane (10 mL). Pentafluorophenol **16** (870 mg, 4.76 mmol, 1.0 eq.) and EDCI (910 mg, 4.76 mmol, 1.0 eq.) were added to the reaction mixture and stirred at RT for 2h. Solvents were evaporated under *vacuum,* affording crude compound **35** as a sticky brownish solid which can be used "as is" in the following step. Formation of compound **35** is confirmed by LCMS.

### c. Preparation of compound 36

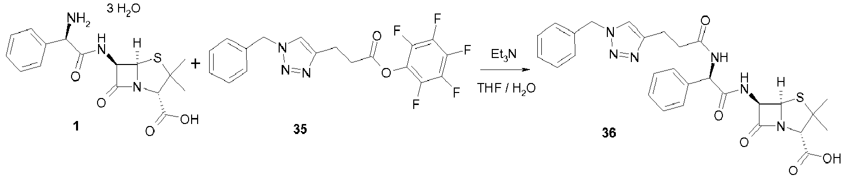

Crude pentafluorophenyl ester **35** (4.76 mmol estimated, 1.1 eq.) was suspended in tetrahydrofurane (20 mL). Ampicillin trihydrate **1** (1.74 g, 4.32 mmol, 1.0 eq.) was solubilized in a mixture of tetrahydrofurane (20 mL), water (10 mL) and triethylamine (1.31 g, 12.96 mmol, 1.81 mL, 3.0 eq.). Ampicillin solution was added to the pentafluorophenyl ester **35** suspension and was stirred for1h at RT. Solvents were evaporated under *vacuum,* the residual aqueous phase cooled down to 0°C and was acidified with (1N) hydrochloric acid solution. The resulting residue was isolated by supernatant removal. 1.6 g of the residue were purified by flash chromatography [Biotage ®; column AIT ® 40g; eluant: EtOAc/ (EtOAc/H₂O/AcOH 3/1/1); gradient : 100/0 → 100/0 (5 CV) then 100/0 → 75/25 (30 CV)] followed by a direct lyophilization, affording compound **36** (410 mg, 33% yield) as a white solid. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done on 165 mg of the residue, followed by a direct lyophilization, affording compound **36** (48 mg, 9% yield) as a white solid **(TMB9).**

### VIII. Preparation of TMB10

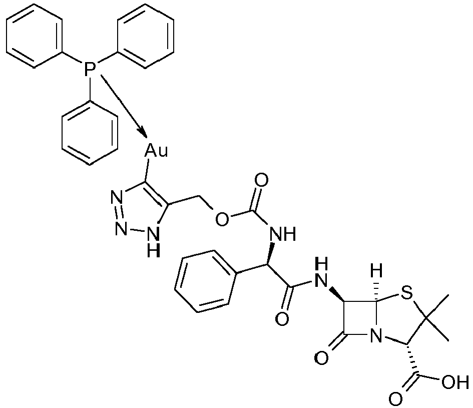

### a. Preparation of compound 37

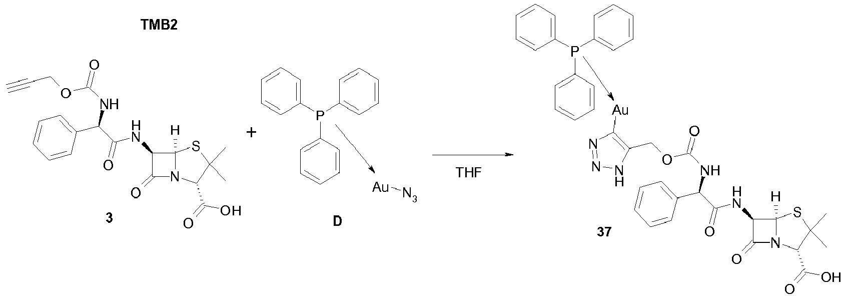

Intermediate TMB2 **3** (167 mg, 0.39 mmol, 1.0 eq.) and triphenylphosphine-gold-azide **D** (389 mg, 0.78 mmol, 2.0 eq.) were suspended in degassed dry tetrahydrofurane (40 mL) and stirred in the dark at RT for 18h. Solvents were evaporated under *vacuum* and the residue was splited in half for purification by flash chromatography [Biotage ®; column AIT ® 12g; eluant: EtOAc/ (EtOAc/H₂O/AcOH 3/1/1); gradient : 100/0 → 100/0 (5CV), then 100/0 → 50/50 (30CV)] **(x2)** followed by a direct lyophilization, affording compound **37** (220 mg, 60% yield) as a white solid. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done on the residue, followed by a direct lyophilization, affording compound **37** (58 mg, 34% yield) as a white solid **(TMB10).**

### IX. Preparation of TMB4

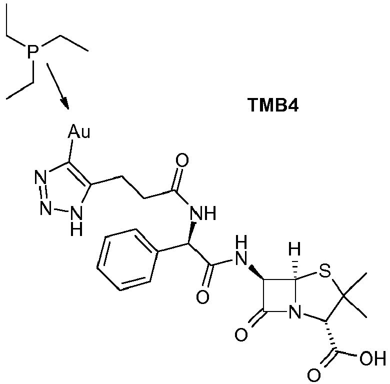

### a. Preparation of intermediate 38

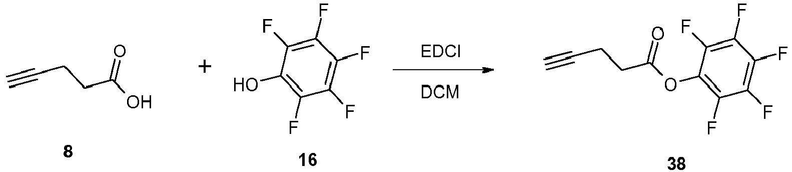

Pentynoic acid **8** (500 mg, 5.10 mmol, 1.0 eq.) was solubilized in dichloromethane (10 mL). Pentafluorophenol **16** (891 mg, 4.84 mmol, 0.95 eq.) and EDCI (928 mg, 4.84 mmol, 0.95 eq.) were added to the reaction mixture and stirred at RT for 2h. Solvents were evaporated under *vacuum,* affording crude compound **38** as a sticky brownish solid which can be used "as is" in the following step. Formation of compound **38** is confirmed by LCMS.

### b. Preparation of intermediate 39

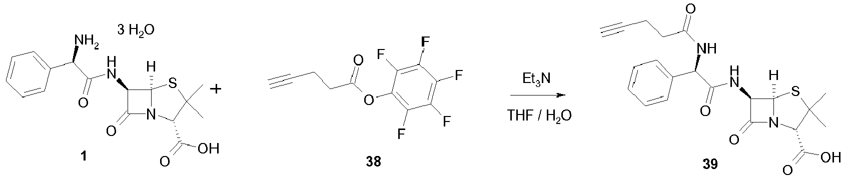

Crude pentafluorophenyl ester **38** (4.84 mmol estimated, 1.2 eq.) was suspended in tetrahydrofurane (20 mL). Ampicillin trihydrate 1 (1.51 g, 3.74 mmol, 1.0 eq.) was solubilized in a mixture of tetrahydrofurane (20 mL), water (10 mL) and triethylamine (1.13 g, 11.22 mmol, 1.56 mL, 3.0 eq.). Ampicillin solution was added to the pentafluorophenyl ester **38** suspension and was stirred for 1h at RT. Solvents were evaporated under *vacuum,* the residual aqueous phase cooled down to 0°C and was acidified with (1N) hydrochloric acid solution. The resulting precipitate was isolated by filtration over fritté, washed with water and was directly lyophilized. The residue was purified by flash chromatography [Biotage ®; column AIT ® 40g; eluant: EtOAc/ (EtOAc/H₂O/AcOH 3/1/1); gradient : 100/0 → 100/0 (5 CV) then 100/0 → 75/25 (30 CV)] followed by a direct lyophilization, affording compound **39** (380 mg, 24% yield) as a white solid.

### c. Preparation of compound 40

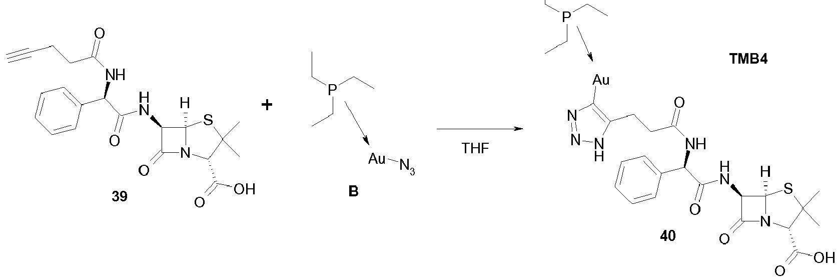

Intermediate **39** (200 mg, 0.47 mmol, 1.0 eq.) and triethylphosphine-gold-azide **B** (330 mg, 0.93 mmol, 2.0 eq.) were suspended in degassed dry tetrahydrofurane (50 mL) and stirred in the dark at RT for 18h. Solvents were evaporated under *vacuum* and the residue split in half for purification by flash chromatography [Biotage ®; column AIT ® 12g; eluant: EtOAc/ (EtOAc/H₂O/AcOH 3/1/1); gradient : 100/0 → 100/0 (5 CV) then 100/0 → 50/50 (30 CV)] **(x2)** followed by a direct lyophilization, affording compound **40** (140 mg, 38% yield) as a white solid. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done on the residue, followed by a direct lyophilization, affording compound **40** (11 mg, 3% yield) as a white solid **(TMB4).**

### X. Preparation of TMB11

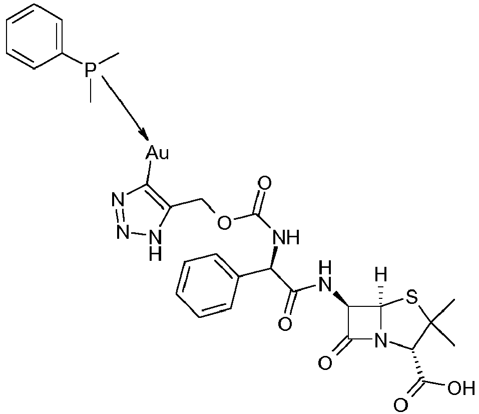

### a. Preparation of compound 41

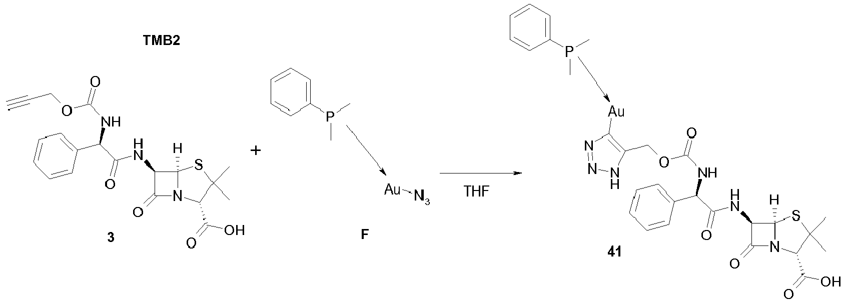

IntermediateTMB2 **3** (131 mg, 0.30 mmol, 1.0 eq.) and dimethylphenylphosphine-gold-azide F (229 mg, 0.61 mmol, 2.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 20g; eluant: EtOAc/ (EtOAc/H₂O/AcOH 3/1/1); gradient : 100/0 → 20/80 (15CV)] followed by a direct lyophilization, affording compound **41** (94 mg, 39% yield) as a white solid **(TMB11).**

### XI. Preparation of TMB12

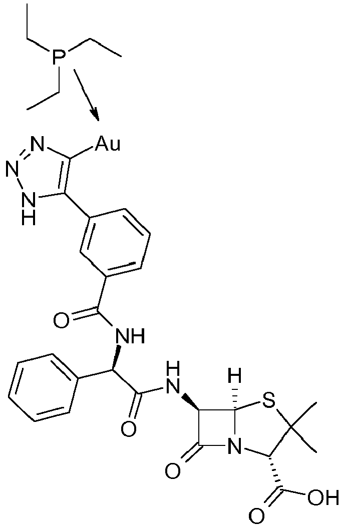

### a. Preparation of intermediate 43

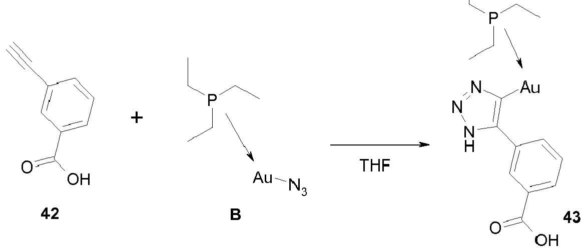

3-Ethynylbenzoic acid **42** (670 mg, 4.58 mmol, 1.0 eq.) and triethylphosphine-gold-azide **B** (1.64 g, 4.58 mmol, 1.0 eq.) were suspended in degassed dry tetrahydrofurane (50 mL) and stirred in the dark at RT for 18h. Solvents were evaporated under *vacuum* and the residue was split in half for purification by flash chromatography [Biotage ®; column AIT ® 12g; eluant: EtOAc/ (EtOAc/H₂O/AcOH 3/1/1); gradient : 100/0 → 100/0 (5 CV) then 100/0 → 50/50 (25 CV)] **(x2)** followed by a direct lyophilization, affording compound **43** (985 mg, 43% yield) as a white solid.

### b. Preparation of intermediate 44

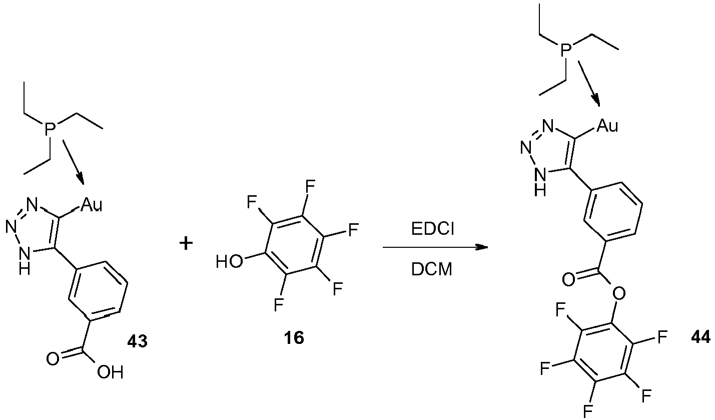

Intermediate **43** (460 mg, 0.91 mmol, 1.0 eq.) was solubilized in dichloromethane (10 mL). Pentafluorophenol **16** (160 mg, 0.87 mmol, 0.95 eq.) and EDCI (166 mg, 0.87 mmol, 0.95 eq.) were added to the reaction mixture and stirred at RT for 2h. Solvents were evaporated under *vacuum,* affording crude compound **44** as a sticky yellow oil which can be used "as is" in the following step. Formation of compound **44** is confirmed by LCMS.

### c. Preparation of compound 45

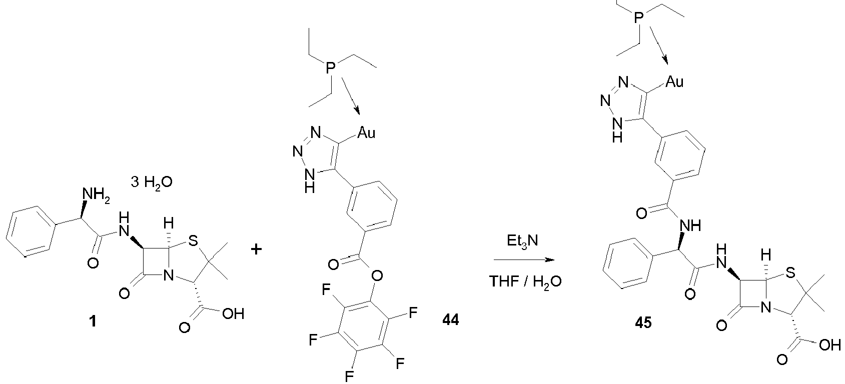

Crude pentafluorophenyl ester **44** (0.91 mmol estimated, 1.0 eq.) was suspended in tetrahydrofurane (100 mL). Ampicillin trihydrate **1** (512 mg, 1.27 mmol, 1.4 eq.) was solubilized in a mixture of tetrahydrofurane (10 mL), water (5 mL) and triethylamine (276 mg, 2.73 mmol, 0.38 mL, 3.0 eq.). Ampicillin solution was added to the pentafluorophenyl ester **44** suspension and was stirred 2h at RT. Solvents were evaporated under *vacuum,* and the residual aqueous phase cooled down to 0°C and was acidified with (1N) hydrochloric acid solution. The resulting precipitate was isolated by supernatant removal, washed with water and directly lyophilized. 200 mg of the residue were purified by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) followed by a direct lyophilization, affording compound **45** (14 mg, 6% yield) as a white solid **(TMB12).**

### XII. Preparation of TMB13

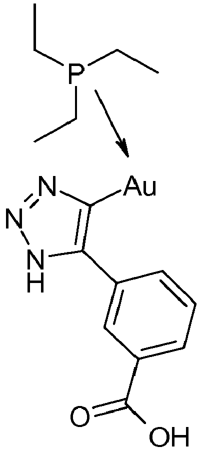

### See procedure for preparation of the intermediate 43

### XIII. Preparation of TMB14

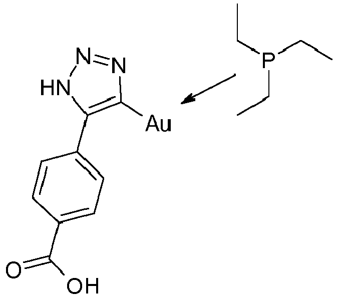

### a. Preparation of compound 47

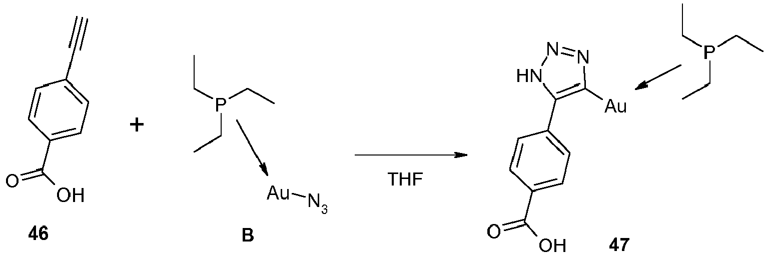

4-Ethynylbenzoic acid **46** (350 mg, 2.40 mmol, 1.0 eq.) and triethylphosphine-gold-azide **B** (855 mg, 2.40 mmol, 1.0 eq.) were suspended in degassed dry tetrahydrofurane (70 mL) and stirred in the dark at RT for 18h. Solvents were evaporated under *vacuum,* the residue was triturated in a DCM/THF/*n*-pentane (1/1/10) mixture and the supernatant was removed. The resulting residue was purified by flash chromatography [Biotage ®; column AIT ® 12g; eluant: EtOAc/ (EtOAc/H₂O/AcOH 3/1/1); gradient : 100/0 → 100/0 (5 CV) then 100/0 → 50/50 (25 CV)] followed by a direct lyophilization, affording compound **47** (170 mg, 11% yield) as a white solid **(TMB14).**

### XIV. Preparation of TMB15

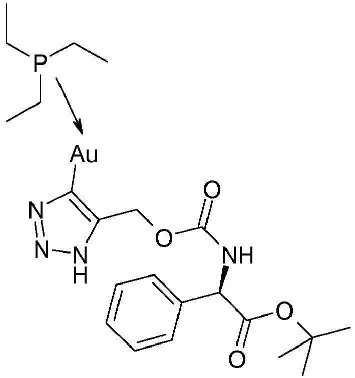

### a. Preparation of intermediate 49

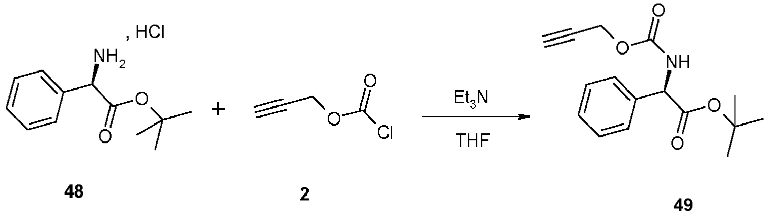

*tertio-*Butyl ester D-phenylglycine hydrochloride **48** (500 mg, 2.05 mmol, 1.0 eq.) was suspended in tetrahydrofurane (30 mL). Solubilization occurs after triethylamine addition (830 mg, 8.20 mmol, 1.14 mL, 4.0 eq.). Propargylchloroformate **2** (486 mg, 4.10 mmol, 0.40 mL, 2.0 eq.) was added dropwise and reaction mixture was stirred at RT for 3h. Reaction mixture was filtered throught cotton pad and solvents were evaporated under *vacuum.* The residue was purified by flash chromatography [Biotage ®; column AIT ® 12g; eluant: Cyclohexane/ EtOAc; gradient : 100/0 → 100/0 (5CV), 100/0 → 50/50 (15CV), then 50/50 → 50/50 (2CV)] affording compound **49** (460 mg, 78% yield) as a colorless oil.

### b. Preparation of compound 50

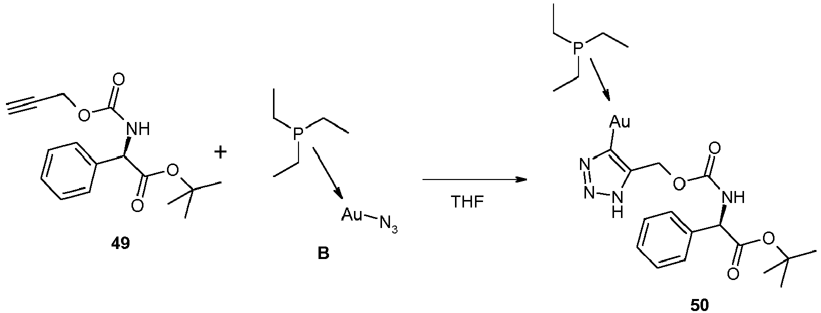

Compound **49** (250 mg, 0.86 mmol, 1.0 eq.) and triethylphosphine-gold-azide **B** (307 mg, 0.86 mmol, 1.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 40g; eluant: EtOAc/ (EtOAc/H₂O/AcOH 3/1/1); gradient : 100/0 → 100/0 (5CV), then 100/0 → 40/60 (25CV)] affording compound **50** (160 mg, 29% yield) as a white powder **(TMB15).**

### XV. Preparation of TMB16

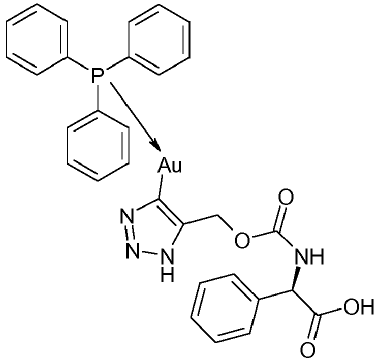

### a. Preparation of compound 51

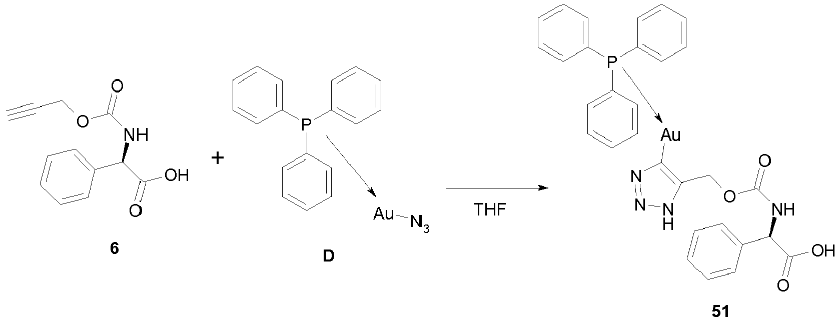

Compound **6** (250 mg, 1.07 mmol, 1.0 eq.) and triphenylphosphine-gold-azide **D** (536 mg, 1.07 mmol, 1.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 40g; eluant: EtOAc/ (EtOAc/H₂O/AcOH 3/1/1); gradient : 100/0 → 100/0 (5 CV), then 100/0 → 40/60 (25 CV)] followed by a direct lyophilization, affording compound **51** (437 mg, 56% yield) as a white solid **(TMB16).**

### XVI. Preparation of TMB17

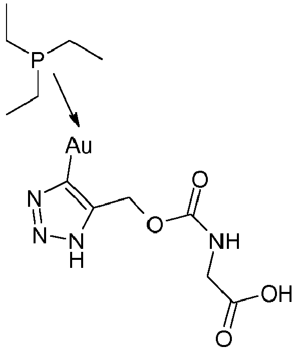

### a. Preparation of intermediate 53

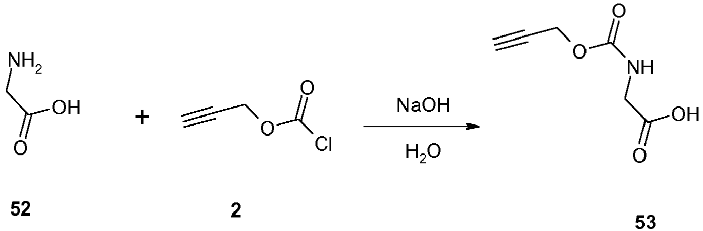

Glycine **52** (1.0 g, 13.32 mmol, 1.0 eq.) was suspended in water (10 mL). Solubilization occurs after sodium hydroxide (2.13 g, 53.28 mmol, 4.0 eq.) addition. Reaction mixture was cooled down at 0°C, propargylchloroformate **2** (3.16 g, 26.64 mmol, 2.60 mL, 2.0 eq.) was added dropwise and reaction mixture was allowed to warm up to RT for 30 minutes. Reaction mixture was acidified with (12N) hydrochloric acid solution, extracted with ethyl acetate (3 x 100 mL), dried over magnesium sulfate and solvents were evaporated under *vacuum,* affording crude intermediate **53** (2.14 g, 99% yield) as a colorless/pinky solid which can be used in the next step without purification.

### b. Preparation of intermediate 54

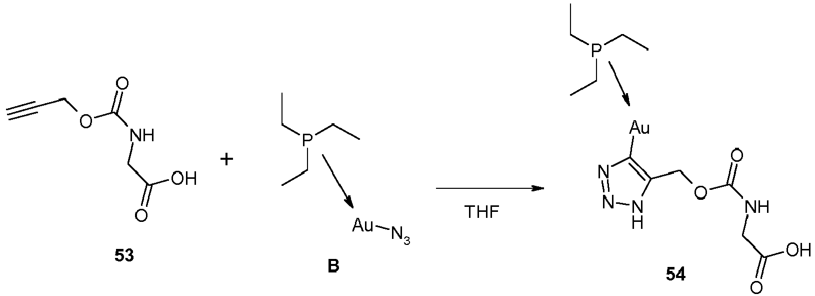

Compound **53** (200 mg, 1.27 mmol, 1.0 eq.) and triethylphosphine-gold-azide **B** (454 mg, 1.27 mmol, 1.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 12g; eluant: EtOAc/ (EtOAc/H₂O/AcOH 3/1/1); gradient : 100/0 → 100/0 (5CV), 100/0 → 80/20 (20CV), then 80/20 → 80/20 (5CV)], followed by a direct lyophilization, affording compound **54** (110 mg, 17% yield) as a white powder. A second purification by semi-preparative HPLC was done (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) followed by a direct lyophilization, affording compound **54** (30 mg, 5% yield) as a white powder **(TMB18).**

### XVII. Preparation of TMB19

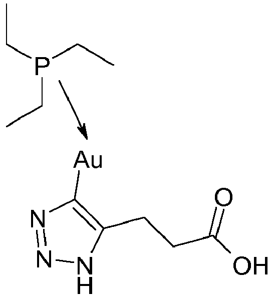

### a. Preparation of compound 55

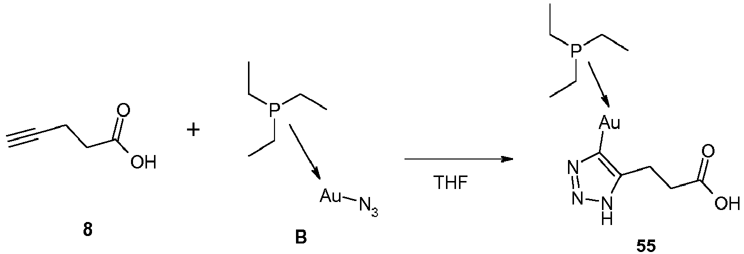

Pentynoic acid **8** (150 mg, 1.53 mmol, 1.0 eq.) and triethylphosphine-gold-azide **B** (546 mg, 1.53 mmol, 1.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 12g; eluant: EtOAc/ (EtOAc/H₂O/AcOH 3/1/1); gradient : 100/0 → 100/0 (5CV), then 100/0 → 50/50 (12CV)] followed by a direct lyophilization, affording compound **55** (270 mg, 39% yield) as a white powder **(TMB19).**

### XVIII. Preparation of TMB20

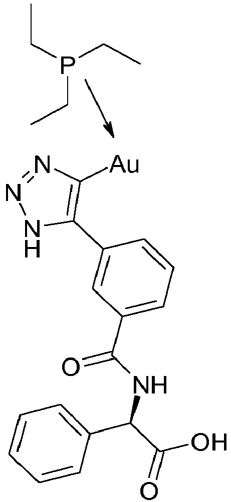

### a. Preparation of compound 56

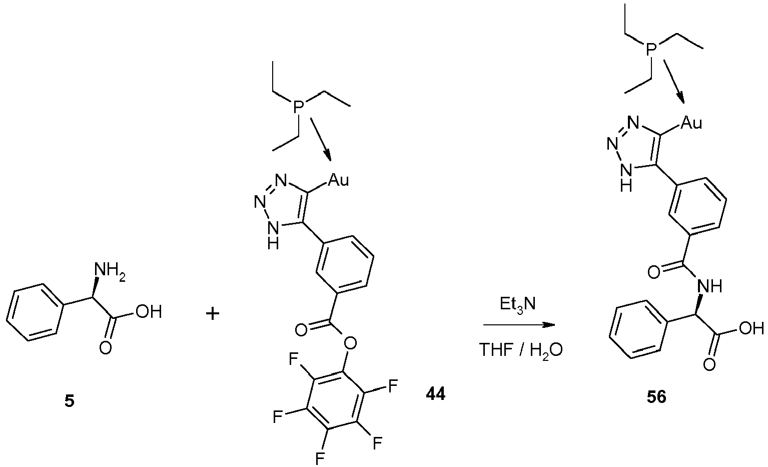

Crude pentafluorophenyl ester **44** (0.94 mmol estimated, 1.0 eq.) was suspended in tetrahydrofurane (20 mL). D-Phenylglycine 5 (142 mg, 0.94 mmol, 1.0 eq.) was solubilized in a mixture of tetrahydrofurane (20 mL), water (10 mL) and triethylamine (285 mg, 2.82 mmol, 0.39 mL, 3.0 eq.). D-Phenylglycine solution was added to the pentafluorophenyl ester **44** suspension and was stirred 2h at RT. Solvents were evaporated under *vacuum,* and the residual aqueous phase cooled down to 0°C and was acidified with (1N) hydrochloric acid solution. Solvents were evaporated under *vacuum* and the residue was purified by flash chromatography [Biotage ®; column AIT ® 40g; eluant: EtOAc/ (EtOAc/H₂O/AcOH 3/1/1); gradient: 100/0 → 100/0 (5CV), then 100/0 → 40/60 (25CV)] followed by a direct lyophilization, affording compound **56** (60 mg, 10% yield) as a white powder. A second purification by semi-preparative HPLC was done (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) followed by a direct lyophilization, affording compound **56** (10 mg, 2% yield) as a white powder **(TMB20).**

### XIX. Preparation of TMB21

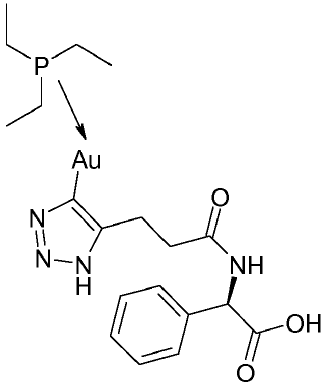

### a. Preparation of intermediate 59

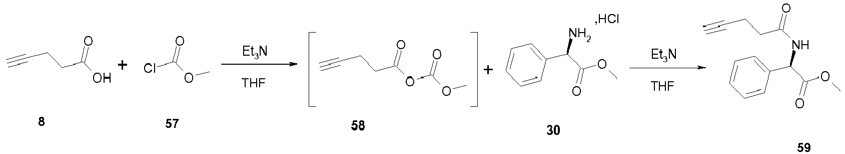

Pentynoic acid **8** (1.00 g, 10.19 mmol, 1.0 eq.) was solubilized in tetrahydrofurane (20 mL) and cooled down at -10°C under argon atmosphere. Triethylamine (3.09 g, 30.57 mmol, 4.26 mL, 3.0 eq.) was added dropwise, followed by methylchloroformate **57** (1.06 g, 11.21 mmol, 0.87 mL, 1.1 eq.). Reaction mixture was stirred at -10°C for 1h. (R)-(-)-2-Phenylglycine methyl ester hydrochloride **30** (2.05 g, 10.19 mmol, 1.0 eq.) was added portionwise to the reaction mixture and the stirring was continued for 18h at RT. Solvents were evaporated under *vacuum,* water (50 mL) was added to the residue and the resulting aqueous mixture was extracted with ethyl acetate (3 x 100 mL). Organic phase was dried over magnesium sulfate and solvents were evaporated under *vacuum.* The residue was purified by flash chromatography [Biotage ®; column AIT ® 40g; eluant: Cyclohexane/ EtOAc; gradient : 100/0 → 100/0 (3CV), 100/0 → 0/100 (15CV), then 0/100 → 0/100 (3CV)] affording compound **59** (1.83 g, 73% yield) as a colorless oil.

### b. Preparation of intermediate 60

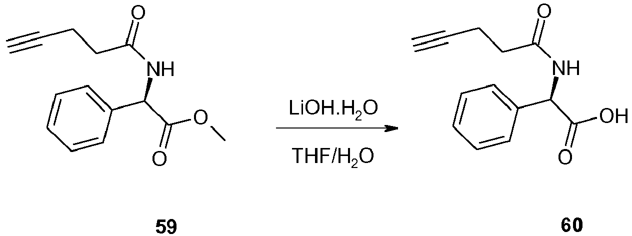

Compound **59** (1.83 g, 7.46 mmol, 1.0 eq.) was solubilized in a mixture of tetrahydrofurane (20 mL) and water (4 mL). Lithium hydroxide hydrate (626 mg, 14.92 mmol, 2.0 eq.) was added to the reaction mixture was stirred at RT for 1h. Solvents were evaporated under *vacuum* and the resulting aqueous solution was was acidified with (1N) hydrochloric acid solution. After extraction with ethyl acetate (3 x 100 mL), drying over magnesium sulfate and solvents evaporation under *vacuum,* the residue was purified by flash chromatography [Biotage ®; column AIT ® 40g; eluant: Cyclohexane/ EtOAc; gradient : 100/0 → 100/0 (3CV), 100/0 → 0/100 (12 CV) then 0/100 → 0/100 (3 CV)] affording compound **60** (1.25 g, 72% yield) as a white powder.

### c. Preparation of compound 61

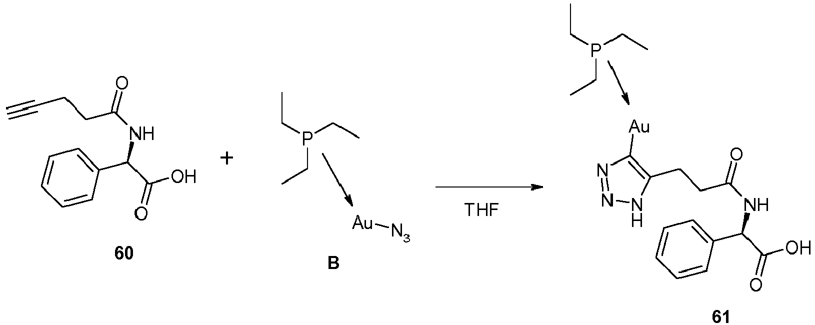

Compound **60** (250 mg, 1.08 mmol, 1.0 eq.) and triethylphosphine-gold-azide **B** (771 mg, 2.16 mmol, 2.0 eq.) were suspended in degassed dry tetrahydrofurane (20 mL) and stirred in the dark at RT for 18h. Solvents were evaporated under *vacuum* and the residue was purified by flash chromatography [Biotage ®; column AIT ® 40g; eluant: EtOAc/ (EtOAc/H₂O/AcOH 3/1/1); gradient : 100/0 → 100/0 (4 CV), 100/0 → 80/20 (15CV) then 80/20 → 80/20 (2 CV)] followed by a direct lyophilization, affording compound **61** (200 mg, 31% yield) as a white solid **(TMB21).**

### XX. Preparation of TMB22

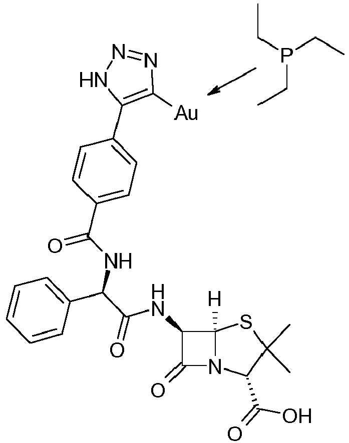

### a. Preparation of intermediate 62

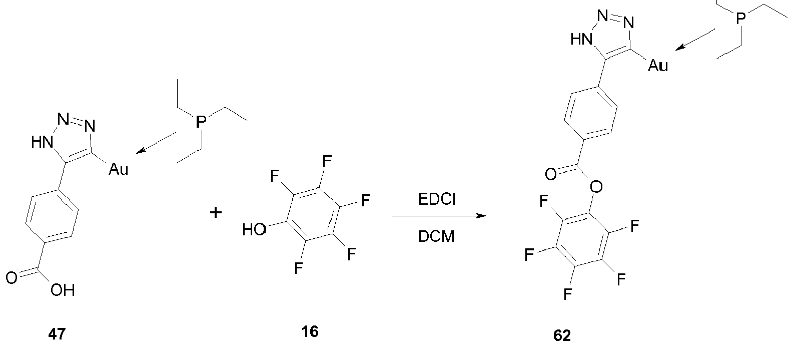

Intermediate **47** (300 mg, 0.60 mmol, 1.0 eq.) was solubilized in dichloromethane (30 mL). Pentafluorophenol **16** (104 mg, 0.57 mmol, 0.95 eq.) and EDCI (109 mg, 0.57 mmol, 0.95 eq.) were added to the reaction mixture and stirred at RT for 18h. Solvents were evaporated under *vacuum,* affording crude compound **62** as a sticky yellow oil which can be used "as is" in the following step. Formation of compound **62** is confirmed by LCMS.

### b. Preparation of compound 63

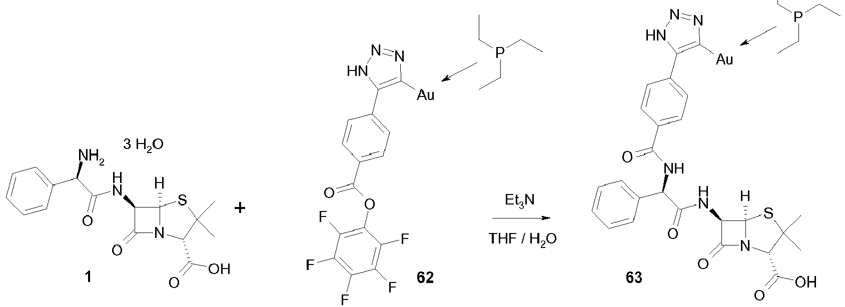

Crude pentafluorophenyl ester **62** (0.60 mmol estimated, 1.0 eq.) was suspended in tetrahydrofurane (12 mL). Ampicillin trihydrate **1** (475 mg, 1.18 mmol, 2.0 eq.) was solubilized in a mixture of tetrahydrofurane (12 mL), water (6 mL) and triethylamine (238 mg, 2.35 mmol, 0.32 mL, 3.9 eq.). Ampicillin solution was added to the pentafluorophenyl ester **62** suspension and was stirred 3h at RT. Solvents were evaporated under *vacuum,* the residual aqueous phase cooled down to 0°C and was acidified with (1N) hydrochloric acid solution. The resulting yellow gum was isolated by supernatant removal and directly lyophilized. The residue was purified by flash chromatography [Biotage ®; column AIT ® 12g; eluant: Cyclohexane/ EtOAc; gradient : 100/0 → 35/65 (12 CV)] affording compound **63** (90 mg, 10% yield) as a white powder. A second purification 3by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done, followed by a direct lyophilization, affording compound **63** (6 mg, 1% yield) as a white solid **(TMB22).**

### XXI. Preparation of TMB23

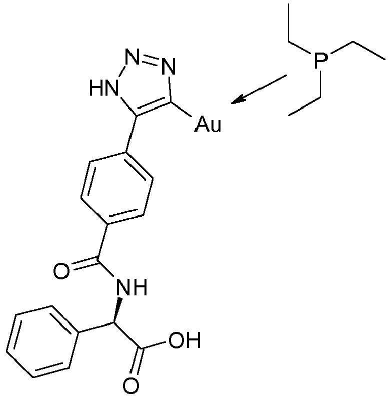

### a. Preparation of intermediate 64

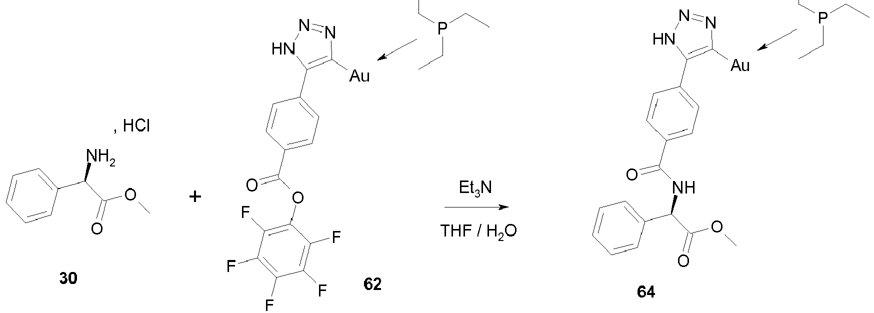

Crude pentafluorophenyl ester **62** (0.34 mmol estimated, 1.0 eq.) was suspended in tetrahydrofurane (20 mL). (*R*)-(-)-2-Phenylglycine methyl ester hydrochloride **30** (83 mg, 0.41 mmol, 1.2 eq.) was solubilized in a mixture of tetrahydrofurane (20 mL) and triethylamine (285 mg, 1.02 mmol, 0.39 mL, 3.0 eq.). (*R*)-(-)-2-Phenylglycine methyl ester hydrochloride **30** solution was added to the pentafluorophenyl ester **62** suspension and was stirred 18h at RT. Reaction mixture was filtetred through cotton pad and solvents were evaporated under *vacuum.* The residue was purified by flash chromatography [Biotage ®; column AIT ® 12g; eluant: EtOAc/ (EtOAc/H₂O/AcOH 3/1/1); gradient : 100/0 → 100/0 (5CV), then 100/0 → 75/25 (7CV)] followed by a direct lyophilization, affording compound **64** (150 mg, 68% yield) as a white powder.

### b. Preparation of compound 65

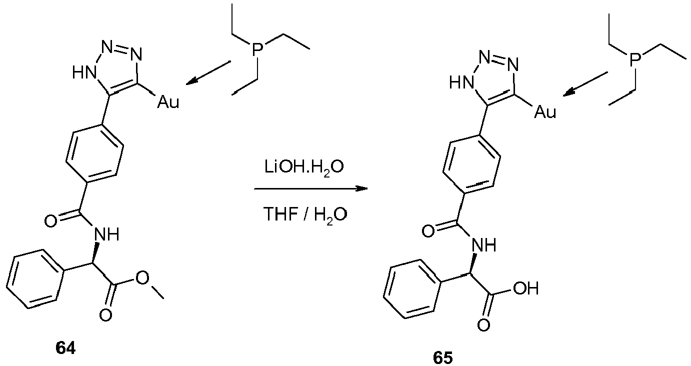

Compound **64** (150 mg, 0.23 mmol, 1.0 eq.) was solubilized in a mixture of tetrahydrofurane (20 mL) and water (2 mL). Lithium hydroxide hydrate (29 mg, 0.69 mmol, 3.0 eq.) was added to the reaction mixture was stirred at RT for 18h. Solvents were evaporated under *vacuum* and the resulting aqueous solution was was acidified with (1N) hydrochloric acid solution. The resulting acidic aqueous phase was directly purified by flash chromatography [Biotage ®; column AIT ® 12g; eluant: eluant: EtOAc/ (EtOAc/H₂O/AcOH 3/1/1); gradient : 100/0 → 100/0 (5CV), 100/0 → 60/40 (9CV) then 60/40 → 60/40 (2CV)] followed by a direct lyophilization, affording compound **65** (40 mg, 27% yield) as a white powder. A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done, followed by a direct lyophilization, affording compound **65** (11 mg, 7% yield) as a white solid **(TMB23).**

### XXII. Preparation of TMB24

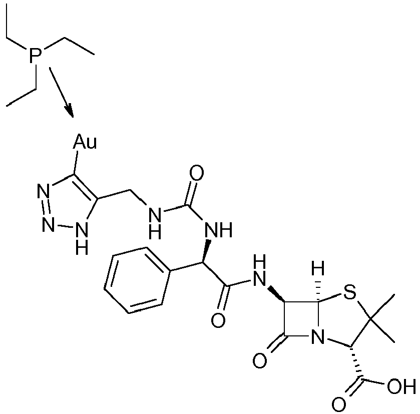

### a. Preparation intermediate 67

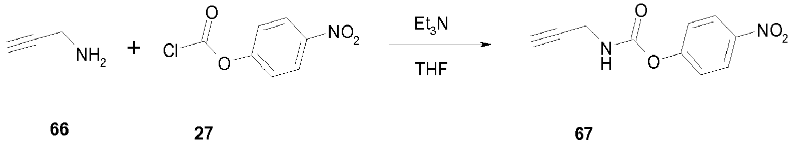

Propargylamine **66** (1.00 g, 18.2 mmol, 1.16 mL, 1.0 eq.) was solubilized in tetrahydrofurane (30 mL) and cooled down at 0°C under argon atmosphere. A solution of p-nitrophenyl-chloroformate **27** (3.7 g, 18.2 mmol, 1.0 eq.) in tetrahydrofurane (10 mL) was added dropwise to reaction mixture and was allowed to warm-up to RT. Stirring was continued for 3h. Solvents were removed under *vacuum,* and the residue was purified by flash chromatography [Biotage ®; column AIT ® 80g; eluant: Cyclohexane/ EtOAc; gradient : 100/0 → 55/45 (12CV), then 55/45 → 20/80 (12CV)] affording compound **67** in mixture with residual p-nitrophenol (1/1) (3.3 g) as a yellow powder.

### b. Preparation intermediate 68

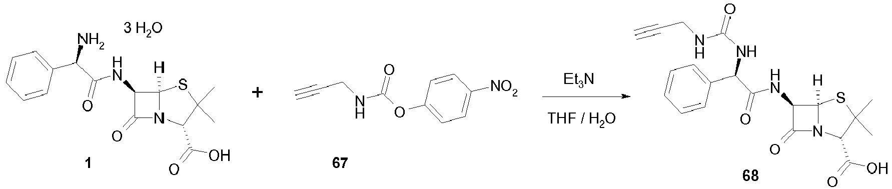

Ampicillin trihydrate **1** (1.29 g, 3.20 mmol, 1.0 eq.) was suspended in a mixture of tetrahydrofurane (20 mL), water (10 mL) and triethylamine (0.97 g, 9.60 mmol, 1.34 mL, 3.0 eq.). Mixture of compound **67** and *p-*nitrophenol (1/1) (1.50 g, 6.40 mmol estimated, 2.0 eq. estimated) was solubilized in tetrahydrofurane (10 mL) and added dropwise to the ampicillin solution. The mixture was stirred for 2h at RT. Solvents were evaporated under *vacuum* and the resulting aqueous solution was was acidified with (1N) hydrochloric acid solution. The precipitate was isolated by supernatant removal and was purified by flash chromatography [Biotage ®; column AIT ® 40g; eluant: EtOAc/ (EtOAc/H₂O/AcOH 3/1/1); gradient : 100/0 → 100/0 (5CV), then 100/0 → 60/40 (17CV)] affording compound **68** (1.04 g, 78% yield) as a white powder.

### c. Preparation of compound 69

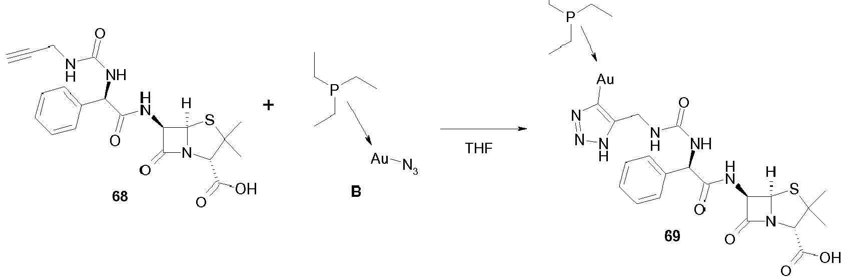

Intermediate **68** (600 mg, 1.40 mmol, 1.0 eq.) and triethylphosphine-gold-azide **B** (1.0 g, 2.80 mmol, 2.0 eq.) were suspended in degassed dry tetrahydrofurane (70 mL) and stirred in the dark at RT for 18h. Yellowish precipitate was collected by filtration and was purified by flash chromatography [Biotage ®; column AIT ® 40g; eluant: EtOAc/ (EtOAc/H₂O/AcOH 3/1/1); gradient : 100/0 → 100/0 (5CV), 100/0 → 25/75 (20CV), then 25/75 → 25/75 (10CV)] followed by a direct lyophilization, affording compound **69** (156 mg, 14% yield). A second purification by semi-preparative HPLC (Gilson PLC 2020, column C8 Phenomenex 10µm PF-C18HP, gradient: water/acetonitrile (0.1% HCOOH) 82/18 → 82/18, 10 minutes and 82/18 → 0/100, 40 minutes) was done, followed by a direct lyophilization, affording compound **69** (41 mg, 4% yield) as a white solid **(TMB24).**

### Example part B : Synthesis part II

### Preparation of trialkylphosphine-gold-azide intermediates

### a. Preparation of triethylphosphine-gold-azide (B)

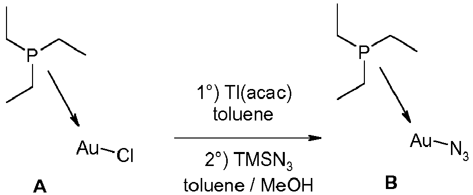

Chloro(triethylphosphine) gold (I) **A** (5.00 g, 14.3 mmol, 1.00 eq.) and thallium(I)acethylacetonate (4.41 g, 14.5 mmol, 1.02 eq.) were suspended in dry toluene (330 mL). Reaction mixture was stirred in the dark at room temperature for 5h. Reaction mixture was filtered through celite ® pad and washed with a minimum of toluene. Trimethylsilyl azide (2.30 g, 20.0 mmol, 2.65 mL, 1.40 eq.) was added dropwise to the filtrate, followed by dry methanol (40 mL). Reaction mixture was stirred in the dark at room temperature for 18h. Precipitate was removed by filtration through celite ® pad and the filtrate was concentrated under *vacuum.* Residue was triturated in cold n-pentane. Supernatant was removed and the white precipitate washed 3 times with cold n-pentane and dried under *vacuum,* affording compound **B** (4.80 g, 94% yield) as a pale yellow solid.

### b. Preparation of triisopropylphosphine-gold-azide (D)

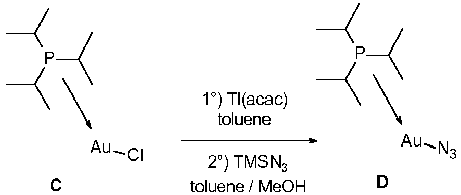

Following the procedure described above, with chloro(triisopropylphosphine) gold (I) **C** (547 mg, 1.39 mmol, 1.00 eq.), thallium(I)acethylacetonate (431 mg, 1.42 mmol, 1.02 eq.) and trimethylsilyl azide (226 mg, 1.95 mmol, 0.26 mL, 1.40 eq.), afford compound **D** (497 mg, 89% yield) as a white solid.

### c. Preparation of tricyclohexylphosphine-gold-azide (D)

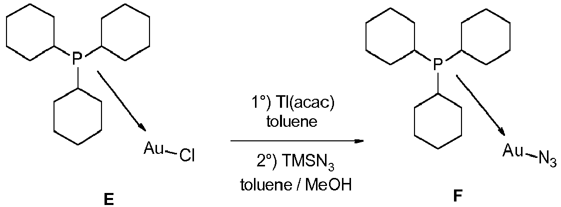

Following the procedure described above, with chloro(tricyclohexylphosphine) gold (I) **E** (500 mg, 0.97 mmol, 1.00 eq.), thallium(I)acethylacetonate (302 mg, 0.99 mmol, 1.02 eq.) and trimethylsilyl azide (156 mg, 1.36 mmol, 0.18 mL, 1.40 eq.), afford compound **F** (460 mg, 91% yield) as a white solid.

### I. Preparation of TMB25

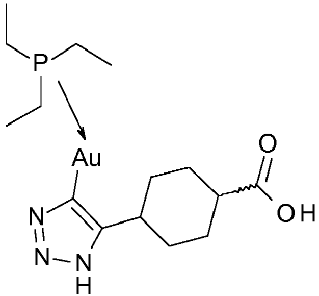

### a. Preparation of intermediate 2

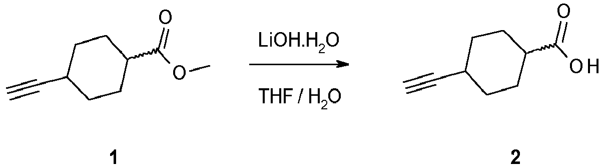

Methyl 4-ethynylcyclohexanecarboxylate **1** (250 mg, 1.50 mmol, 1.0 eq.) was solubilized in a mixture of tetrahydrofurane (10 mL) and water (1 mL). Lithium hydroxide hydrate (189 mg, 4.50 mmol, 3.0 eq.) was added to the reaction mixture which was stirred at room temperature for 18h. Solvents were evaporated under *vacuum* and the resulting aqueous solution was acidified with (6N) hydrochloric acid solution. After evaporation under *vacuum,* the residue was purified by flash chromatography [Biotage ®; column AIT ® 12g; eluant: Cyclohexane / EtOAc; gradient: 100/0 → 50/50 (12 CV)] affording compound **2** (80 mg, 35% yield) as a white powder.

### b. Preparation of compound 3

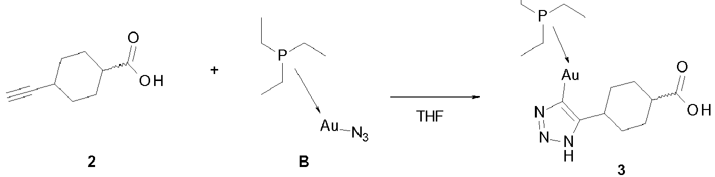

Compound **2** (80 mg, 0.52 mmol, 1.0 eq.) and triethylphosphine-gold-azide **B** (241 mg, 0.68 mmol, 1.60 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **3** (110 mg, 41% yield) as a white solid **(TMB25).**

### II. Preparation of TMB26

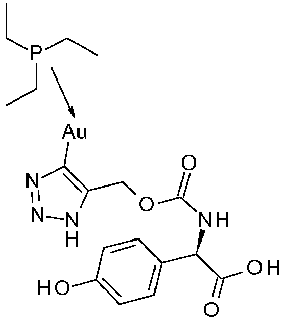

### a. Preparation of intermediate 6

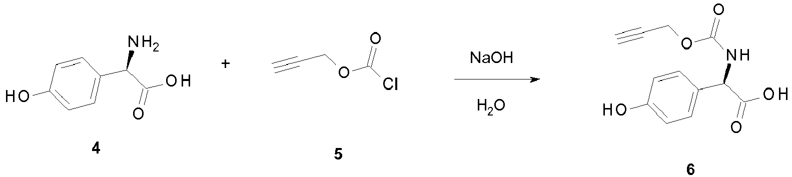

4-Hydroxy-D-phenylglycine **4** (1.00 g, 5.98 mmol, 1.0 eq.) was suspended in water (10 mL). Solubilization occurs after sodium hydroxide (0.96 g, 23.92 mmol, 4.0 eq.) addition. Reaction mixture was cooled down at 0°C, and propargylchloroformate **5** (0.78 g, 6.58 mmol, 0.64 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum* affording compound **6** (770 mg, 52% yield) as a white solid, without further purification.

### b. Preparation of compound 7

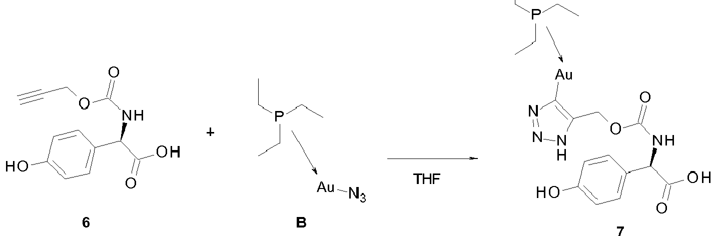

Compound **6** (100 mg, 0.40 mmol, 1.0 eq.) and triethylphosphine-gold-azide **B** (314 mg, 0.88 mmol, 2.2 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **7** (61 mg, 25% yield) as a white solid **(TMB26).**

### III. Preparation of TMB27

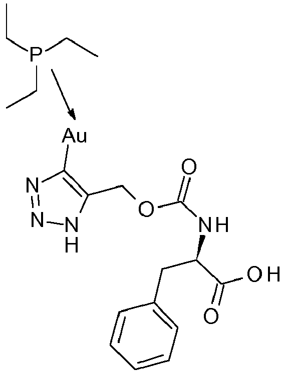

### a. Preparation of intermediate 9

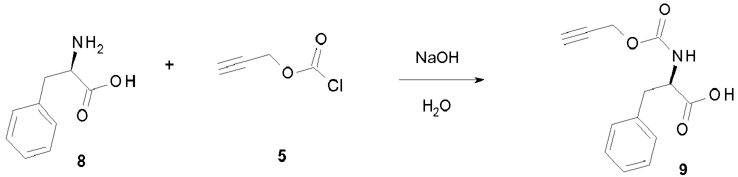

D-Phenylalanine **8** (1.00 g, 6.05 mmol, 1.0 eq.) was suspended in water (10 mL). Solubilization occurs after sodium hydroxide (0.97 g, 24.20 mmol, 4.0 eq.) addition. Reaction mixture was cooled down at 0°C, and propargylchloroformate **5** (0.79 g, 6.66 mmol, 0.65 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 0/100 (12 CV)] affording compound **9** (750 mg, 50% yield) as a colorless oil.

### b. Preparation of compound 10

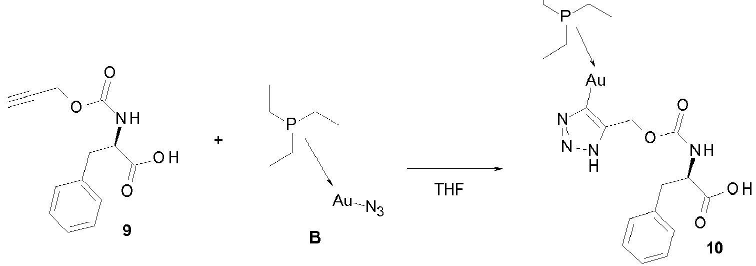

Compound **9** (100 mg, 0.40 mmol, 1.0 eq.) and triethylphosphine-gold-azide **B** (144 mg, 0.40 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **10** (110 mg, 45% yield) as a white solid **(TMB27).**

### IV. Preparation of TMB28

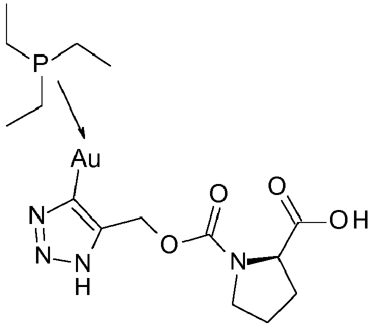

### a. Preparation of intermediate 12

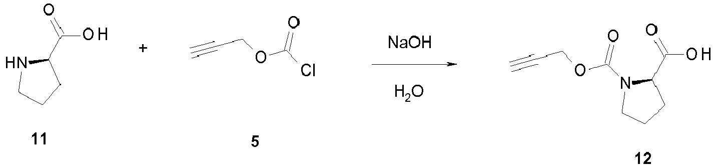

D-Proline **11** (1.00 g, 8.68 mmol, 1.0 eq.) was suspended in water (10 mL). Solubilization occurs after sodium hydroxide (1.39 g, 34.72 mmol, 4.0 eq.) addition. Reaction mixture was cooled down at 0°C, and propargylchloroformate **5** (1.13 g, 9.55 mmol, 0.93 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 0/100 (12 CV)] affording compound **12** (900 mg, 53% yield) as a colorless oil.

### b. Preparation of compound 13

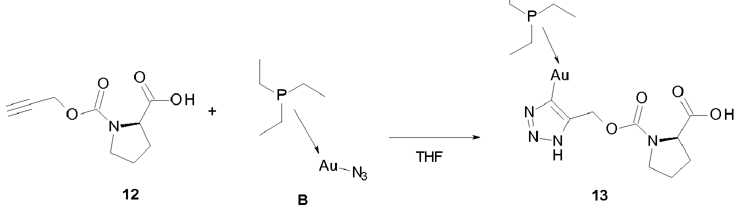

Compound **12** (100 mg, 0.51 mmol, 1.0 eq.) and triethylphosphine-gold-azide **B** (181 mg, 0.51 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **13** (120 mg, 43% yield) as a white solid **(TMB28).**

### V. Preparation of TMB29

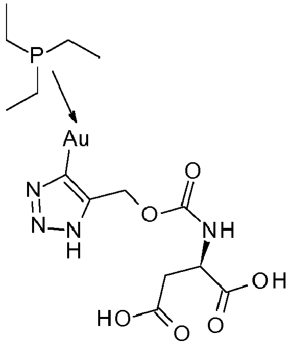

### a. Preparation of intermediate 15

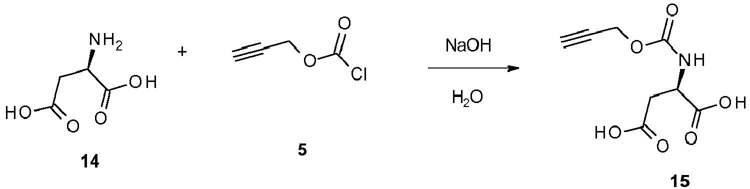

D-Aspartic acid **14** (1.00 g, 7.51 mmol, 1.0 eq.) was suspended in water (10 mL). Solubilization occurs after sodium hydroxide (1.20 g, 30.04 mmol, 4.0 eq.) addition. Reaction mixture was cooled down at 0°C, and propargylchloroformate **5** (0.98 g, 8.26 mmol, 0.81 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 0/100 (12 CV)] affording compound **15** (640 mg, 40% yield) as a white powder.

### b. Preparation of compound 16

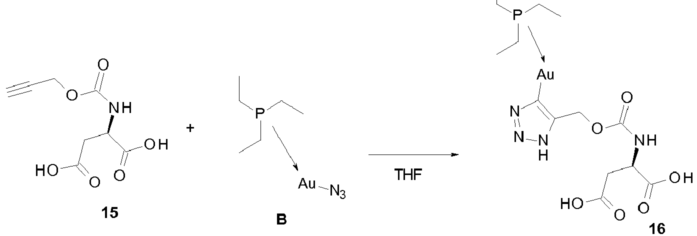

Compound **15** (200 mg, 0.93 mmol, 1.0 eq.) and triethylphosphine-gold-azide **B** (365 mg, 1.02 mmol, 1.1 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **16** (167 mg, 31% yield) as a white solid **(TMB29).**

### c. Preparation of TMB30

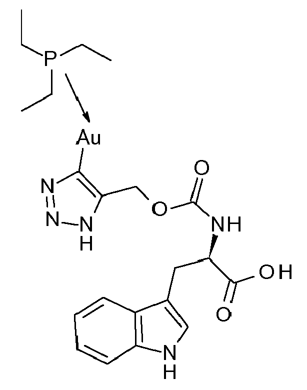

### d. Preparation of intermediate 18

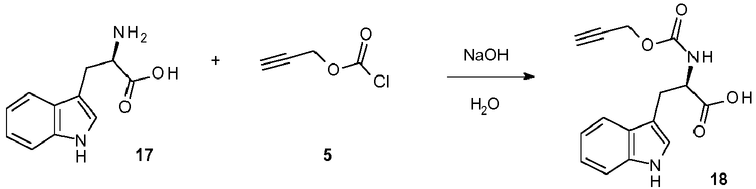

D-Tryptophane **17** (1.00 g, 4.89 mmol, 1.0 eq.) was suspended in water (10 mL). Solubilization occurs after sodium hydroxide (0.78 g, 19.56 mmol, 4.0 eq.) addition. Reaction mixture was cooled down at 0°C, and propargylchloroformate **5** (0.64 g, 5.39 mmol, 0.52 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 0/100 (12 CV)] affording compound **18** (530 mg, 38% yield) as a white foam.

### e. Preparation of compound 19

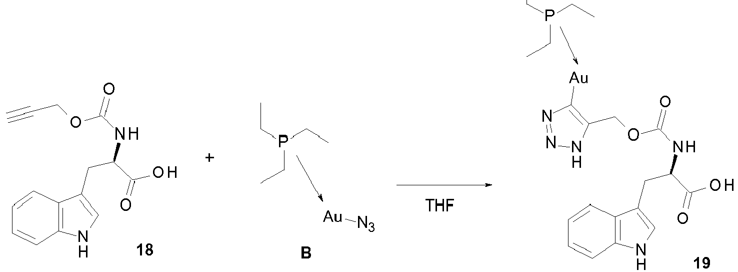

Compound **18** (100 mg, 0.35 mmol, 1.0 eq.) and triethylphosphine-gold-azide **B** (137 mg, 0.38 mmol, 1.1 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **19** (61 mg, 27% yield) as a white solid **(TMB30).**

### VI. Preparation of TMB31

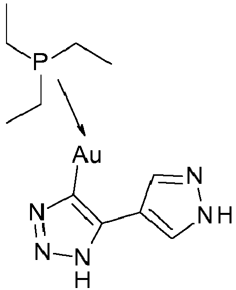

### a. Preparation of compound 21

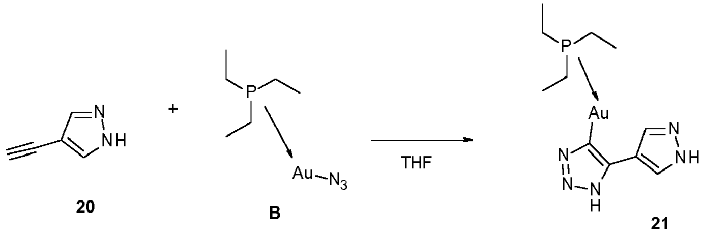

4-Ethynyl-*1H*-pyrazole **20** (100 mg, 1.09 mmol, 1.0 eq.) and triethylphosphine-gold-azide **B** (426 mg, 1.09 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **21** (266 mg, 54% yield) as a white solid **(TMB31).**

### VII. Preparation of TMB32

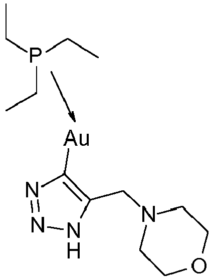

### a. Preparation of compound 23

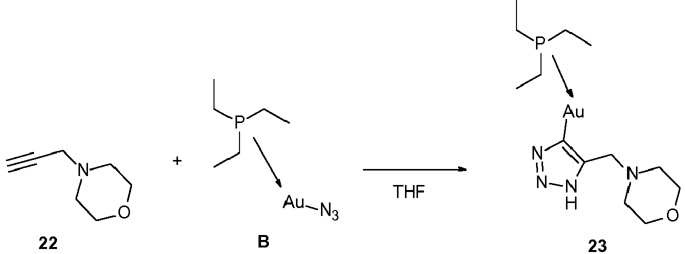

4-Prop-2-ynylmorpholine **22** (150 mg, 1.20 mmol, 1.0 eq.) and triethylphosphine-gold-azide **B** (470 mg, 1.32 mmol, 1.1 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **23** (266 mg, 46% yield) as a white sticky paste. A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **23** (123 mg, 21% yield) as a black sticky oil **(TMB32).**

### VIII. Preparation of TMB33

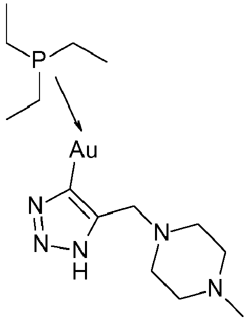

### a. Preparation of compound 25

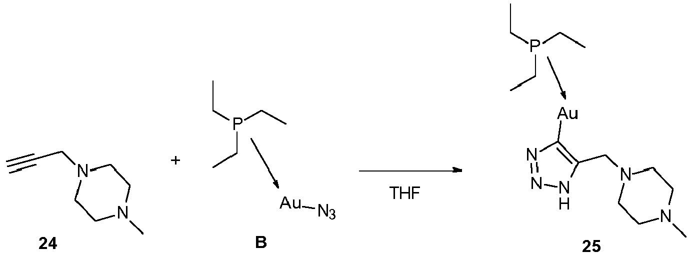

1-Methyl-4-prop-2-ynyl-piperazine **24** (150 mg, 1.08 mmol, 1.0 eq.) and triethylphosphine-gold-azide **B** (426 mg, 1.19 mmol, 1.1 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **25** (248 mg, 46% yield) as a yellow sticky paste. A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **25** (74 mg, 14% yield) as a black sticky oil **(TMB33).**

### IX. Preparation of TMB34

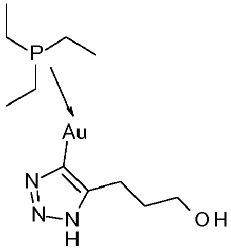

### a. Preparation of compound 27

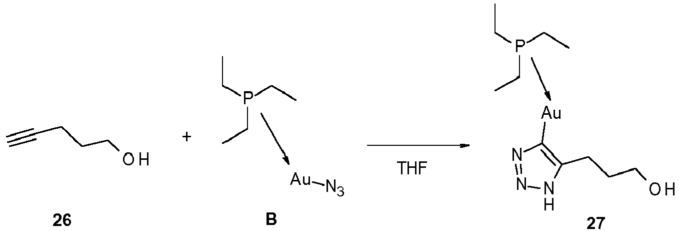

Pent-4-yn-1-ol **26** (100 mg, 1.19 mmol, 1.0 eq.) and triethylphosphine-gold-azide **B** (467 mg, 1.31 mmol, 1.1 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **27** (60% pure by LCMS). A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **27** (32 mg, 6% yield) as a colorless sticky oil **(TMB34).**

### X. Preparation of TMB35

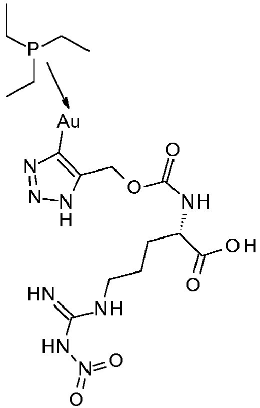

### a. Preparation of intermediate 29

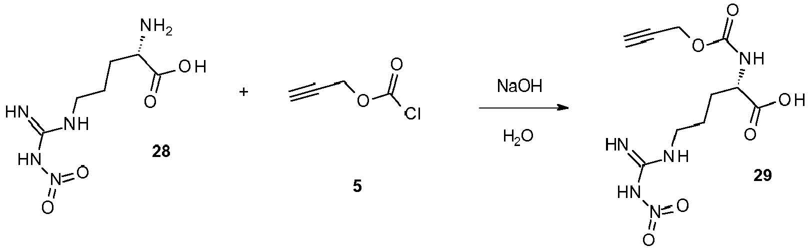

*N*_{ω}-Nitro-L-arginine **28** (1.00 g, 4.56 mmol, 1.0 eq.) was suspended in water (10 mL). Solubilization occurs after sodium hydroxide (0.73 g, 18.24 mmol, 4.0 eq.) addition. Reaction mixture was cooled down at 0°C, and propargylchloroformate **5** (0.60 g, 5.02 mmol, 0.49 mL, 1.1 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum* affording compound **29** (600 mg, 44% yield) as a white powder.

### b. Preparation of compound 30

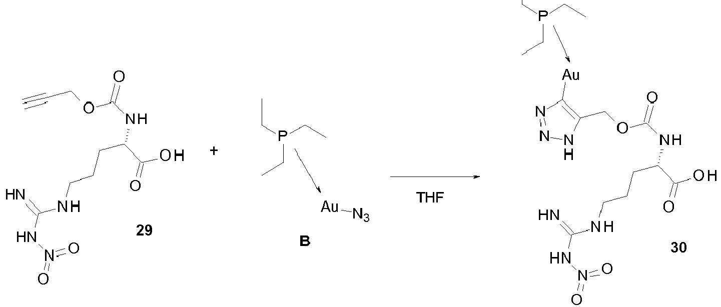

Compound **29** (100 mg, 0.33 mmol, 1.0 eq.) and triethylphosphine-gold-azide **B** (119 mg, 0.33 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **30** (40 mg, 18% yield) as a white solid **(TMB35).**

### XI. Preparation of TMB36

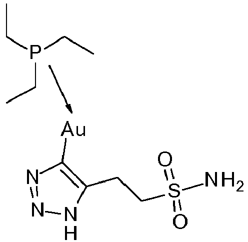

### a. Preparation of compound 32

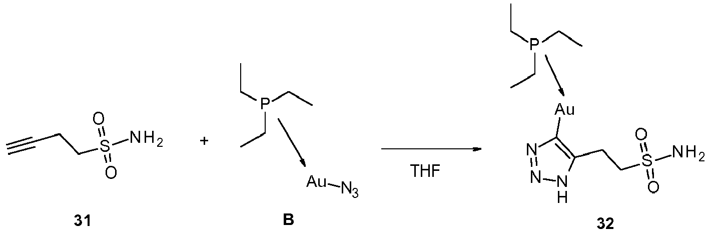

But-3-yne-1-sulfonamide **31** (100 mg, 0.75 mmol, 1.0 eq.) and triethylphosphine-gold-azide **B** (268 mg, 0.75 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **32** (165 mg, 45% yield) as a white solid **(TMB36).**

### XII. Preparation of TMB37

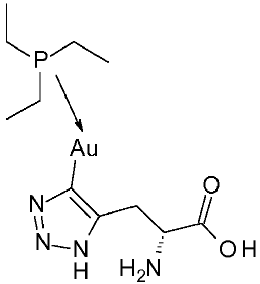

### a. Preparation of intermediate 34

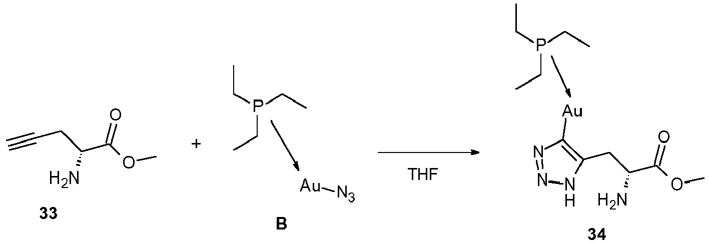

(R)-2-Amino-4-pentynoic acid, methyl ester **33** (100 mg, 0.79 mmol, 1.0 eq.) and triethylphosphine-gold-azide **B** (281 mg, 0.79 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 50/50 (25 CV)] followed by a direct lyophilization, affording compound **34** in mixture with residual phosphine derivatives (240 mg) as a yellow oil. Mixture was used without further purification for the next step.

### b. Preparation of compound 35

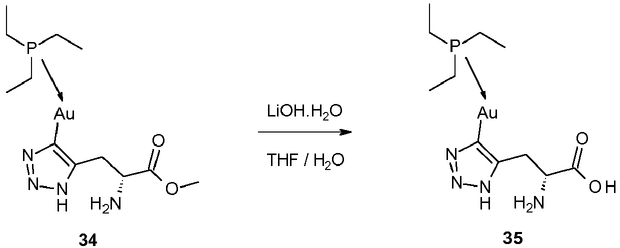

Compound **34** in mixture with residual phosphine derivatives (120 mg, 0.25 mmol, 1.0 eq.) was solubilized in a mixture of tetrahydrofurane (10 mL) and water (2.5 mL). Lithium hydroxide hydrate (31 mg, 0.75 mmol, 3.0 eq.) was added to the reaction mixture which was stirred at room temperature for 18h. Solvents were evaporated under *vacuum* and the resulting residue was purified by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes), followed by a direct lyophilization, to afford compound **35** (17 mg, 5% yield) as a white powder **(TMB37).**

### XIII. Preparation of TMB38

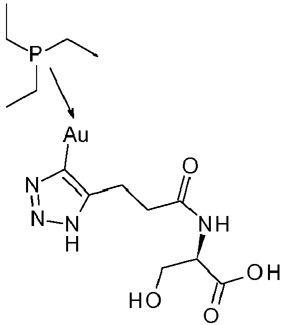

### a. Preparation of intermediate 40

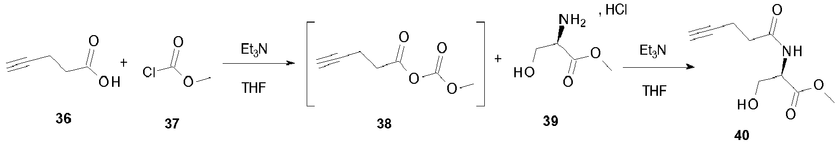

Pentynoic acid **36** (1.00 g, 10.19 mmol, 1.0 eq.) was solubilized in tetrahydrofurane (20 mL) and cooled down at -10°C under argon atmosphere. Triethylamine (3.09 g, 30.57 mmol, 4.26 mL, 3.0 eq.) was added dropwise, followed by methylchloroformate **37** (1.06 g, 11.21 mmol, 0.87 mL, 1.1 eq.). Reaction mixture was stirred at -10°C for 1h. D-Serine methyl ester hydrochloride **39** (1.60 g, 10.19 mmol, 1.0 eq.) was added portionwise to the reaction mixture and the stirring was continued for 18h at room temperature. Solvents were evaporated under *vacuum,* water (50 mL) was added to the residue and the resulting aqueous mixture was extracted with ethyl acetate (3 x 100 mL). Organic phase was dried over magnesium sulfate and solvents were evaporated under *vacuum.* The residue was purified by flash chromatography [Biotage ®; column AIT ® 40g; eluant: Cyclohexane/ EtOAc; gradient: 100/0 → 0/100 (24CV)] affording compound **40** (306 mg, 15% yield) as a colorless oil.

### b. Preparation of intermediate 41

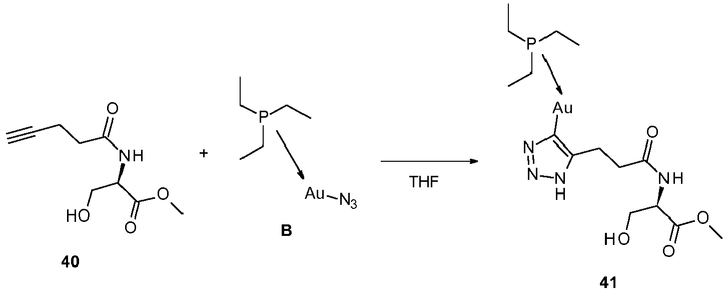

Compound **40** (100 mg, 0.50 mmol, 1.0 eq.) and triethylphosphine-gold-azide **B** (179 mg, 0.50 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (3 CV), 100/0 → 30/70 (25 CV)] followed by a direct lyophilization, affording compound **41** (98 mg, 25% yield) as a white powder.

### c. Preparation of compound 42

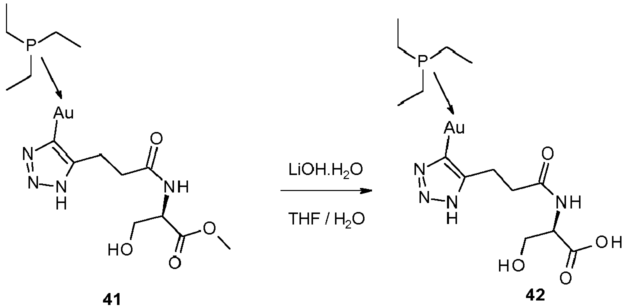

Compound **41** (98 mg, 0.18 mmol, 1.0 eq.) was solubilized in a mixture of tetrahydrofurane (10 mL) and water (2.5 mL). Lithium hydroxide hydrate (22 mg, 0.53 mmol, 3.0 eq.) was added to the reaction mixture which was stirred at room temperature for 18h. Solvents were evaporated under *vacuum* and the resulting residue was purified by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes), followed by a direct lyophilization, to afford compound **42** (16 mg, 17% yield) as a white powder **(TMB38).**

### XIV. Preparation of TMB39

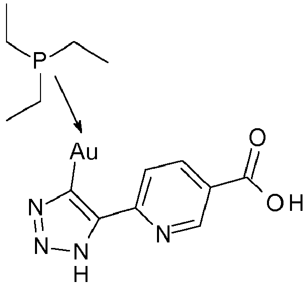

### a. Preparation of intermediate 45

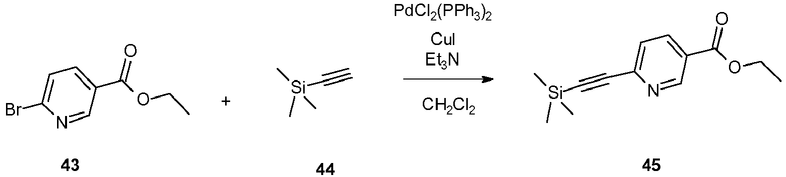

Ethyl 6-bromonicotinic acid ester **43** (1.00 g, 4.35 mmol, 1.0 eq.), triethylamine (1.96 g, 19.60 mmol, 2.7 mL, 4.5 eq.) and copper iodide (41 mg, 0.22 mmol, 0.05 eq.) were solubilized / suspended in dry dichloromethane (40 mL) and degassed with argon bubbling. After trimethylsilyl acetylene **44** (0.55 g, 5.65 mmol, 0.78 mL, 1.3 eq.) and *trans-*dichlorobis(triphenylphosphine)palladium (II) (153 mg, 0.22 mmol, 0.05 eq.) addition, reaction vessel was sealed and reaction mixture was stirred at room temperature for 18h. Reaction mixture was washed with a saturated solution of sodium chloride (2x100 mL), dried over magnesium sulfate and solvents were evaporated under *vacuum.* The residue was purified by flash chromatography [Biotage ®; column AIT ® 40g; eluant: Cyclohexane/ EtOAc; gradient: 100/0 → 80/20 (12CV)] affording compound **45** (892 mg, 83% yield) as a brown oil.

### b. Preparation of intermediate 46

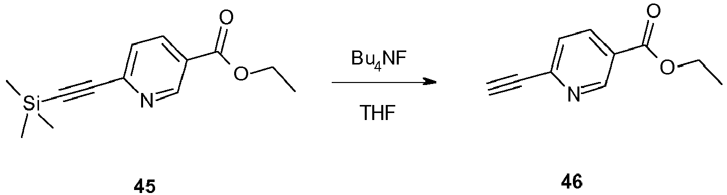

Tetra-*N*-butylammonium fluoride (1.0M) solution in tetrahydrofurane (5.4 mL, 5.40 mmol, 1.5 eq.) was added dropwise to a solution of compound **45** (890 mg, 3.60 mmol, 1.0 eq.) in tetrahydrofurane (45 mL). Reaction mixture was stirred at room temperature for 30 min. Solvents were evaporated under *vacuum* and the residue was purified by flash chromatography [Biotage ®; column AIT ® 40g; eluant: Cyclohexane/ EtOAc; gradient: 100/0 → 75/25 (12CV)] affording compound **46** (138 mg, 22% yield) as a brown solid.

### c. Preparation of intermediate 47

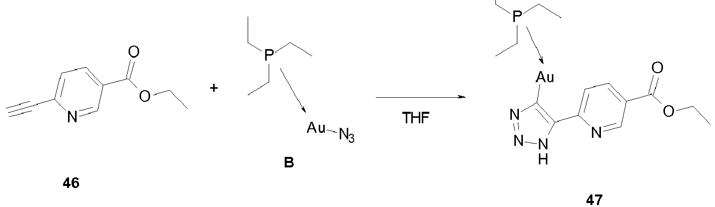

Compound **46** (138 mg, 0.79 mmol, 1.0 eq.) and triethylphosphine-gold-azide **B** (281 mg, 0.79 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 0/100 (25 CV)] followed by a direct lyophilization, affording compound **47** (236 mg, 56% yield) as a yellow solid.

### d. Preparation of compound 48

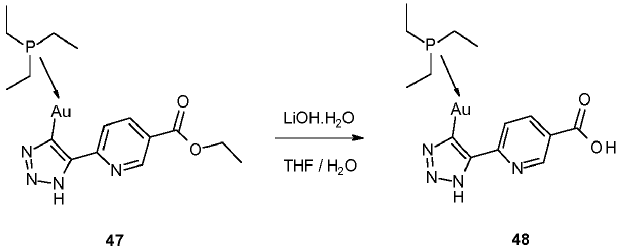

Compound **47** (220 mg, 0.41 mmol, 1.0 eq.) was solubilized in a mixture of tetrahydrofurane (20 mL) and water (5 mL). Lithium hydroxide hydrate (52 mg, 1.24 mmol, 3.0 eq.) was added to the reaction mixture which was stirred at room temperature for 18h. Solvents were evaporated under *vacuum* and the resulting residue was purified by flash chromatography [Biotage ®; column AIT ® 12g; eluant: EtOAc / (EtOAc/H₂O/AcOH 3/1/1); 100/0 → 0/100 (25 CV)] followed by a direct lyophilization, affording compound **48** (107 mg, 52% yield) as a yellowish solid. A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **48** (76 mg, 37% yield) as pale yellow solid **(TMB39).**

### XV. Preparation of TMB40

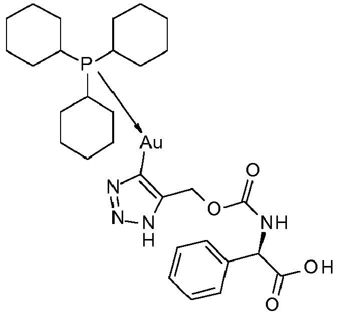

### c. Preparation of intermediate 50

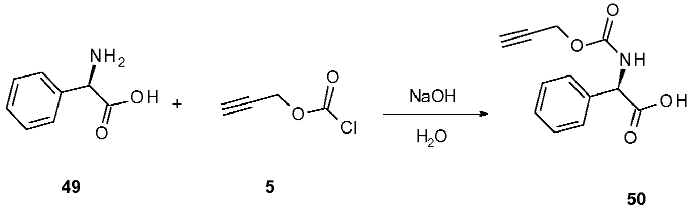

D-Phenylglycine **49** (1.00 g, 6.61 mmol, 1.0 eq.) was suspended in water (10 mL). Solubilization occurs after sodium hydroxide (1.06 g, 26.50 mmol, 4.0 eq.) addition. Reaction mixture was cooled down at 0°C, and propargylchloroformate **5** (1.57 g, 13.22 mmol, 1.29 mL, 2.0 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane/ EtOAc; gradient: 100/0 → 0/100 (12CV)] affording compound **50** (1.09 g, 71% yield) as a white solid.

### d. Preparation of compound 51

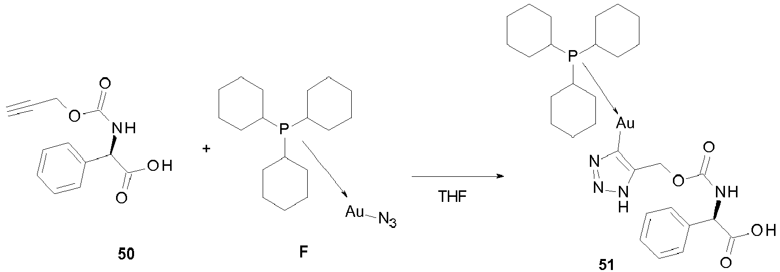

Compound **50** (100 mg, 0.43 mmol, 1.0 eq.) and tricyclohexylphosphine-gold-azide **F** (223 mg, 0.43 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc/ (EtOAc/H₂O/AcOH 3/1/1); gradient: then 100/0 → 80/20 (30CV)] followed by a direct lyophilization, affording compound **51** (163 mg, 50% yield) as a white solid. A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **51** (67 mg, 21% yield) as a white solid **(TMB40).**

### XVI. Preparation of TMB41

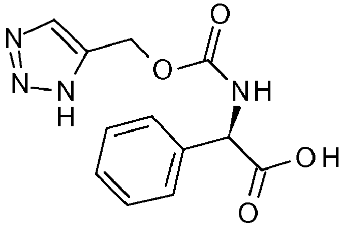

### c. Preparation of intermediate 53

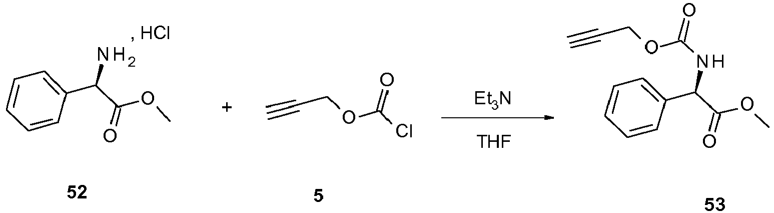

Methyl ester D-phenylglycine hydrochloride **52** (2.06 g, 10.2 mmol, 1.0 eq.) was solubilized in tetrahydrofurane / water mixture (20 mL / 10 mL). After triethylamine addition (3.12 g, 30.6 mmol, 4.3 mL, 3.0 eq.), propargylchloroformate **5** (1.22 g, 10.2 mmol, 1.0 mL, 1.0 eq.) was added dropwise and reaction mixture was stirred at room temperature for 2h. Tetrahydrofurane was removed under *vacuum,* and the aqueous residue was extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane/ EtOAc; gradient: 100/0 → 40/60 (12CV)] affording compound **53** (1.3 g, 51% yield) as a yellow oil.

### d. Preparation of intermediate 55

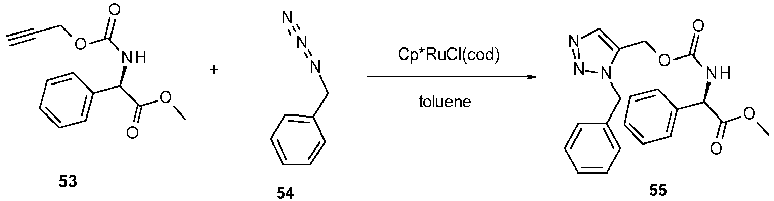

Compound **53** (1.00 g, 4.04 mmol, 1.0 eq.) was solubilized in toluene (20 mL). The solution was degassed by argon bubbling for 30 min. Benzyl azide **54** (1.07 g, 8.09 mmol, 1.01 mL, 2.0 eq.) was added to the reaction mixture and argon bubbling was continued for additionnal 10 min. Chloro(pentamethylcyclopentadienyl)(cyclooctadiene)ruthenium(II) (77 mg, 0.20 mmol, 0.05 eq.) was added, vessel was sealed and reaction mixture was stirred at 80°C for 18h. Reaction was cooled down to room temperature and solvents were evaporated under *vacuum.* The residue was purified by flash chromatography [Biotage ®; column AIT ® 80g; eluant: Cyclohexane/ EtOAc; gradient: 100/0 → 0/100 (24 CV)] affording compound **55** (316 mg, 21% yield) as a brown/orange oil.

### e. Preparation of intermediate 56

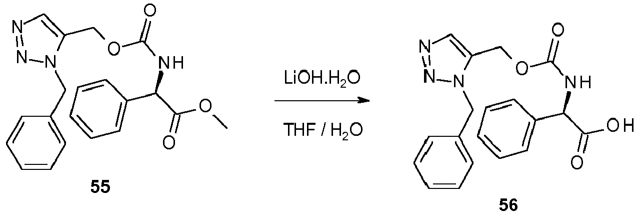

Compound **55** (310 mg, 0.81 mmol, 1.0 eq.) was solubilized in a mixture of tetrahydrofurane (40 mL) and water (10 mL). Lithium hydroxide hydrate (102 mg, 2.44 mmol, 3.0 eq.) was added to the reaction mixture which was stirred at room temperature for 18h. Solvents were evaporated under *vacuum* and the resulting residue was purified by flash chromatography [Biotage ®; column AIT ® 12g; eluant: CH₂Cl₂ / MeOH; 100/0 → 80/20 (12 CV)], affording compound **56** (210 mg, 87% yield) as a white solid.

### d. Preparation of compound 57

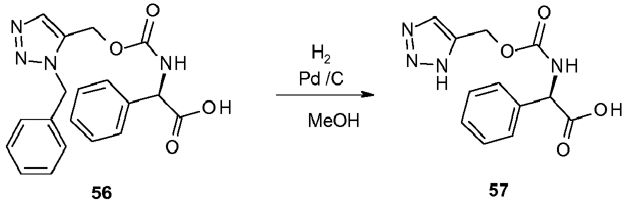

Compound **56** (210 mg, 0.57 mmol, 1.0 eq.) was solubilized in methanol (30 mL) and was hydrogenated with Pd/C at 50°C for 18h (H₂ → 5 bars). Reaction mixture was filtered through Celite ® pad, washed with methanol and solvents were evaporated under *vacuum.* The residue was purified by semi-preparative HPLC (Gilson PLC 2020, column C8 Princeton SPHER.60-1 Opm, gradient: water/acetonitrile (0.1% HCOOH) 95/5 → 95/5, 10 minutes and 95/5 → 0/100, 25 minutes), followed by a direct lyophilization, affording compound **57** (69 mg, 44% yield) as a white solid **(TMB41).**

### XVII. Preparation of TMB42

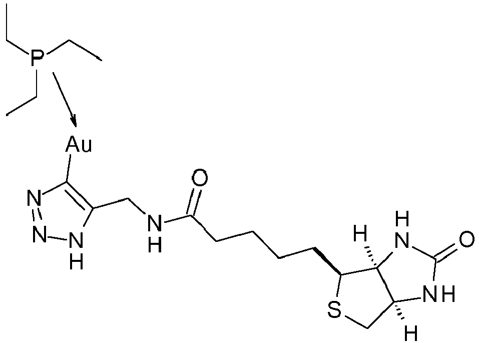

### a. Preparation of intermediate 60

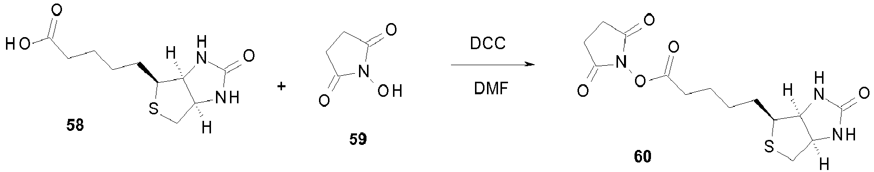

D-Biotin **58** (1.00 g, 4.09 mmol, 1.0 eq.), 1,3-dicyclohexylcarbodiimide (1.10 g, 5.32 mmol, 1.3 eq.) and N-hydroxysuccinimide **59** (518 mg, 4.50 mmol, 1.1 eq.) were suspended in dry dimethylformamide (30 mL) and stirred at room temperature for 18h. Reaction mixture was filtered through cotton pad in order to remove urea byproduct derived from DCC. Solvents were evaporated under high *vacuum.* The residue was triturated in diethylether (100 mL) and collected by filtration through fritté affording compound **60** (1.65g, yield >100%) as a white powder. The compound was used in the next step without any further purification.

### b. Preparation of intermediate 62

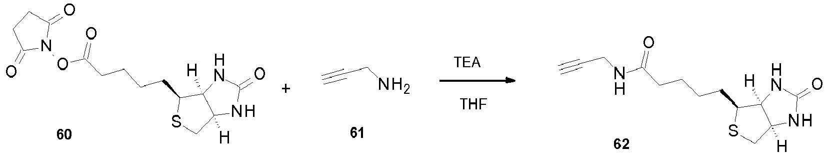

Compound **60** (850 mg, 2.49 mmol, 1.0 eq.) was solubilized in dry dimethylformamide (20 mL). After triethylamine (756 mg, 7.47 mmol, 1.0 mL, 3.0 eq.) and propargylamine **61** (274 mg, 4.98 mmol, 0.3 mL, 2.0 eq.) addition, reaction mixture was stirred at room temperature for 18h. Solvents were evaporated under *vacuum* and the residue was purified by flash chromatography [Biotage ®; column AIT ® 40g; eluant: Cyclohexane / EtOAc; gradient: 100/0 → 100/0 (3 CV), 100/0 → 0/100 (12CV) and then 0/100 → 0/100 (3CV)]. A second elution was done [CH₂Cl₂ / MeOH; gradient: 100/0 → 100/0 (3 CV), 100/0 → 80/20 (12CV) and then 80/20 → 80/20 (3CV)], affording compound **62** (690 mg, yield 98%) as a ligh brown solid.

### c. Preparation of compound 63

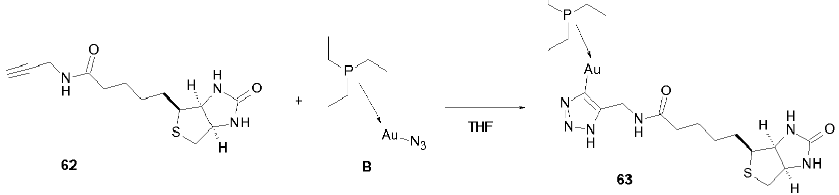

Compound **62** (100 mg, 0.36 mmol, 1.0 eq.) and triethylphosphine-gold-azide **B** (127 mg, 0.36 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 0/100 (25 CV)] followed by a direct lyophilization, affording compound **63** (71 mg, 31% yield) as a white solid. A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **63** (44 mg, 19% yield) as a white solid **(TMB42).**

### XVIII. Preparation of TMB43

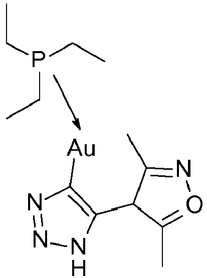

### a. Preparation of intermediate 65

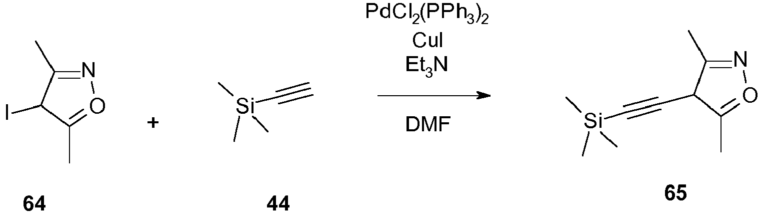

3,5-Dimethyl-4-iodoisoxazole **64** (5.00 g, 22.4 mmol, 1.0 eq.), triethylamine (9.07 g, 89.7 mmol, 12.5 mL, 4.0 eq.) and copper iodide (213 mg, 1.12 mmol, 0.05 eq.) were solubilized / suspended in dry dimethylformamide (50 mL) and degassed with argon bubbling. After trimethylsilyl acetylene **44** (2.62 g, 26.9 mmol, 3.7 mL, 1.2 eq.) dropwise addition, *trans-* dichlorobis(triphenylphosphine)palladium (II) (787 mg, 1.12 mmol, 0.05 eq.) was added portionwise and reaction vessel was sealed. Reaction mixture was stirred at 75°C for 4h. After cooling down at room temperature, reaction mixture was diluted in 200 mL of diethylether and washed with a saturated solution of sodium chloride (2x100 mL), dried over magnesium sulfate and solvents were evaporated under *vacuum.* The residue was purified by flash chromatography [Biotage ®; column AIT ® 120g; eluant: Cyclohexane/ EtOAc; gradient: 100/0 → 90/10 (12CV)] affording compound **65** (3.2 g, 74% yield) as a dark brown oil.

### b. Preparation of intermediate 66

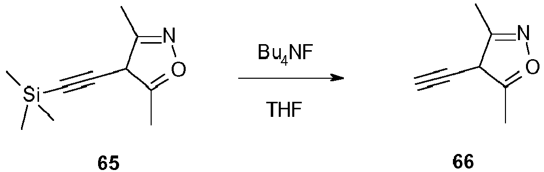

Tetra-*N*-butylammonium fluoride (1.0M) solution in tetrahydrofurane (24.8 mL, 24.8 mmol, 1.5 eq.) was added dropwise to a solution of compound **65** (3.2 g, 16.60 mmol, 1.0 eq.) in tetrahydrofurane (100 mL). Reaction mixture was stirred at room temperature for 30 min. Solvents were evaporated under *vacuum* and the residue was purified by flash chromatography [Biotage ®; column AIT ® 120g; eluant: Cyclohexane/ EtOAc; gradient: 100/0 → 80/20 (12CV)] affording compound **66** (620 mg, 31% yield) as a white solid.

### c. Preparation of intermediate 47

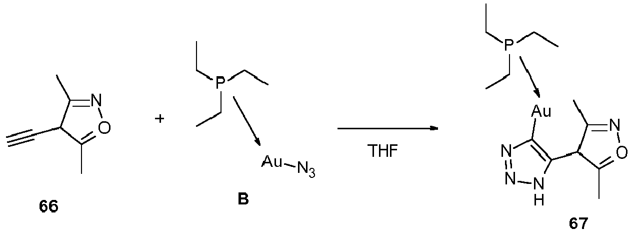

Compound **66** (200 mg, 1.65 mmol, 1.0 eq.) and triethylphosphine-gold-azide **B** (590 mg, 1.65 mmol, 1.0 eq.) were suspended in degassed dry tetrahydrofurane (40 mL) and stirred in the dark at room temperature for 18h. Solvents were evaporated under *vacuum* and the residue was purified by flash chromatography [Biotage ®; column AIT ® 40g; eluant: EtOAc / (EtOAc/H₂O/AcOH 3/1/1); gradient: 100/0 → 70/30 (25 CV)] followed by a direct lyophilization, affording compound **67** (100 mg, in mixture with residual phosphine derivatives). A second purification by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes) was done, followed by a direct lyophilization, affording compound **67** (20 mg, 3% yield) as a white solid **(TMB43).**

### XIX. Preparation of TMB44

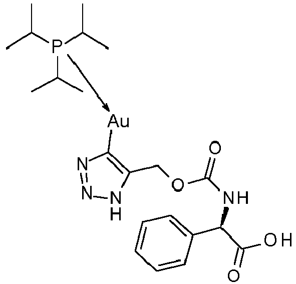

### See procedure for the preparation of the intermediate 50

### a. Preparation of Compound 68

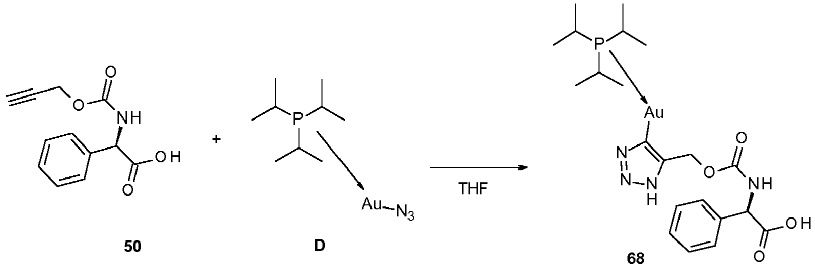

Intermediate **50** (100 mg, 0.43 mmol, 1.0 eq.) and triisopropylphosphine-gold-azide **D** (171 mg, 0.43 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc/ (EtOAc/H₂O/AcOH 3/1/1); gradient: then 100/0 → 50/50 (25 CV)] followed by a direct lyophilization, affording compound **68** (130 mg, 48% yield) as a white solid **(TMB44).**

### XX. Preparation of TMB45

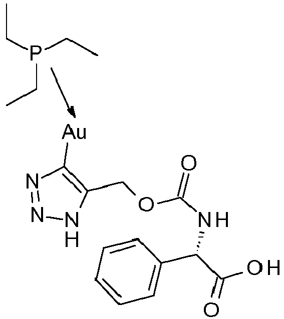

### a. Preparation of intermediate 70

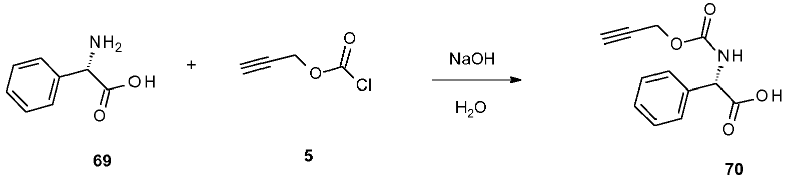

L-Phenylglycine **69** (1.00 g, 6.61 mmol, 1.0 eq.) was suspended in water (10 mL). Solubilization occurs after sodium hydroxide (1.06 g, 26.5 mmol, 4.0 eq.) addition. Reaction mixture was cooled down at 0°C, and propargylchloroformate **5** (1.58 g, 13.2 mmol, 1.3 mL, 2.0 eq.) was added dropwise. Reaction mixture was allowed to warm up to room temperature for 30 minutes. Reaction mixture was acidified with (12N) hydrochloric acid solution, extracted with ethyl acetate (3 x 100 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 0/100 (12 CV)] affording compound **70** (977 mg, 63% yield) as a white solid.

### b. Preparation of compound 71

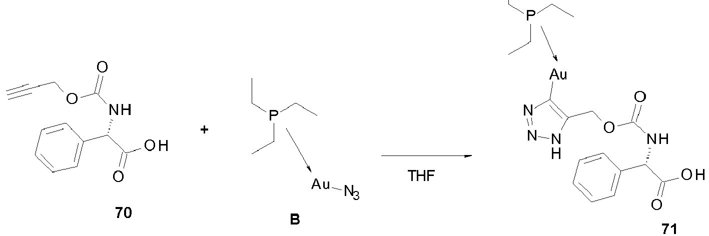

Compound **70** (100 mg, 0.43 mmol, 1.0 eq.) and triethylphosphine-gold-azide **B** (153 mg, 0.43 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 25/75 (25 CV)] followed by a direct lyophilization, affording compound **71** (114 mg, 45% yield) as a white solid **(TMB45).**

### XXI. Preparation of TMB46

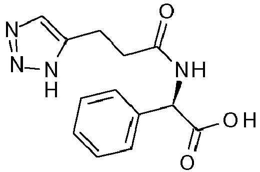

### a. Preparation of intermediate 72

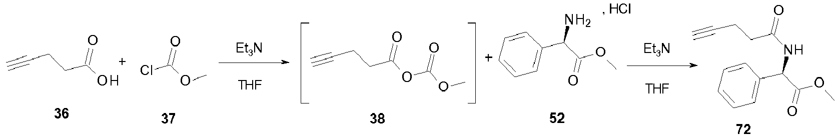

Pentynoic acid **36** (1.00 g, 10.19 mmol, 1.0 eq.) was solubilized in tetrahydrofurane (20 mL) and cooled down at -10°C under argon atmosphere. Triethylamine (3.09 g, 30.57 mmol, 4.26 mL, 3.0 eq.) was added dropwise, followed by methylchloroformate **37** (1.06 g, 11.21 mmol, 0.87 mL, 1.1 eq.). Reaction mixture was stirred at -10°C for 1h. Methyl ester D-phenylglycine hydrochloride **52** (2.10 g, 10.19 mmol, 1.0 eq.) was added portionwise to the reaction mixture and the stirring was continued for 18h at room temprature. Solvents were evaporated under *vacuum,* water (50 mL) was added to the residue and the resulting aqueous mixture was extracted with ethyl acetate (3 x 100 mL). Organic phase was dried over magnesium sulfate and solvents were evaporated under *vacuum.* The residue was purified by flash chromatography [Biotage ®; column AIT ® 40g; eluant: Cyclohexane/ EtOAc; gradient: 100/0 → 40/60 (24CV)] affording compound **72** (1.40 g, 56% yield) as a colorless oil.

### b. Preparation of intermediate 73

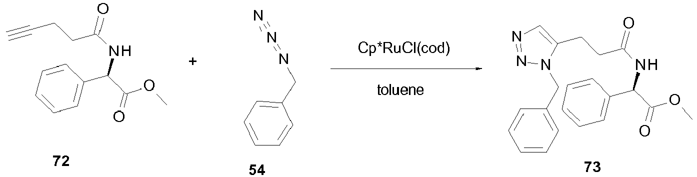

Compound **72** (1.00 g, 4.08 mmol, 1.0 eq.) was solubilized in toluene (20 mL). The solution was degassed by argon bubbling for 30 min. Benzyl azide **54** (1.09 g, 8.15 mmol, 1.02 mL, 2.0 eq.) was added to the reaction mixture and argon bubbling was continued for additionnal 10 min. Chloro(pentamethylcyclopentadienyl)(cyclooctadiene)ruthenium(II) (77 mg, 0.20 mmol, 0.05 eq.) was added, vessel was sealed and reaction mixture was stirred at 80°C for 18h. Reaction was cooled down to room temperature and solvents were evaporated under *vacuum.* The residue was purified by flash chromatography [Biotage ®; column AIT ® 80g; eluant: Cyclohexane/ EtOAc; gradient: 100/0 → 0/100 (24 CV)] affording compound **73** (1.20 g, 79% yield) as a yellow foam.

### c. Preparation of intermediate 74

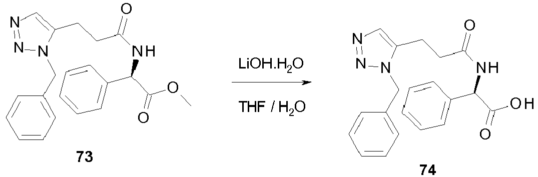

Compound **73** (1.20 g, 3.17 mmol, 1.0 eq.) was solubilized in a mixture of tetrahydrofurane (120 mL) and water (30 mL). Lithium hydroxide hydrate (400 mg, 9.51 mmol, 3.0 eq.) was added to the reaction mixture which was stirred at room temperature for 18h. Solvents were evaporated under *vacuum* and the resulting residue was purified by flash chromatography [Biotage ®; column AIT ® 40g; eluant: CH₂Cl₂ / MeOH; 100/0 → 80/20 (12 CV)], affording compound **74** (1.10 mg, 99% yield) as a white foam.

### d. Preparation of compound 75

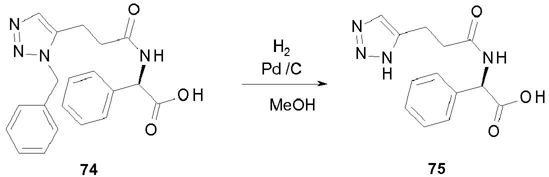

Compound **74** (1.10 g, 3.02 mmol, 1.0 eq.) was solubilized in methanol (60 mL) and was hydrogenated with Pd/C at 50°C for 22h (H₂ → 5 bars), then at 50°C for additionnal 24h (H₂ → 6 bars). Reaction mixture was filtered through Celite ® pad, washed with methanol and solvents were evaporated under *vacuum* affording compound **75** (690 mg, 83% yield) as a white solid. A 200 mg portion of the residue was purified by semi-preparative HPLC (Gilson PLC 2020, column C8 Princeton SPHER.60-10µm, gradient: water/acetonitrile (0.1% HCOOH) 95/5 → 95/5, 10 minutes and 95/5 → 0/100, 25 minutes), followed by a direct lyophilization, affording compound **75** (160 mg, calculated 67% yield) as a white solid **(TMB46).**

### XXII. Sourcing of intermediate TMB47

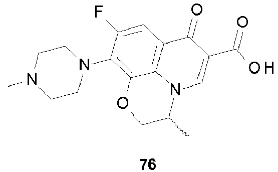

Ofloxacin **76** was sourced from Preswick Chemical Library®.

### XXIII. Preparation of TMB48

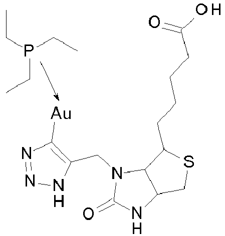

### a. Preparation of intermediate 79

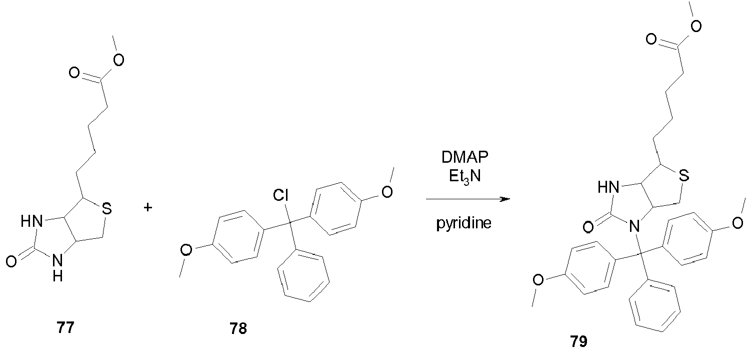

5-(2-Oxo-hexahydro-thieno[3,4-d]imidazol-6-yl)-pentanoic acid methyl ester **77** (1.00 g, 3.87 mmol, 1.0 eq.), 4,4'-Dimethoxytrityl chloride **78** (3.93 g, 11.61 mmol, 3.0 eq.), triethylamine (467 mg, 4.64 mmol, 0.65 mL, 1.2 eq.) and DMAP (118 mg, 0.97 mmol, 0.25 eq.) were solubilized in dry pyridine (30 mL) and stirred at 75°C for 18h in a sealed reaction vessel. Reaction mixture was cooled down to room temperature and solvents were evaporated under *vacuum.* The residue was purified by flash chromatography [Biotage ®; column AIT ® 80g; eluant: Cyclohexane / EtOAc; gradient: 100/0 → 100/0 (3 CV), 100/0 → 0/100 (12CV) and then 0/100 → 0/100 (3CV)], affording compound **79** (1.10 g, yield 51%) as a yellow foam.

### b. Preparation of intermediate 81

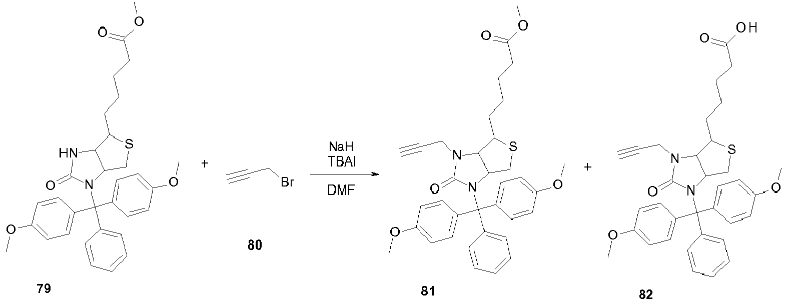

Sodium hydride (60% in mineral oil) (196 mg, 4.90 mmol, 5.0 eq.) was suspended in dry dimethylformamide (20 mL) and cooled down to 0°C under argon atmosphere. Compound **79** (550 mg, 0.98 mmol, 1.0 eq.) was solubilized in dry dimethylformamide (20 mL) and added dropwise to the sodium hydride suspension. *Tetra*-butylammonium iodide (72 mg, 0.20 mmol, 0.2 eq.) was added portionwise to reaction mixture, followed by propargyl bromide solution **80** (80% wt. in toluene) (140 mg, 1.18 mmol, 0.13 mL, 1.2 eq.). The mixture was stirred at room temperature for 3h. After water addition (20 mL), volatiles were removed under *vacuum.* Residual aqueous phase was acidified (pH = 2) by potassium bisulfate addition and extracted with ethyl acetate (3 x 100 mL). Organics were dried over magnesium sulfate and solvents were evaporated under *vacuum* affording compound **81** in mixture with compound **82** (75/25). The mixture was purified by flash chromatography [Biotage ®; column AIT ® 120g; eluant: Cyclohexane / EtOAc; gradient: 100/0 → 100/0 (3 CV) and then 100/0 → 40/60 (22CV)]. A second elution was done [CH₂Cl₂ / MeOH; gradient: 100/0 → 100/0 (3 CV), and then 100/0 → 75/25 (12CV)], affording compound **81** (180 mg, 31% yield) as an orange foam and a fraction of compound **81** in mixture with compound **82** (10/90) (320 mg, approx. 56% yield) as a black oil. Both fraction were combined for the next step.

### c. Preparation of intermediate 83

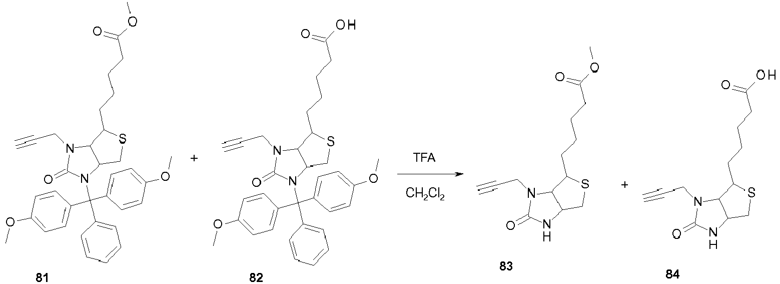

A mixture of compound **81** and compound **82** (500 mg, approx. 0.85 mmol, 1.0 eq.), was solubilized in dichloromethane (50 mL) and, after trifluoroacetic acid addition (10 mL), stirred at room temperature for 1h. Solvents were evaporated under high *vacuum,* affording a mixture of compound **83,** compound **84** and byproduct issued of 4,4'-Dimethoxytrityl deprotection. This mixture was found to be not separable after biotage purification. As a consecquence, the crude mixture was used for the next step (saponification).

### d. Preparation of intermediate 84

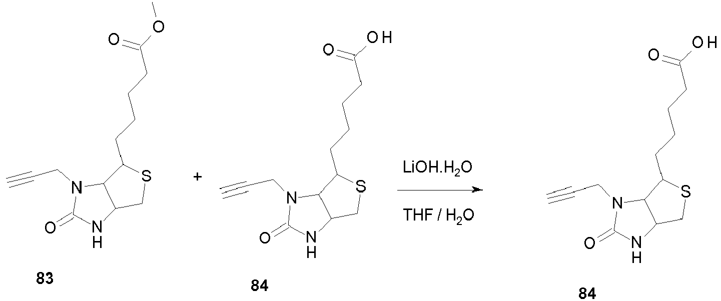

Previous crude mixture of compounds **83** and **84** was solubilized in a mixture of tetrahydrofurane (50 mL) and water (10 mL). Lithium hydroxide hydrate (409 mg, 9.77 mmol, estimated 5.0 eq.) was added to the reaction mixture which was stirred at room temperature for 3h. Solvents were evaporated under *vacuum* and the resulting residue was acidified with HCl (2.0M) in diethyl ether. Solvent were removed under *vacuum* and the residue was purified by flash chromatography [Biotage ®; column AIT ® 40g; eluant: Cyclohexane / EtOAc; gradient: 100/0 → 100/0 (5 CV), 100/0 → 0/100 (15CV), then 0/100 → 0/100 (5CV)], followed by liophilization, affording compound **84** (100 mg, calculated 72% yield for 3 steps) as a brown oil.

### e. Preparation of compound 85

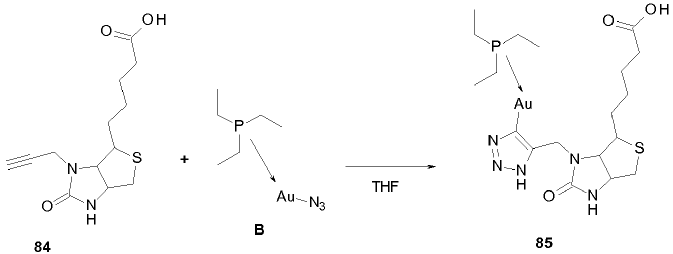

Compound **84** (100 mg, 0.35 mmol, 1.0 eq.) and triethylphosphine-gold-azide **B** (139 mg, 0.35 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (2 CV), then 100/0 → 50/50 (15 CV)] followed by a direct lyophilization, affording compound **85** (30 mg, 13% yield) as a white solid **(TMB48).**

### XXIV. Preparation of TMB49

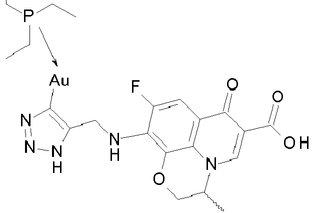

### a. Preparation of compound 87

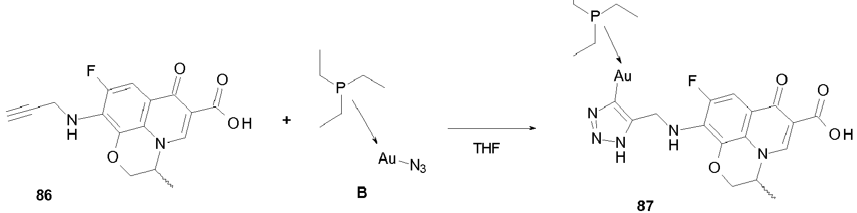

Compound **86,** (100 mg, 0.32 mmol, 1.0 eq.) and triethylphosphine-gold-azide **B** (113 mg, 0.32 mmol, 1.0 eq.) were solubilized in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 100/0 (2 CV), then 100/0 → 40/60 (15 CV)] followed by a direct lyophilization, affording compound **87** (88 mg, 41% yield) as a pale yellow solid **(TMB49).**

### XXV. Preparation of TMB50

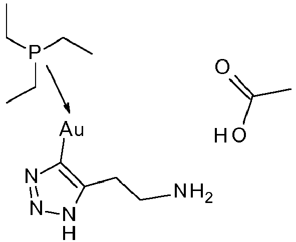

### a. Preparation of compound 89

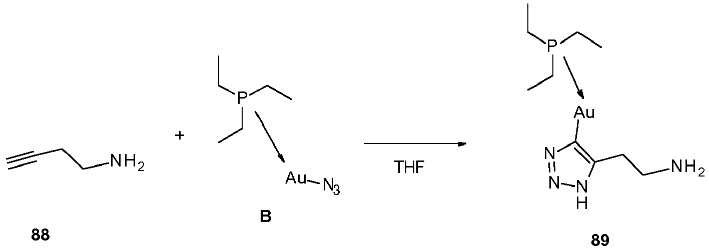

1-Amino-3-butyne **88** (100 mg, 1.45 mmol, 0.12 mL, 1.0 eq.) and triethylphosphine-gold-azide **B** (517 mg, 1.45 mmol, 1.0 eq.) were solubilized in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 0/100 (15 CV)] followed by a direct lyophilization, affording compound **89** obtained as acetic acid salt (178 mg, 23% yield) as a pale yellow solid **(TMB50).**

### XXVI. Preparation of intermediate TMB51

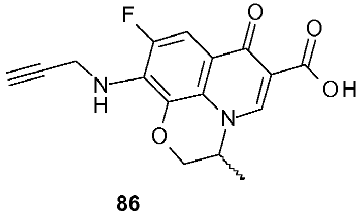

Compound **86** nd was purified by flash chromatography (315 mg scale) [Biotage ®; column AIT ® 12g; eluant: CH₂Cl₂ / MeOH; gradient: 100/0 → 90/10 (12 CV)] followed by a lyophilization, affording compound **86** (185 mg, 59% yield) as a yellowish solid **(TMB51).**

### XXVII. Preparation of TMB52

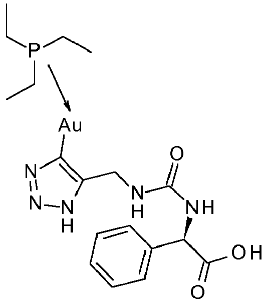

### e. Preparation of intermediate 88

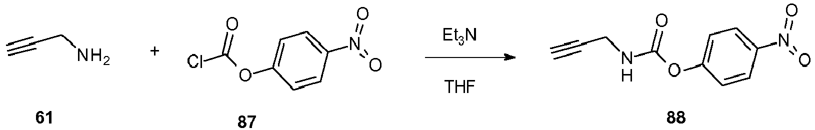

*p*-Nitrophenyl-chloroformate **87** (3.7 g, 18.2 mmol, 1.0 eq.) was solubilized in tetrahydrofurane (12 mL) and added dropwise to a cold solution (0°C) of propargylamine **61** (1.0 g, 18.2 mmol, 1.16 mL, 1.0 eq.) and triethylamine (3.7 g, 36.3 mmol, 4.90 mL, 2.0 eq.) in tetrahydrofurane (35 mL), under argon atmosphere. Reaction mixture was stirred for 3h at room temperature and the precipitate was filtered off. The filtrate was evaporated under *vacuum* and the residue was purified by flash chromatography [Biotage ®; column AIT ® 120g; eluant: Cyclohexane/ EtOAc; gradient: 100/0 → 20/80 (15 CV)] affording compound **88** (2.0 g, 37% yield) as a pale yellow solid.

### f. Preparation of intermediate 89

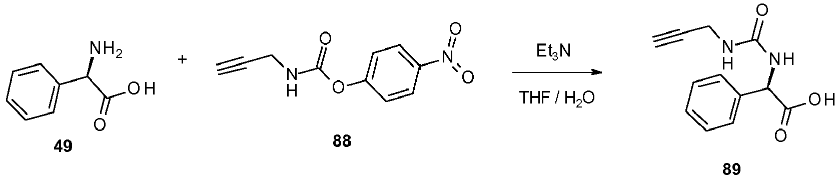

A solution of compound **88** (1.00 g, 4.27 mmol, 1.0 eq.) in tetrahydrofurane (20 mL) was prepared. D-Phenylglycine **49** (0.64 g, 4.27 mmol, 1.0 eq.) was suspended in water (10 mL) and tetrahydrofurane (20 mL). Solubilization occurs after triethylamine (1.30 g, 12.81 mmol, 1.78 mL, 3.0 eq.) addition. This solution was added dropwise to the previous solution of compound **88.** The reaction mixture was stirred at room temperature for 1h and volatiles were evaporated under *vacuum.* The residual aqueous phase was cooled in an ice bath and acidified with with hydrochloric acid solution (12N). No precipitation occurs, but a gummy residue was formed. Supernatant was removed and the gummy residue was solubilized in ethyl acetate (20 mL), dried over magnesium sulfate and solvents were evaporated under *vacuum.* The residue was purified by flash chromatography [Biotage ®; column AIT ® 40g; eluant: Cyclohexane / EtOAc; gradient: 100/0 → 100/0(3 CV), then 100/0 → 0/100 (15 CV)] affording compound **89** (240 mg, 24% yield) as a white solid.

### g. Preparation of compound 90

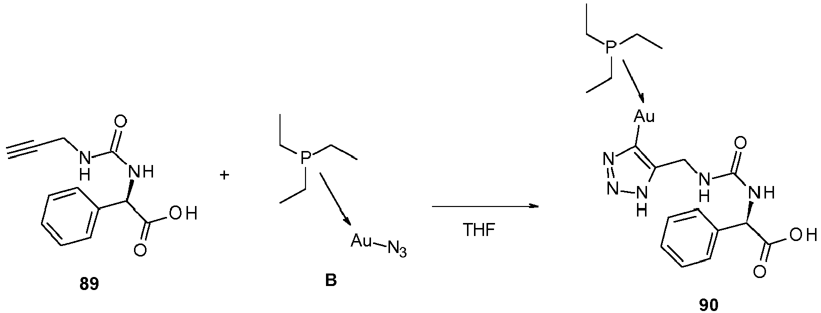

Compound **89** (100 mg, 0.43 mmol, 1.0 eq.) and triethylphosphine-gold-azide **B** (154 mg, 0.43 mmol, 1.0 eq.) were suspended in degassed dry tetrahydrofurane (20 mL) and stirred in the dark at room temperature for 18h. Solvents were evaporated under *vacuum* and the residue was purified by flash chromatography [Biotage ®; column AIT ® 12g; eluant: EtOAc / (EtOAc/H₂O/AcOH 3/1/1); gradient: 100/0 → 20/80 (25 CV)] followed by a direct lyophilization, affording compound **90** (41 mg, 16% yield) as a white solid **(TMB52).**

### Examples part B : CYTOTOXICITY studies

### Materials & Methods :

The human hepatocyte cytotoxicity profile has been evaluated between 7 and 25 µM for TMB derivatives as a toxicity appeared from 25 µM for the initial hit TMB3 during a preliminary assessment.

The cytotoxicity profile depends on the nature of the chemical series. Also, apart from very short derivatives, the TMB compounds exhibit generally an acceptable cytotoxicity up to 17 µM allowing a therapeutic window with regard to the extremely weak minimum inhibitory concentrations obtained during microbiological tests and the extreme fragility of hepatocytes cells.

This preliminary in vitro cytotoxicity provides an indication of an acceptable therapeutic window if we consider that the MICs measured correspond to a range of concentrations of 0.08-0.63µM for TMB3 compound depending on the bacteria strains and that a cytotoxicity appears at 20 µM for this molecule.

### Cytotoxicity profile

The cytotoxicity profile depends on the nature of the chemical series. Also, apart from very short derivatives, the compounds according to the invention exhibit generally an acceptable cytotoxicity up to 17 µM allowing a therapeutic window with regard to the extremely weak minimum inhibitory concentrations obtained during microbiological tests and the extreme fragility of hepatocytes cells.

### Examples part C : BIOLOGICAL DATA

### Materials & Methods :

### Antibacterial profiles

The antibacterial profile of each derivative was evaluated by the standard microdilution method for susceptibility testing on 16 different strains, following the guidelines of CLSI. The selected panel included:
- S. aureus ATCC25923 (MecA negative, wild-type)
- S. aureus ATCC29213 (MecA negative, wild-type)
- S. aureus ATCC700699 (MecA positive, glycopeptide resistant)
- S. aureus ST20131365 (MecA positive, glycopeptide resistant)
- S. epidermidis ATCC14990 (MecA negative, wild-type)
- S. epidermidis ATCC35984 (MecA positive)
- S. epidermidis ST20140436 (MecA positive, glycopeptide resistant)
- S. epidermidis ST20150446 (MecA positive, glycopeptide resistant)
- E. faecalis JH2-2 (wild-type)
- E. faecalis UCN41 (VanA positive, glycopeptide resistant)
- E. faecalis V583 (VanB positive, glycopeptide resistant)
- E. faecium ATCC19434T (wild-type)
- E. faecium BM4147 (VanA positive, glycopeptide resistant)
- E. faecium AUS0004 (VanB positive, glycopeptide resistant)
- E. coli ATCC25922 (wild-type)
- Clostridium difficile ATCC9689 (wild-type)

### Antibacterial activity

Both series issued from the SAR study were evaluated for their antibacterial activity on a panel of characterized strains obtained from the French National Reference Centers for Staphylococci (Lyon, Pr. Frédérique Laurent) and for Enterococci (Caen, Pr. Vincent Cattoir), respectively. As shown in the following tables, both series contain very active compounds on multi-resistant Gram-positive bacteria (Minimal Inhibitory Concentrations lower than comparator antibiotic ampicillin). Furthermore, a very important antibacterial effect has been shown too on a strain of *Clostridium difficile,* a Gram-positive, spore-forming, bacillus responsible for post-antibiotic diarrhoae.

The antibacterial activity of test compounds was evaluated on a selected panel of Gram-positive (4 *Staphylococcus aureus,* 4 *Staphylococcus epidermidis,* 3 *Enterococcus faecalis,* 3 *Enterococcus faecium,* 1 *Clostridium difficile)* and Gram-negative strains (1 *Escherichia coli,* 1 *Klebsiella pneumoniae,* 1 *Pseudomonas aeruginosa,* 1 *Acinetacter baumannii*) with known features of antibiotic resistance (from wild-type susceptibility to multi-drug resistance). The Minimal Inhibitory Concentration (MIC) of test compounds was determined in duplicate by the microdilution method in 96-well microplates with a final volume of 100µL per well and Mueller-Hinton broth as the growth medium, as recommended by the CLSI (Clinical and Laboratory Standards Institute, document Mo7-A10, USA).

### Results :

Altogether, the data from Table 1, 2, 3, 4, 5, 6, 7, 9, 10, 11 and 12 obtained suggest that the compounds of the invention are excellent candidates for the development of new antibacterial agents against several multiresisant bacteria. In particular, as demonstrated in the following tables 1 - 12, examples of compounds of the present invention have Minimal Inhibitory Concentrations (MIC) close or lower than comparator antibiotic ampicillin and clearly demonstrating that they have antimicrobial activity on different strain or type of bacteria, in particular on multi-resistant bacteria.

In particular the compounds TMB1, TMB3, TMB4, TMB8, TMB12, TMB13, TMB14, TMB15, TMB18, TMB19, TMB20, TMB21, TMB22, TMB23, TMB24, TMB25, TMB26, TMB27, TMB28, TMB29, TMB30, TMB31, TMB32, TMB33, TMB34, TMB35, TMB36, TMB37, TMB38, TMB39, TMB42, TMB43, TMB45, TMB47, TMB48, TMB49, TMB50, TMB52 demonstrating that they have antimicrobial activity. In contrary the compounds TMB2, TMB5, TMB6, TMB7, TMB9, TMB41, TMB46, TMB51, which not comprise gold (I)-phosphine or 1,2,3-triazole moiety do not show an antibacterial activity.
In the tables below "Ampi" means ampicilline.

**Table 1 :**

| | | Ampi | TMB1 | TMB3 | TMB4 | TMB5 |
|---|---|---|---|---|---|---|
| S. aureus | ATCC25923 | 0.5 | 0.125 | 0.125 | 0.125 | 2 |
| | ATCC29213 | 0.5 | 0.125 | 0.125 | 0.125 | 2 |
| | ATCC700699 | 8/>8 | 0.125/0.25 | 0.125/0.25 | 0.125 | >8 |
| | ST20131365 | 4 | 0.125 | 0.125/0.25 | 0.125 | >8 |
| S. epidermis | ATCC14990 | 1 | ≤0.06 | ≤0.06 | ≤0.06 | 1 |
| | ATCC35984 | >8 | ≤0.06 | ≤0.06 | ≤0.06 | >8 |
| | ST20140436 | 4 | ≤0.06 | ≤0.06 | ≤0.06 | 8/>8 |
| | ST20150446 | 8 | ≤0.06 | ≤0.06/0.125 | 0.125 | >8 |
| E. faecalis | JH2-2 | 0.25/0.5 | 0.25/0.5 | 0.5/1 | 0.25/5 | 2/4 |
| | UCN41 | 0.5 | 0.5 | 1 | 0.5 | 8 |
| | V583 | 0.5 | 0.5/1 | 1 | 0.5 | 4 |
| E. faecium | ATCC19434T | 0.5 | 0.5 | 1 | 0.5 | 8 |
| | BM4147 | 4/8 | 1 | 2 | 1 | >8 |
| | AUS0004 | >8 | 0.5 | 1 | 0.5 | >8 |
| E. coli | ATCC25922 | 2 | >8 | >8 | >8 | >8 |
| Clostridium difficile | ATCC70057 | 0.5 | ≤0.06 | ≤0.06-0.125 | ≤0.06-0.125 | 4 |

**Table 2**

| | | Ampi | TMB3 | TMB6 | TMB7 | TMB8 | TMB9 |
|---|---|---|---|---|---|---|---|
| S. aureus | ATCC25923 | 0.5 | 0.125 | 0.5 | 1 | 0.25 | 1 |
| | ATCC29213 | 0.5 | 0.125 | 0.5/1 | 1 | 0.25 | 1 |
| | ATCC700699 | 8/>8 | 0.125/ 0.25 | >8 | >8 | 0.25/ 0.5 | >8 |
| | ST20131365 | 4 | 0.125/ 0.25 | >8 | >8 | 0.25/ 0.5 | >8 |
| S. epidermis | ATCC14990 | 1 | ≤0.06 | 0.5 | 1 | ≤0.06 | 1 |
| | ATCC35984 | >8 | ≤0.06 | >8 | >8 | ≤0.06 | >8 |
| | ST20140436 | 4 | ≤0.06 | 4 | 8 | 0.125 | 8 |
| | ST20150446 | 8 | ≤0.06/ 0.125 | 4/8 | >8 | 0.125 | 8/>8 |
| E. faecalis | JH2-2 | 0.25/0. 5 | 0.5/1 | 2/4 | 1/2 | 1 | 2 |
| | UCN41 | 0.5 | 1 | 8 | 4 | 1/2 | 8 |
| | V583 | 0.5 | 1 | 4 | 2 | 2 | 4 |
| E. faecium | ATCC19434T | 0.5 | 1 | 8 | 8 | 1/2 | 8 |
| | BM4147 | 4/8 | 2 | >8 | >8 | 2 | >8 |
| | AUS0004 | >8 | 1 | >8 | >8 | 2 | >8 |
| E. coli | ATCC25922 | 2 | >8 | >8 | >8 | >8 | >8 |
| Clostridium difficile | ATCC70057 | 0.5-1 | ≤0.06-0.125 | 2-4 | 4 | ≤0.06 - 0.125 | 2-4 |

**Table 3**

| | | Ampi | TMB18 | TMB1 9 | TMB20 | TMB21 |
|---|---|---|---|---|---|---|
| S. aureus | ATCC25923 | 0.5 | 0.125 | ≤0.06/ 0.125 | 0.125 | ≤0.06/0.1 25 |
| | ATCC29213 | 0.5 | 0.125 | 0.125 | ≤0.06/0.1 25 | ≤0.06/0.1 25 |
| | ATCC700699 | 8/>8 | 0.125 | 0.125/ 0.25 | 0.125 | 0.125 |
| | ST20131365 | 4 | 0.125 | 0.125 | 0.125 | 0.125 |
| S. epidermis | ATCC14990 | 0.5 | ≤0.06 | ≤0.06 | ≤0.06 | ≤0.06 |
| | ATCC35984 | >8 | ≤0.06 | ≤0.06 | ≤0.06 | ≤0.06 |
| | ST20140436 | 8 | ≤0.06 | ≤0.06 | ≤0.06 | ≤0.06 |
| | ST20150446 | 8 | ≤0.06 | ≤0.06 | ≤0.06/0.1 25 | ≤0.06 |
| E. faecalis | JH2-2 | 0.25/0. 5 | 0.25 | 0.125/ 0.25 | 0.25 | 0.25 |
| | UCN41 | 0.5 | 0.25/0. 5 | 0.125/ 0.25 | 0.25 | 0.25 |
| | V583 | 0.5 | 0.25/0. 5 | 0.125/ 0.25 | 0.25/0.5 | 0.25/0.5 |
| E. faecium | ATCC19434T | 0.5 | 0.25 | 0.125/ 0.25 | 0.25 | 0.25 |
| | BM4147 | 4 | 0.5 | 0.25 | 0.25/0.5 | 0.25/0.5 |
| | AUS0004 | >8 | 0.25 | 0.125 | 0.25 | 0.25 |
| E. coli | ATCC25922 | 2 | >8 | >8 | >8 | >8 |
| Clostridium difficile | ATCC700057 | 0.5 | ≤0.06 | ≤0.06 | ≤0.06 | ≤0.06 |

**Table 4**

| | | Ampi | TMB3 | TMB12 | TMB1 3 | TMB1 4 | TMB 15 |
|---|---|---|---|---|---|---|---|
| S. aureus | ATCC25923 | 0.5 | 0.25 | ≤0.06/ 0.125 | 0.125 | 0.125 | 0.25/ 0.5 |
| | ATCC29213 | 0.5 | 0.125 | ≤0.06/ 0.125 | 0.125/ 0.25 | 0.125 | 0.5 |
| | ATCC700699 | 8/>8 | 0.125/ 0.25 | 0.125/ 0.25 | 0.25 | 0.125 | 0.5 |
| | ST20131365 | 4 | 0.25 | 0.5 | 0.25 | 0.125/ 0.25 | 0.25/ 0.5 |
| S. epidermis | ATCC14990 | 0.5 | ≤0.06 | ≤0.06 | ≤0.06 | ≤0.06 | ≤0.0 6 |
| | ATCC35984 | >8 | ≤0.06 | ≤0.06 | ≤0.06 | ≤0.06 | ≤0.0 6 |
| | ST20140436 | 8 | ≤0.06 | ≤0.06 | ≤0.06 | ≤0.06/ 0.125 | 0.12 5 |
| | ST20150446 | 8 | ≤0.06/ 0.125 | ≤0.06 | ≤0.06 | ≤0.06/ 0.125 | 0.12 5/0.2 5 |
| E. faecalis | JH2-2 | 0.25/ 0.5 | 0.5/1 | 0.5 | 1/2 | 0.5 | 2 |
| | UCN41 | 0.5 | 1 | 0.5/1 | 1/2 | 0.5/1 | 2 |
| | V583 | 0.5 | 1 | 1 | 1/2 | 1 | 2/4 |
| E. faecium | ATCC19434T | 0.5 | 0.5/1 | 0.5 | 0.5 | 0.5 | 2 |
| | BM4147 | 4 | 1 | 1 | 2 | 0.5 | 4 |
| | AUS0004 | >8 | 1 | 0.5 | 1/2 | 0.5 | 2 |
| E. coli | ATCC25922 | 2 | >8 | >8 | >8 | >8 | >8 |
| Clostridium difficile | ATCC700057 | 0.5 | 0.125 | ≤0.06 | ≤0.06 | 0.125 | 0.12 5/0.2 5 |

**Table 5**

| | | Ampi | TMB3 | TMB22 | TMB23 | TMB24 |
|---|---|---|---|---|---|---|
| S. aureus | ATCC25923 | 0.5 | 0.25 | 0.125 | ≤0.06 | 0.125 |
| | ATCC29213 | 0.5 | 0.25 | 0.125 | 0.125 | ≤0.06/0.125 |
| | ATCC700699 | 8 | 0.25 | 0.125/0.25 | 0.125 | 0.125/0.25 |
| | ST20131365 | 4 | 0.25 | 0.25 | 0.125 | 0.25 |
| S. epidermis | ATCC14990 | 0.5 | ≤0.06 | ≤0.06 | ≤0.06 | ≤0.06 |
| | ATCC35984 | >8 | ≤0.06 | ≤0.06/0.125 | ≤0.06/0.125 | 0.125 |
| | ST20140436 | 8 | 0.125 | ≤0.06/0.125 | ≤0.06/0.125 | ≤0.06/0.125 |
| | ST20150446 | 4/8 | 0.125 | ≤0.06/0.125 | ≤0.06 | ≤0.06/0.125 |
| E. faecalis | JH2-2 | 0.5 | 1 | 0.5 | 0.25 | 0.25/0.5 |
| | UCN41 | 0.5 | 1 | 1 | 0.125 | 0.25 |
| | V583 | 0.5 | 1 | 0.5 | 0.125/0.25 | 0.25/0.5 |
| E. faecium | ATCC19434T | 0.5 | 1 | 1 | 0.25 | 0.5 |
| | BM4147 | 8 | 1 | 1 | 0.25/0.5 | 0.5 |
| | AUS0004 | >8 | 1 | 1 | 0.25 | 0.5 |
| E. coli | ATCC25922 | 2 | >8 | >8 | >8 | >8 |
| Clostridium difficile | ATCC700057 | 0.25/0.5 | 0.125 | ≤0.06 | ≤0.06 | ≤0.06 |

**Table 6**

| | | Ampi | TMB3 | TMB25 | TMB2 7 | TMB2 8 | TMB2 9 | TMB30 |
|---|---|---|---|---|---|---|---|---|
| S. aureus | ATCC25923 | 0.5 | 0.25 | ≤0.06/ 0.125 | 0.25 | 0.125 | 0.125 | 0.25 |
| | ATCC29213 | 0.5 | 0.125 | 0.25 | 0.25/0 .5 | 0.25 | 0.125 | 0.25 |
| | ATCC700699 | 8 | 0.25 | 0.25 | 0.25 | 0.125 | 0.125 | 0.25 |
| | ST20131365 | 4 | 0.25 | ≤0.06/ 0.125 | 0.5 | 0.25/0 .5 | 0.125 | 0.25 |
| S. epidermis | ATCC14990 | 0.5 | ≤0.06 | ≤0.06 | ≤0.06 | ≤0.06 | ≤0.06 | ≤0.06 |
| | ATCC35984 | >8 | ≤0.06 | ≤0.06 | ≤0.06 | ≤0.06 | ≤0.06 | ≤0.06 |
| | ST20140436 | 8 | 0.125 | ≤0.06/ 0.125 | 0.125 | 0.125 | ≤0.06 | 0.125 |
| | ST20150446 | 8 | 0.125 | 0.125/ 0.25 | 0.125 | 0.125 | ≤0.06/ 0125 | 0.125 |
| E. faecalis | JH2-2 | 0.5 | 0.5 | 0.125 | 0.5 | 0.25 | 0.25 | 0.5 |
| | UCN41 | 0.5 | 0.5 | 0.125 | 0.5/1 | 0.25 | 0.25 | 0.5 |
| | V583 | 0.5 | 1 | 0.125 | 1 | 0.25 | 0.25 | 0.5 |
| E. faecium | ATCC19434T | 0.5 | 0.5 | 0.125 | 1 | 0.25 | 0.25 | 0.5 |
| | BM4147 | 8 | 1 | 0.125 | 1 | 0.5 | 0.25/0 .5 | 1 |
| | AUS0004 | >8 | 1 | 0.125 | 2 | 0.5 | 0.5 | 1 |
| E. coli | ATCC25922 | 2 | >8 | >8 | >8 | >8 | >8 | >8 |
| Clostridiu m difficile | ATCC700057 | 0.5 | 0.06/0. 125 | ≤0.06 | 0.125 | 0.125/ 0.25 | ≤0.06 | 0.125 |

**Table 7**

| | | Ampi | TMB3 | TMB3 2 | TMB33 | TMB34 | TMB37 |
|---|---|---|---|---|---|---|---|
| S. aureus | ATCC25923 | 0.5 | 0.25 | ≤0.06/ 0.125 | 0.125 | ≤0.06 | 0.125 |
| | ATCC29213 | 0.5 | 0.25 | ≤0.06/ 0.125 | 0.25 | ≤0.06 | 0.125/ 0.25 |
| | ATCC700699 | >8 | 0.25 | 0.125/ 0.25 | 0.25 | ≤0.06 | 0.25 |
| | ST20131365 | 4 | 0.25 | 0.125 | 0.25 | ≤0.06 | 0.25 |
| S. epidermis | ATCC14990 | 0.5 | ≤0.06 | ≤0.06 | ≤0.06 | ≤0.06 | ≤0.06 |
| | ATCC35984 | >8 | ≤0.06 | ≤0.06 | ≤0.06 | ≤0.06 | ≤0.06 |
| | ST20140436 | 4/8 | 0.125 | ≤0.06/ 0125 | 0.25 | ≤0.06 | 0.125 |
| | ST20150446 | 4/8 | 0.125 | 0.125/ 0.25 | 0.25 | ≤0.06 | 0.125 |
| E. faecalis | JH2-2 | 0.5 | 0.5 | 0.125 | 0.25 | 0.125 | 0.5 |
| | UCN41 | 1 | 1 | 0.25 | 1 | 0.125 | 0.5 |
| | V583 | 0.5 | 1 | 0.25 | 0.5 | 0.125 | 0.5 |
| E. faecium | ATCC19434T | 0.5 | 0.5 | 0.25 | 0.25 | 0.125 | 0.5 |
| | BM4147 | 4 | 1 | 0.25 | 0.5 | 0.125 | 0.5 |
| | AUS0004 | >8 | 1 | 0.25 | 0.5 | 0.125 | 0.5 |
| E. coli | ATCC25922 | 2 | >8 | >8 | >8 | >8 | >8 |
| Clostridium difficile | ATCC700057 | 0.5 | ≤0.06 | ≤0.06 | ≤0.06 | ≤0.06 | ≤0.06 |

**Table 8 :**

| | | Ampi | TMB3 | TMB26 | TMB3 1 | TMB35 | TMB3 6 |
|---|---|---|---|---|---|---|---|
| S. aureus | ATCC25923 | 0.5 | 0.25 | 0.125 | ≤0.06 | 0.125 | ≤0.06 |
| | ATCC29213 | 0.5 | 0.25 | 0.125 | ≤0.06 | 0.25 | ≤0.06/ 0.125 |
| | ATCC700699 | >8 | 0.25 | 0.25 | 0.125 | 0.25 | 0.125 |
| | ST20131365 | 4 | 0.25 | 0.125 | ≤0.06 /0.12 5 | 0.25 | ≤0.06/ 0.125 |
| S. epidermis | ATCC14990 | 0.5 | ≤0.06 | ≤0.06 | ≤0.06 | ≤0.06 | ≤0.06 |
| | ATCC35984 | >8 | ≤0.06 | ≤0.06 | ≤0.06 | ≤0.06/0. 125 | ≤0.06 |
| | ST20140436 | 4/8 | 0.125 | ≤0.06 | ≤0.06 | 0.125 | ≤0.06 |
| | ST20150446 | 4/8 | 0.125 | ≤0.06 | ≤0.06 | 0.125/0. 25 | ≤0.06 |
| E. faecalis | JH2-2 | 0.5 | 0.5 | 0.25 | 0.25 | 1 | 0.25 |
| | UCN41 | 1 | 1 | 0.25 | 0.25 | 1 | 0.25 |
| | V583 | 0.5 | 1 | 0.5 | 0.25 | 1 | 0.5 |
| E. faecium | ATCC19434T | 0.5 | 0.5 | 0.25 | 0.25 | 0.5 | 0.25 |
| | BM4147 | 4 | 1 | 0.5 | 0.25 | 1 | 0.5 |
| | AUS0004 | >8 | 1 | 0.5 | 0.25 | 1 | 0.25 |
| E. coli | ATCC25922 | 2 | >8 | >8 | >8 | >8 | >8 |
| Clostridium difficile | ATCC700057 | 0.5 | ≤0.06 | 0.125 | ≤0.06 | 0.125 | ≤0.06 |

**Table 9 :**

| | | Ampi | TMB3 | TMB38 | TMB39 | TMB41 |
|---|---|---|---|---|---|---|
| S. aureus | ATCC25923 | 0.25 | 0.125 | ≤0.06 | ≤0.06 | >8 |
| | ATCC29213 | 0.5 | 0.25 | ≤0.06/0.125 | ≤0.06 | >8 |
| | ATCC700699 | 8 | 0.25 | 0.125 | ≤0.06 | >8 |
| | ST20131365 | 4 | 0.25 | ≤0.06 | ≤0.06 | >8 |
| S. epidermis | ATCC14990 | 0.5 | ≤0.06 | ≤0.06 | ≤0.06 | >8 |
| | ATCC35984 | >8 | ≤0.06 | ≤0.06 | ≤0.06 | >8 |
| | ST20140436 | 4 | 0.25 | ≤0.06 | ≤0.06 | >8 |
| | ST20150446 | 4 | 0.125 | ≤0.06 | ≤0.06 | >8 |
| E. faecalis | JH2-2 | 0.5 | 0.5 | 0.125 | 0.25 | >8 |
| | UCN41 | 0.5 | 0.5 | 0.125 | 0.25 | >8 |
| | V583 | 0.5 | 1 | 0.125 | 0.25 | >8 |
| E. faecium | ATCC19434T | 0.5 | 0.5 | ≤0.06 | 0.125 | >8 |
| | BM4147 | 4 | 1 | 0.125 | 0.125 | >8 |
| | AUS0004 | >8 | 1 | 0.125 | 0.5 | >8 |
| Clostridium difficile | ATCC700057 | 0.5 | 0.125/0.25 | 0.125/0.25 | 0.25/0.5 | >8 |

**Table 10 :**

| | | Ampi | TMB3 | TMB42 | TMB43 | TMB45 | TMB46 |
|---|---|---|---|---|---|---|---|
| S. aureus | ATCC25923 | 0.25 | 0.125 | 0.125 | ≤0.06 | 0.125 | >8 |
| | ATCC29213 | 0.5 | 0.25 | 0.25 | 0.125 | 0.25 | >8 |
| | ATCC700699 | 8 | 0.25 | 0.25 | 0.125 | 0.25 | >8 |
| | ST20131365 | 4 | 0.25 | 0.25 | 0.125 | 0.25 | >8 |
| S. epidermis | ATCC14990 | 0.5 | ≤0.06 | ≤0.06 | ≤0.06 | ≤0.06 | >8 |
| | ATCC35984 | >8 | ≤0.06 | ≤0.06 | ≤0.06 | ≤0.06 | >8 |
| | ST20140436 | 4 | 0.25 | ≤0.06 | 0.125 | ≤0.06 | >8 |
| | ST20150446 | 4 | 0.125 | ≤0.06 | ≤0.06 | ≤0.06 | >8 |
| E. faecalis | JH2-2 | 0.5 | 0.5 | 0.25 | 0.25 | 0.5 | >8 |
| | UCN41 | 0.5 | 0.5 | 1 | 0.25 | 0.25 | >8 |
| | V583 | 0.5 | 1 | 1 | 0.25 | 0.5 | >8 |
| E. faecium | ATCC19434T | 0.5 | 0.5 | 0.25 | 0.125 | 0.25 | >8 |
| | BM4147 | 4 | 1 | 0.5 | 0.125 | 0.25 | >8 |
| | AUS0004 | >8 | 1 | 0.5 | 0.25 | 0.5 | >8 |
| Clostridium difficile | ATCC700057 | 0.5 | 0.125 /0.25 | 0.25 | 0.25 | 0.125 | >8 |

**Table 11 :**

| | | Ampi | TMB3 | TMB47 | TMB48 | TMB49 | TMB50 |
|---|---|---|---|---|---|---|---|
| S. aureus | ATCC-25923 | 0.25 | 0.25 | 0.25 | 0.25 | 0.125 | 0.125 |
| | ATCC-700699 | 8 | 0.25 | >8 | 0.5 | 0.25 | 0.125 |
| S. epidermis | ATCC-14990 | 0.5/1 | ≤0.06 | 0.25 | 0.125 | ≤0.06 | ≤0.06 |
| | ATCC-35984 | >8 | ≤0.06 | 0.125 | 0.125 | ≤0.06 | ≤0.06 |
| E. faecalis | JH2-2 | 0.5 | 0.5 | 2 | 1 | 1 | 0.25 |
| | UCN41 | 0.5 | 0.5 | 2 | 1 | 1 | 0.5 |
| E. faecium | ATCC-19434T | 0.5 | 0.5 | 4 | 1 | 0.5/1 | 0.25 |
| | BM-4147 | 4 | 1 | 1 | 2 | 1 | 0.25 |

**Table 12 :**

| | | Ampi | TMB3 | TMB51 | TMB52 | Auranofin |
|---|---|---|---|---|---|---|
| S. aureus | ATCC-25923 | 0.25/ 0.5 | 0.25 | 2 | ≤0.06/ 0.125 | ≤0.06/0.125 |
| | ATCC-700699 | 8 | 0.25 | >8 | 0.125 | ≤0.06 |
| S. epidermis | ATCC-14990 | 0.5/1 | ≤0.06 | 4 | ≤0.06 | ≤0.06 |
| | ATCC-35984 | >8 | ≤0.06 | 2 | ≤0.06 | ≤0.06 |
| E. faecalis | JH2-2 | 0.5 | 0.5 | >8 | 0.25 | ≤0.06/0.125 |
| | UCN41 | 0.5 | 0.5 | >8 | 0.25 | ≤0.06/0.125 |
| E. faecium | ATCC-19434T | 0.5 | 0.5 | >8 | 0.25 | ≤0.06 |
| | BM-4147 | 4 | 1 | >8 | 0.25 | ≤0.06/0.125 |

### Conclusion :

The patent application described the synthesis and the biological properties of unprecedented organometallic species with impressive antibacterial properties and limited cytoxicities in vitro. These compounds were prepared using a copper free click chemistry reaction (1,3 dipolar cycloaddition) between a phosphine-gold(I) azide and an organic precursor bearing an alkyne moiety. In the resulting conjugate, the gold(I) atom has a phosphine ligand and is connected by a covalent gold-carbon bond to a 1,2,3-triazole ring (the three nitrogen are coming from the gold azide and the two carbons from the alkyne function). The antibacterial activity of gold(I) derivatives described in the patent application shows : (1) the crucial role of gold(I) in the biological properties, (2) an inverted correlation between the antibacterial activity and the size and/or lipophilicity of the phosphine ligand and (3) that the 1,2,3-triazole moiety alone is not responsible for biological activities reported in the present application.

### REFERENCES

[1] Dalton Trans. 2014 Apr 28;43(16):5950-69. doi: 10.1039/c4dt00022f.
[2] WO2017/093545.
[3] Protective groups in organic synthesis", T. W. Greene, P. G. M. Wuts, John Wiley and sons, Inc.
[4] PAC, 1996, 68, 2193 (Basic terminology of stereochemistry (IUPAC Recommendations 1996)) on page 2205.

## Claims

1. Gold (I)-phosphine 1,2,3-triazole compound of formula I or I' : 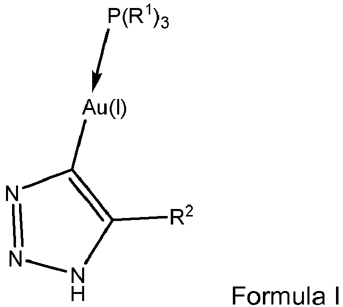 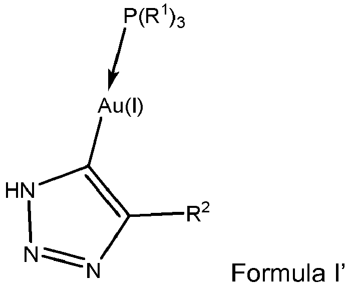 wherein:
- each R¹ group, identical or different, independently represents a linear C₁ to C₁₄ alkyl group, branched or unbranched, cyclic or noncyclic, saturated or unsaturated, optionally comprising heteroatom(s) chosen from the group consisting of O, N and S and optionally comprising aromatic or non-aromatic cycle(s) or heterocycle(s), two or more R¹ groups being optionally covalently linked to form a substituted or non-substituted heterocycle,
- R² represents a substituent of general formula II:
-X-([Y]ₐ-[Z]_{b})_{c} Formula II
in which,
∘ a is an integer equal to 0, 1 or 2,
∘ b is an integer equal to 0, 1 or 2,
∘ c is an integer equal to 0, 1 or 2,
∘ X is a monovalent, divalent or trivalent C₁ to C₂₀ group, branched or unbranched, saturated or unsaturated, substituted or non-substituted, optionally comprising heteroatom(s) chosen from the group consisting of O, N and S and optionally comprising one or more aromatic or non-aromatic, substituted or non-substituted cycle(s) or heterocycle(s), preferably a monovalent or divalent C₁ to C₁₄ group and more preferably a monovalent or divalent C₁ to C₉ group,
∘ Y independently represents an amino acid residue,
∘ Z independently represents -NR³R'³, -SO₂NR³R'³ -OR³, a beta-lactam derivative, a (fluoro)quinolone derivative, a cycline derivative, an oxazolidinone derivative, a macrolide derivative, a ketolide derivative, a puromycin derivative, a aminoside derivative, a lincosamide derivative, a sulfamide derivative, a phenicol derivative, a polymyxin derivative, a rifamycin derivative, a glycopeptide derivative or biotin,
∘ R³ and R'³, identical or different, independently represent H or a C₁ to C₁₈ linear or branched alkyl group, optionally comprising heteroatom(s) chosen from the group consisting of O and N and optionally comprising one or more aromatic or non-aromatic, substituted or non-substituted cycle(s) or heterocycle(s), preferably a C₁ to C₉ linear or branched alkyl group and more preferably C₁ to C₅ linear or branched alkyl group, R³ and R'³ are optionally covalently linked to form a substituted or non-substituted heterocycle,
pharmaceutically acceptable salt or solvate thereof,
and/or isotopes thereof, preferably deuterium, tritium or ¹⁴C isotopes.

2. Compound according to claim 1, wherein R¹ is chosen in the group comprising methyl, ethyl, n-propyl, i-propyl, n-butyl, i-butyl, t-butyl, pentyl, cyclopentyl, hexyl, cyclohexyl, phenyl, benzyl, preferably methyl, ethyl, cyclohexyl, phenyl and i-propyl.

3. Compound according to claim 1 or 2, wherein the linker X is chosen from the following structures: 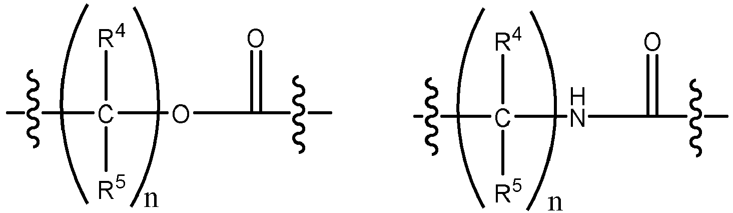 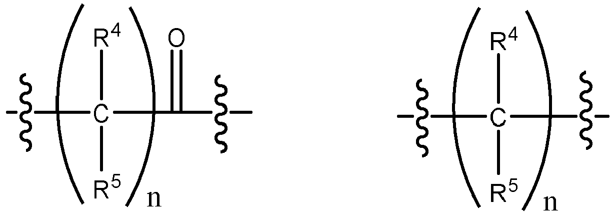 wherein
∘ each R⁴ and R⁵, identical or different, independently represents -H, a halogen, a C₁ to C₅ linear or branched alkyl group, a C₁ to C₅ linear or branched alkoxy group, -OH or - NR³R'³, R³ and R'³ identical or different, independently being H or a C₁ to C₁₈ linear or branched alkyl group, optionally comprising heteroatom(s) chosen from the group consisting of O and N, R³ and R'³ are optionally covalently linked to form a substituted or non-substituted heterocycle, R⁴ and R⁵ being optionally covalently linked to form a cycle or heterocycle,
∘ n is an integer from 1 to 5, 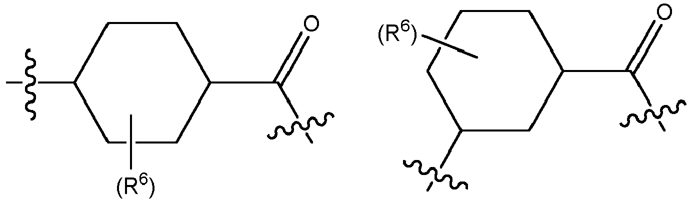 wherein
∘ R⁶ is one ore more substituent(s) on the ring and independently represents one or more -H, halogen(s), C₁ to C₅ linear or branched alkyl group(s), a C1 to C5 linear or branched alkoxy group(s), -OH or -NR¹⁰R'¹⁰, R¹⁰ and R'¹⁰ identical or different, independently being H or a C₁ to C₁₃ linear or branched alkyl group, optionally comprising heteroatom(s) chosen from the group consisting of O and N, R¹⁰ and R'¹⁰ are optionally covalently linked to form a substituted or non-substituted heterocycle, R⁶ groups being optionally covalently linked to form a cycle or heterocycle, 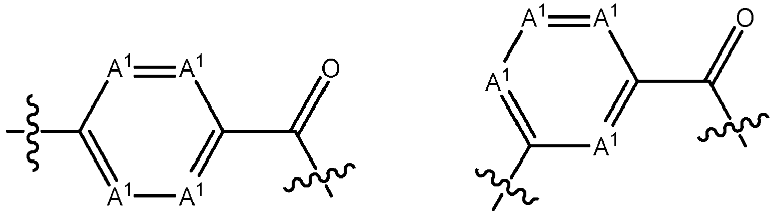 wherein
∘ each A¹ independently represents N, CH or CR¹¹,
∘ R¹¹ is one ore more substituent(s) on the ring and independently represents one or more -H, halogen(s), a C₁ to C₅ linear or branched alkyl group(s), a C₁ to C₅ linear or branched alkoxy group(s), -OH or -NR¹²R'¹², R¹² and R'¹² identical or different, independently being H or a C₁ to C₁₇ linear or branched alkyl group, optionally comprising heteroatom(s) chosen from the group consisting of O and N, R¹² and R'¹² are optionally covalently linked to form a substituted or non-substituted heterocycle, R¹¹ groups being optionally covalently linked to form a cycle or heterocycle, 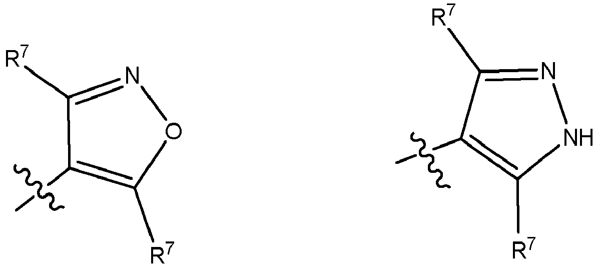 wherein
∘ each R⁷ independently represents -H, a halogen, a C₁ to C₅ linear or branched alkyl group, a C₁ to C₅ linear or branched alkoxy group, -OH or -NR¹²R'¹², R¹² and R'¹² identical or different, independently being H or a C₁ to C₁₇ linear or branched alkyl group, optionally comprising heteroatom(s) chosen from the group consisting of O and N, R¹² and R'¹² are optionally covalently linked to form a substituted or non-substituted heterocycle, R⁷ groups being optionally covalently linked to form a cycle or heterocycle, 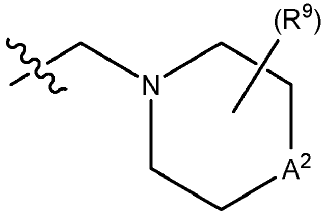 wherein
∘ A² represents CH₂, CHR⁸, NR⁸, O, S or SO₂,
∘ R⁹ is one ore more substituent(s) on the ring and independently represents one or more -H, halogen(s), C₁ to C₅ linear or branched alkyl group(s), C₁ to C₅ linear or branched alkoxy group(s), -OH or -NR¹³R'¹³, R¹³ and R'¹³ identical or different, independently being H or a C₁ to C₁₅ linear or branched alkyl group, optionally comprising heteroatom(s) chosen from the group consisting of O and N, R¹³ and R'¹³ are optionally covalently linked to form a substituted or non-substituted heterocycle, R⁹ groups being optionally covalently linked to form a cycle or heterocycle,R⁸ represents -H or a C₁ to C₅ linear or branched alkyl group, 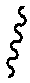 representing the point of attachment to the 1,2,3-triazole and to the Y or Z group.

4. Compound according to any of preceding claims, wherein the amino acid residue Y is chosen in the group comprising natural and synthetic, alpha, beta or gamma amino acid residues.

5. Compound according to claim 4 where the amino acid is chosen in the group comprising arginine, ω-NO₂-arginine, histidine, lysine, aspartic acid, glutamic acid, serine, threonine, asparagine, glutamine, selenocysteine, glycine, proline, alanine, isoleucine, leucine, phenylalanine, tyrosine, tryptophan, valine, phenylglycine, hydroxyphenylglycine, substituted or not substituted, preferably glycine, phenylglycine, hydroxyphenylglycine, ω-NO₂-arginine, aspartic acid, tryptophan, tyrosine and phenylalanine.

6. Compound according to any of preceding claims, wherein the group Z is a beta-lactam derivative chosen in the group comprising penicillins or cephalosporins derivatives or is fluoroquinolone.

7. Process of synthesis of a compound according to any preceding claim comprising a step of reacting a compound of formula Ia : 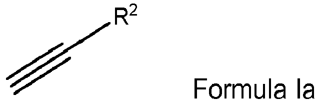 with a compound of formula Ib: 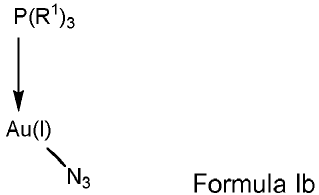 wherein R¹ and R² are defined as above.

8. Compound of formula la: 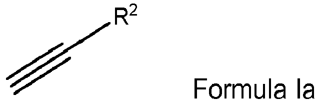 wherein R² is defined as above.

9. Compound of formula Ib: 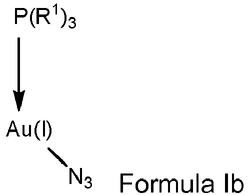 wherein R¹ is defined as above.

10. Compound according to any one of claims 1 to 6 for use in a human or animal medicine.

11. Compound according to any one of claims 1 to 6 for use in the prevention and/or treatment of an infection, preferably bacterial or fungal.
